# EUROPEAN PATENT SPECIFICATION

(11) **EP 1 019 439 B1**
(45) Date of publication and mention of the grant of the patent: **16.11.2011**
(21) Application number: 98949572.6
(22) Date of filing: 28.09.1998
(51) Int. Cl.: C07K 14/725, A61K 38/00, A61K 39/00, C07K 14/705, C12N 15/00

(54) **SOLUBLE SINGLE-CHAIN T-CELL RECEPTOR PROTEINS**
LÖSLICHE, EINZELKETTIGE PROTEINE DES T-ZELLREZEPTORS
PROTEINES SOLUBLES DU RECEPTEUR DES LYMPHOCYTES T A CHAINE UNIQUE

(30) Priority: 02.10.1997 US 943086
(43) Date of publication of application: 19.07.2000
(73) Proprietor: Altor BioScience Corporation, Miramar, Florida 33025 (US)
(72) Inventor: WEIDANZ, Jon, A., Miami, FL 33184 (US); CARD, Kimberlyn, F., Pembroke Pines, FL 33025 (US); WONG, Hing, C., Fort Lauderdale, FL 33332 (US)
(74) Representative: Plougmann & Vingtoft A/S
(86) International application number: PCT/US1998/020263
(87) International publication number: WO 1999/018129

(56) References cited:
- WO-A-91/18019
- WO-A-92/02629
- WO-A-96/13593
- WO-A-96/18105
- WO-A-98/39482
- WO-A1-92/02629
- US-A- 5 565 335
- MOLLOY P.E. ET AL.: "Production of soluble single-chain T-cell receptor fragments in Escherichia coli trxB mutants" MOL. IMMUNOL., vol. 35, February 1998 (1998-02), pages 73-81, XP002212852
- BRODNICKI T.C., HOLMAN P.O. AND KRANZ D.M.: "reactivity and epitope mapping of single-chain T cell rceptors with monoclonal antibodies" MOL. IMMUNOL., vol. 33, no. 3, March 1996 (1996-03), pages 253-263, XP002212853
- WONG C P ET AL: "SINGLE CHAIN T CELL RECEPTOR AS A TUMOR SPECIFIC VACCINE" FASEB JOURNAL, FED. OF AMERICAN SOC. FOR EXPERIMENTAL BIOLOGY, BETHESDA, MD, US, vol. 9, no. 3, 9 March 1995 (1995-03-09), page A514 XP002002800 ISSN: 0892-6638
- WEBER S ET AL: "SPECIFIC LOW-AFFINITY RECOGNITION OF MAJOR HISTOCOMPATIBILITY COMPLEX PLUS PEPTIDE BY SOLUBLE T-CELL" NATURE, MACMILLAN JOURNALS LTD. LONDON, GB, vol. 356, 30 April 1992 (1992-04-30), pages 793-796, XP002008364 ISSN: 0028-0836
- WUELFING C AND PLUECKTHUN A.: "SOLUBLE T-CELL RECEPTOR FRAMENTS- GUIDANCE OF FOLDING AND ASSEMBLY" IMMUNOLOGIST, HOGREFE AND HUBER PUBLISHERS, TORONTO, CA, vol. 3, no. 2, 1 March 1995 (1995-03-01), pages 59-66, XP000575900 ISSN: 1192-5612
- NOVOTNY J ET AL: "A SOLUBLE, SINGLE-CHAIN T-CELL RECEPTOR FRAGMENT ENDOWED WITH ANTIGEN-COMBINING PROPERTIES" PROCEEDINGS OF THE NATIONAL ACADEMY OF SCIENCES OF USA, NATIONAL ACADEMY OF SCIENCE. WASHINGTON, US, vol. 88, no. 19, 1 October 1991 (1991-10-01), pages 8646-8650, XP002002796 ISSN: 0027-8424
- GREGOIRE C ET AL: "ENGINEERED SECRETED T-CELL RECEPTOR ALPHABETA HETERODIMERS" PROCEEDINGS OF THE NATIONAL ACADEMY OF SCIENCES OF USA, NATIONAL ACADEMY OF SCIENCE. WASHINGTON, US, vol. 88, no. 18, 15 September 1991 (1991-09-15), pages 8077-8081, XP002039182 ISSN: 0027-8424
- SCHODIN B.A. ET AL.: "Binding properties and solubility of single-chain T cell rceptors expressed in E. coli" MOL. IMMUNOL., vol. 33, no. 9, June 1996 (1996-06), pages 819-829, XP002212854
- MARIUZZA R ET AL: "Secretion of a homodimeric ValphaCkappa T-cell receptor-immunoglobulin chimeric protein" JOURNAL OF BIOLOGICAL CHEMISTRY, AMERICAN SOCIETY OF BIOLOGICAL CHEMISTS, BALTIMORE, MD, US, vol. 264, no. 13, 5 May 1989 (1989-05-05), pages 7310-7316, XP002149783 ISSN: 0021-9258
- WARD E S: "PRODUCTION AND MANIPULATION OF ANTIBODIES AND T-CELL RECEPTORS USING RECOMBINANT DNA TECHNOLOGY" THERAPEUTIC IMMUNOLOGY, BLACKWELL SCIENTIFIC PUBL. LONDON, GB, 1996, pages 335-346, XP000856686 ISSN: 0967-0149

## Description

### 1. Field of the Invention

The present invention relates to soluble fusion proteins comprising an immunoglobulin light-chain constant region or fragment thereof covalently linked to a single-chain T-cell receptor, single chain T-cell receptors, DNA segments encoding such proteins, DNA vectors comprising such DNA segments, methods of isolating such soluble fusion proteins and using such proteins. The proteins are useful for a variety of applications including *in vitro* screens for detection of cells expressing desired peptide-MHC complexes.

### 2. Background

A T-cell response is modulated by antigen binding to a T-cell receptor (TCR). One type of TCR is a membrane bound heterodimer consisting of an a and β chain resembling an immunoglobin variable (V) and constant (C) region. The TCR a chain includes a covalently linked V-α and C-α chain, whereas the β chain includes a V-β chain covalently linked to a C-β chain. The V-α and V-β chains form a pocket or cleft that can bind a superantigen or antigen in the context of a major histocompatibility complex (MHC) (known in humans as an HLA complex). See generally Davis Ann. Rev. of Immunology 3: 537 (1985); Fundamental Immunology 3rd Ed., W. Paul Ed. Rsen Press LTD. New York (1993).

The TCR is believed to play an important role in the development and function of the immune system. For example, the TCR has been reported to mediate cell killing, increase B cell proliferation, and impact the development and severity of various disorders including cancer, allergies, viral infections and autoimmune disorders. Accordingly, it has been of great interest to develop methods of obtaining TCRs for use in commercial, research and medical settings.

Generally stated, TCRs have been difficult to isolate in significant quantities. For example, one major obstacle has been the production of soluble TCRs in the absence of the TCR transmembrane region. More specifically, it has been reported that if TCRα and β chains are not assembled into a CD3 complex comprising γ, δ, ε, and ζ chains, the protein is typically degraded. See e.g., Shin et al. (1993) Science 259: 1901.

Attempts to produce soluble TCRs without transmembrane regions have often been performed in bacteria. However, bacterial TCR expression typically results in formation of inclusion bodies exemplifying insoluble and improperly folded molecules. In many cases, TCRs can be obtained by these methods after difficult protein solubilization and re-folding steps. These steps substantially reduce TCR yields and negatively impact TCR stability and functionality.

Further attempts to obtain soluble TCRs have focussed on designing more soluble TCR molecules. For example, TCRs have been fused with various protein and polypeptide sequences such as phosphatidylinositol linkage sequences and CD3 chains. Oftentimes, the goal has been to express the TCR in a specific cell compartment to assist protein folding. TCR fusion proteins expressed on the cell surface are usually cleaved by conventional methods in an attempt to obtain soluble TCR. However, only small amounts of fully soluble and functional TCR are usually produced. See e.g., Matsui, K., et al. (1991) Science 254, 1788 (1991); Engel, I., et al. (1992) Science 256, 1318; Lin, A.Y. et al., (1990) Science 249, 677.

Other attempts to produce soluble TCRs have included synthesizing heterodimeric TCR molecules that include TCR V chains covalently linked to immunoglobin constant regions. For example, Gregoire, C., et al. (1991) PNAS, 88, 8077 have reported secretion of heterodimeric αβ TCR chains that include a Cα ,Vα sequence joined to the C region of a kappa light chain (Cκ). The reference also disclosed a VβCβCκ sequence. See also Weber, S., et al. (1992) Nature, 356: 793.

Although synthesis of the heterodimeric TCRs has been reported to enhance soluble expression in some cases, the methods have substantial shortcomings. For example, synthesis often requires co-transfection of DNA into specialized cells which must correctly assemble the multiple protein chains. These methods often substantially reduce yields of functional heterodimeric αβ TCR.

Significantly, the Cκ chain has been reported to negatively impact TCR heterodimer expression. For example, it has been suggested that replacing the Cκ chain may improve TCR heterodimer valency in some cases. Further, chimeric Vβ chains comprising Cκ regions have been disclosed as being particularly difficult to process, secrete, and/or fold. See Gregoire, et al., *supra*; Mariuzza, R.A. and Winter, G., (1989) 264:7310; Gascoigne, N.R.J., et al., PNAS (USA) (1987), 84:2936.

Still other attempts to produce fully soluble and functional TCRs have included construction of single-chain proteins comprising TCR V regions (ie. sc-TCRs). Generally stated, sc-TCRs include a fused V-α and V-β chain. See Novotny, J. et al. PNAS (USA) 88, 8646 (1991); Soo Hoo, W.F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); PCT WO 96/13593; Ward, E.S.et al., J. Mol. Biol. 224, 885, (1992); and Schlueter, C.J. et al. J. Mol. Biol. 256, 859 (1996).

However, prior methods for producing the sc-TCRs have often yielded insoluble and improperly folded molecules. For example, published PCT application WO 96/18105 discloses methods of expressing sc-TCRs that generally require inconvenient protein re-folding and solubilization steps. The methods typically produce unacceptably low amounts of sc-TCRs. See also Ward, E.S. et al. *supra,* and Schlueter, C.J. *supra.*

Several strategies have been developed in attempts to improve sc-TCR yields. The strategies include targeting protein expression to cell surfaces and use of solublizing carrier proteins. See e.g., published PCT applications WO 96/18105 and WO 96/13593. However, the sc-TCRs produced by these methods often require time-consuming manipulations to obtain even modest amounts of protein.

It has been of great interest to study interactions of immune system molecules *in vitro* and *in vivo.* There is a great need, therefore, for soluble and fully functional TCR molecules that can be readily produced in significant amounts. For example, it has been proposed that the TCR V region provides an attractive target for small molecules that can potentially modulate TCR function, (e.g., TCR antagonists or agonists). It would thus be desirable to have convenient methods of producing soluble and fully functional sc-TCR fusion proteins in significant quantities.

### SUMMARY OF THE INVENTION

The present invention relates to soluble single-chain T-cell receptor proteins, e.g., fusion proteins that include a single-chain T-cell receptor covalently linked to an immunoglobin light chain constant region. It has been found that fusion of an immunoglobin light chain constant region to the single-chain T-cell receptor unexpectedly facilitates soluble expression. The single-chain T-cell receptor proteins can be isolated in significant quantities without performing difficult solubilization, cleaving or re-folding steps.

The single-chain T-cell receptor (i.e., sc-TCR) proteins of the present invention are fully functional and soluble. In one aspect, the sc-TCR proteins include a covalently linked TCR Vα and Vα chain fused to an immunoglobin light chain constant region (ie. Ig-C_{L}) or suitable Ig-C_{L} fragment. A sc-TCR fusion protein of the invention will sometimes be referred to herein as a "sc-TCR fusion protein", "soluble fusion protein" or similar phrase. Expression of the soluble sc-TCR proteins reduces formation of insoluble bodies in host cells, thereby significantly increasing stability and yields of the protein, as well as enhancing ease of purification.

The sc-TCR fusion proteins generally include the Ig-C_{L} chain suitable fragmen covalently linked to a single-chain V chain. The V chain includes Vα,β sequence in which the V-α chain is generally fused to a V-β chain through a peptide linker sequence. The fusion product is further covalently linked through the V-α or V-β chain to the Ig-C_{L} chain or suitable Ig-C_{L} fragment. The Ig-C_{L} chain or fragment thereof can be spaced from the single-chain V sequences, e.g., by a peptide linker sequence such as those provided herein.

In general, the Ig-C_{L} chain is a κ- or λ-type immunoglobin light chain constant region of known sequence. The κ-type immunoglobin light chain constant region will often be referenced herein as "Cκ chain", whereas the λ-type immunoglobin constant chain light chain region will often be referred to as "C_{λ}, chain". For example, the Ig-C_{L} chain can be a Cκ chain or a suitable fragment thereof such as those disclosed below. The present invention provides fully soluble and functional fusion proteins and methods of obtaining significant quantities of the sc-TCR fusion proteins or sc-TCRs derived from same for a variety of useful applications.

The sc-TCR proteins of the present invention provide a number of significant advantages. For example, prior practice generally required extensive manipulation of TCR-related proteins (e.g., TCR receptors, TCR heterodimers, sc-TCRs), before significant amounts of protein could be obtained. In contrast, the sc-TCR proteins of the present invention can be obtained in significant amounts. For example, the sc-TCR fusion proteins include a fused Ig-C_{L} chain or suitable Ig-C_{L} fragment that positively impacts solubility, yield, and functionality of the sc-TCR fusion proteins. Thus, analysis of TCR and sc-TCR interactions with e.g., small molecules (e.g., synthetic peptides, drugs, etc.) and antigen presenting cells (APCs) will be facilitated by use of the soluble fusion proteins provided herein. Additionally, a wide variety of the sc-TCR fusion proteins can be presented for interaction with superantigens or APCs as will be described more fully below.

Further, fusion of the Ig-C_{L} chain or suitable Ig-C_{L} fragment to the sc-TCR provides a convenient means of detecting and purifying the fusion proteins by conventional immunological methods such as those disclosed below.

Still further advantages are provided by DNA constructs disclosed below that encode the sc-TCR proteins. For example, some of the DNA constructs provided include DNA segments that encode sc-TCRs in a cassette format that can be switched, with another segment encoding a desired sc-TCR. The DNA construct can encode a fused Ig-C_{L} chain or Ig-C_{L} fragment that is fully compatible with many TCR Vαβ sequences and can be expressed in host cells to provide significant quantities of protein. The present invention thus provides methods of producing substantial amounts of fully soluble and functional sc-TCR proteins for research, medical or commercial use.

The sc-TCR proteins of the present invention have numerous uses *in vitro* and *in vivo.*

For example, the sc-TCR proteins can be used *in vitro* to detect and analyze ligands such as peptides and MHC/HLA molecular components of TCR ligands. The sc-TCR proteins can also be used as provided for diagnostic purposes such as for the detection of T-cells with pathogenic properties. The sc-TCRs can additionally be employed in functional, cellular and molecular assays, and in structural analysis, including X-ray crystallography, nuclear magnetic resonance imaging, computational techniques such as computer graphic display. Of particular interest are those known techniques that can be readily adapted for the identification of small molecules, that modulate interaction between TCRs and MHC complexes. Related techniques can be used to screen for small molecules that potentially block interaction between a TCR and a TCR specific antibody. Particularly contemplated are those screens designed to detect TCR antagonists *in vitro.*

The sc-TCR proteins also have significant uses in *vivo.* For example, the proteins can be employed to compete with pathogenic T-cells such as those implicated in an immune-related disorder or disease; or to immunize mammals, e.g., humans, against TCR structures such as extracellular regions that occur on the surface of T-cells and which perform or help other molecules perform pathogenic or otherwise harmful functions. Particularly, the sc-TCR proteins can be used to raise antibodies according to known immunological methods such as those described below. The antibodies produced by the methods can be used in therapeutic strategies designed to modulate immune responses *in vivo,* e.g., by inhibiting or reducing numbers of specific T-cells that recognize a desired antigen. Particularly, monoclonal antibodies can be selected that specifically bind TCR epitopes so that a restricted T-cell subset or population implicated in a pathology can be targeted and eliminated or substantially reduced in number. As will be described more fully below, the sc-TCR proteins or antibodies binding same can be unmodified, or if desired, can be covalently linked to a desired molecule, e.g., drugs, toxins, enzymes or radioactive substances sometimes through a linked peptide linker sequence.

Additionally, the sc-TCR proteins of the present invention can be used to screen for APCs expressing a desired peptide-MHC complex *in vitro.* More specifically, it has been useful in several settings to obtain and expand selected APCs comprising a desired peptide-MHC complex.

Furthermore, DNA constructs encoding the sc-TCR fusion proteins can be used to make a bacteriophage display library in accordance with methods described in pending U.S. Application Serial No. 08/813,781 filed on March 7, 1997.

Still further, the sc-TCR proteins can be used to screen peptide libraries *in vitro* to detect peptide binding sequences, such as potential superantigens.

The invention also pertains to a DNA segment ( cDNA or genomic DNA) comprising a sequence encoding a sc-TCR protein of interest. For example, the DNA segment can encode a desired sc-TCR covalently linked to the Ig-C_{L} chain or suitable Ig-C_{L} fragment. Nucleic acid encoding the sc-TCR can be obtained from a variety of sources including polymerase chain reaction (PCR) amplification of publicly available TCR and Ig-C_{L} chain DNA sequences. The nucleic acid segment can be provided as a DNA vector capable of expressing the sc-TCR protein in a suitable eukaryotic or prokaryotic cell expression system. The segment may include operably linked transcriptional elements such as a promoter, leader, transciptional initiation sequence, and optimal enhancer sequences to drive expression of the soluble sc-TCR protein in a desired cell expression system. Alternatively, the DNA vector itself may provide some or all of the control elements.

The nucleic acid segment encoding the sc-TCR protein is often constructed so that naturally-occurring TCR control elements are removed. However, in some cases it may be desirable to drive expression of the sc-TCR protein with an immunoglobin promoter and/or enhancer such as those known in the field. The Ig-C_{L} chain or suitable Ig-C_{L} fragment encoded by a nucleic acid segment of the invention often includes intron and exon sequence in those instances where the soluble fusion protein is expressed in a cell type that can perform RNA splicing, typically a mammalian cell. In cases where it is desired to express the sc-TCR fusion protein in a prokaryotic cell, the introns can be removed or substantially reduced in size to maximize expression. As will be more fully described below, a variety of Ig-C_{L} chains or suitable fragments thereof can be fused to the sc-TCR molecules as desired, provided that the expressed sc-TCR fusion protein is fully soluble and functional as determined by assays described below.

Further provided are methods of isolating the soluble sc-TCR proteins disclosed herein. The methods generally include introducing a desired DNA segment or a vector comprising same into cells capable of expressing the fusion proteins. Host cells comprising the segment or vector are cultured in medium capable of supporting cell proliferation. The host cells can be eukaryotic cells, preferably mammalian cells, that are capable of expressing the soluble fusion proteins in soluble form. However in some instances it may be desirable to express the soluble fusion proteins in bacteria. As will be explained in more detail below, bacterial cells expressing a soluble sc-TCR protein can be cultured under slow induction conditions. The slow induction conditions generally refer to cell growth conditions in which an essential nutrient is depleted from the medium over several hours, thereby inducing soluble expression of the sc-TCR protein in significant quantities. It is thus desirable in such cases to design bacterial expression vectors to maximize expression under the slow induction conditions. Soluble sc-TCR proteins expressed in eukaryotic or prokaryotic cells can be purified, if desired, to produce substantially pure sc-TCR fusion protein.

In some instances, the soluble sc-TCR proteins will include one or more fused effectors or tags (typically one or two). For example, in some cases the tags can be used to help purify the sc-TCR protein from naturally-occurring cell components which typcially accompany the fusion protein. In other cases, the protein tag can be used to introduce a pre-determined chemical or proteolytic cleavage site into the soluble protein. Particularly, contemplated is introduction of a segment encoding a tag into a DNA vector, e.g., between sequence encoding the soluble fusion protein and the Ig-C_{L} chain or suitable fragment so that the sc-TCR molecule can be cleaved (ie. separated) from the Ig-C_{L} chain or fragment if desired.

The sc-TCR fusion proteins and sc-TCR molecules can be used in a variety of applications including detection and/or imaging cells or tissue *in vivo,* as well as damaging or killing cells *in vitro* or *in vivo.* In general, targeted cells or tissue will include one or more ligands capable of selectively binding the sc-TCR. Exemplary cells include tumor cells such as melanoma and virally-infected cells (e.g., cells infected with a primate DNA or RNA virus such as cytomegalovirus or the AIDS virus, respectively).

An undesired immune response in an animal such as a mammal may be reduced or eliminated by one or a combination of alternative strategies. In general, an immune response in a mammal can be reduced or eliminated by providing an effective amount of an sc-TCR protein in a pharmaceutically acceptable formulation. The sc-TCR portion of the soluble fusion protein can be used in such the treatment methods. Generally, the sc-TCR fusion protein or sc-TCR will be one which is capable of competing for ligand (e.g., antigen) with one or more TCRs, particularly those TCRs associated with pathogenic T-cells.

Methods for increasing the specific binding affinity of the sc-TCR proteins are also provided herein. For example, the pending U.S. Application No. 08/813,781, discloses methods of making sc-TCR "muteins" which exhibit about 2 to 10 fold greater specific binding affinity for antigen than a corresponding TCR (i.e., the TCR naturally comprising the Vαβ chains of the sc-TCR fusion protein). The methods generally involve using standard oligonucleotide-directed primer mutagenesis of the sc-TCR molecule such that one or more pre-determined amino acid mutations are introduced into the molecule to improve specific binding. The disclosed sc-TCR muteins are particularly useful *in vitro* and *in vivo,* e.g., in the suppression or elimination of T-cells implicated in an undesired immune response. The methods disclosed in the pending U.S. application can be readily adapted to improve specific binding of the present sc-TCR fusion proteins and sc-TCR molecules as desired.

Additionally, methods of making antibodies (polyclonal, monoclonal or chimeric) which specifically bind a TCR are described herein. The methods generally include using a substantially purified sample of an sc-TCR protein as immunogen. In some cases it will be preferred to use the sc-TCR molecule as immunogen, e.g., to minimize immunological rejection against immunologically reactive Ig-C_{L} chains or fragments. Exemplarly antibodies are monoclonal antibodies obtained by conventional hybridoma manipulations. The antibodies can also be generated from an immunogenic peptide that comprises one or more epitopes of the sc-TCR. Such antibodies, are useful for a variety of applications including detecting pathogenic T-cells in a biological sample such as blood, serum, or a tissue specimen. As referred to previously, the antibodies can be used to deplete or inhibit the activity of targeted T-cells *in vivo* or *in vitro* particularly by reducing interaction between targeted T-cells and antigen or MHC/HLA peptide complex. It will be useful in some settings to covalently attach a suitable cytotoxic, anti-metabolic, or detectable label to the antibody by methods well-known in the field.

The present invention further pertains to use of a soluble fusion protein of the invention for the preparation of a medicament for inducing an immune response in a mammal, particularly a primate such as a human, In cases where the sc-TCR fusion protein is used to induce the immune response, it is preferred that the Ig-C_{L} chain or fragment haplotype be compatible with the host being used. With these immunogens it is possible to immunize the mammal against TCR antigenic structures that occur on the surface of T-cells (ie. targeted T-cells) and which are implicated in a pathogenic or otherwise undesirable immune response. Such T-cells can be identified by conventional methods and can be purified if desired and assayed for capacity to undergo proliferation. Methods for identifying, purifying, and assaying T-cells are described below.

T-cells that exhibit a desired proliferative response can be established as cultured cell lines from which nucleic acid can be isolated and used to produce sc-TCR fusion proteins. Target TCR antigenic structures of interest will typically include clonotypic epitopes, V-α or V-β family-specific epitopes, conformational epitopes, and linear epitopes. Immunization against the TCR antigenic structures generally inhibits T-cell activity, thereby reducing or eliminating the pathogenic or undesirable effects of the T-cells. The mammal is typically immunized by administering the sc-TCR or sc-TCR fusion protein in an immunologically effective amount (ie. capable of producing significant antibody titres) and in a pharmaceutically acceptable mixture, so that targeted T-cells are depleted or eliminated by the host immune system.

Further contemplated are assays for detecting a small molecule capable of specifically binding a TCR. Generally, the assays will be *in vitro* screens in which an sc-TCR protein of the invention is bound to a solid support (e.g., a microtitre plate) and incubated with a small molecule of interest under conditions which favor specific binding between the sc-TCR and the small molecule. For example, an sc-TCR or sc-TCR fusion protein of interest can be used in well-known panning-type screens to detect and isolate small molecules that specifically bind TCR V regions. It will be preferred in some cases to use the sc-TCR fusion molecules so that the Ig-C_{L} or suitable Ig-C_{L} or fragment is bound to the solid support, thereby minimizing interference between the support and the sc-TCR V regions.

Particularly contemplated are screens to detect small molecules capable of inhibiting specific binding between a superantigen or a peptide-MHC complex and a TCR. Generally stated, the screens involve *in vitro* assays in which an sc-TCR protein of the invention is incubated with the small molecule and the superantigen or peptide-MHC complex under conditions which permit the soluble fusion protein to form a specific binding complex. In a separate control reaction, the soluble fusion protein is incubated with the superantigen or peptide-MHC complex under the same incubation conditions. A ligand capable of inhibiting specific binding between the superantigen or peptide-MHC complex and the sc-TCR is then selected in accordance with conventional protein binding assay techniques.

Also contemplated are screens to detect ligands generated by combinatorial-type chemistry. For example, in one assay, a sc-TCR molecule of interest is bound to the solid support and mixed with a first known ligand, e.g., a superantigen, and a second ligand series produced by standard combinational chemistry. An exemplary second ligand series is a pool of peptides typcially differing at one pre-determined amino acid. Incubation conditions are then performed under conditions which favor specific binding between the first ligand and the sc-TCR protein. A peptide in the second ligand series which effectively blocks interaction between the sc-TCR protein and the first ligand can be detected and isolated in accord with standard methods. Ligands detected by such screens have a variety of uses, including use in therapeutic compositions and detection of APCs *in vitro* and *in vitro.*

### BRIEF DESCRIPTION OF THE DRAWINGS

Figs. 1A and 1B are drawings showing the pJRS149 (1A) and pKC12.2 (1B) vectors.

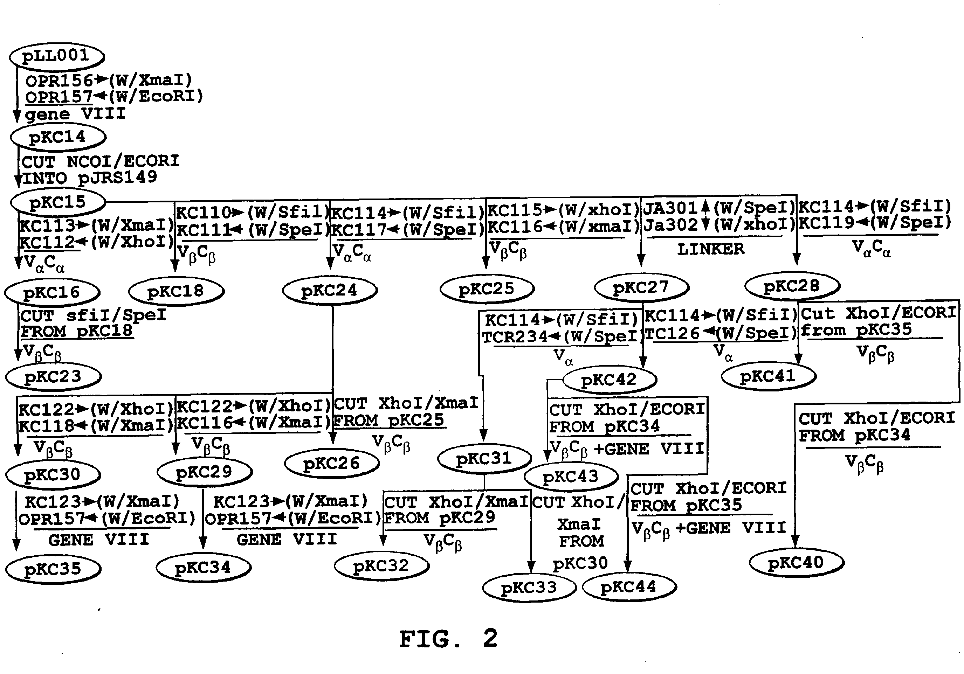

Fig. 2 is a flow chart outlining the construction of various DNA vectors.
Fig. 3 is a flow chart outlining the construction of the pKC45, pKC46 (pSUN18) and pKC51 DNA vectors from pKC12.2.
Fig. 4 is a drawing illustrating the PEN2 DNA vector.
Fig. 5 is a drawing showing inserts of pKC60 and pKC67 DNA vectors.

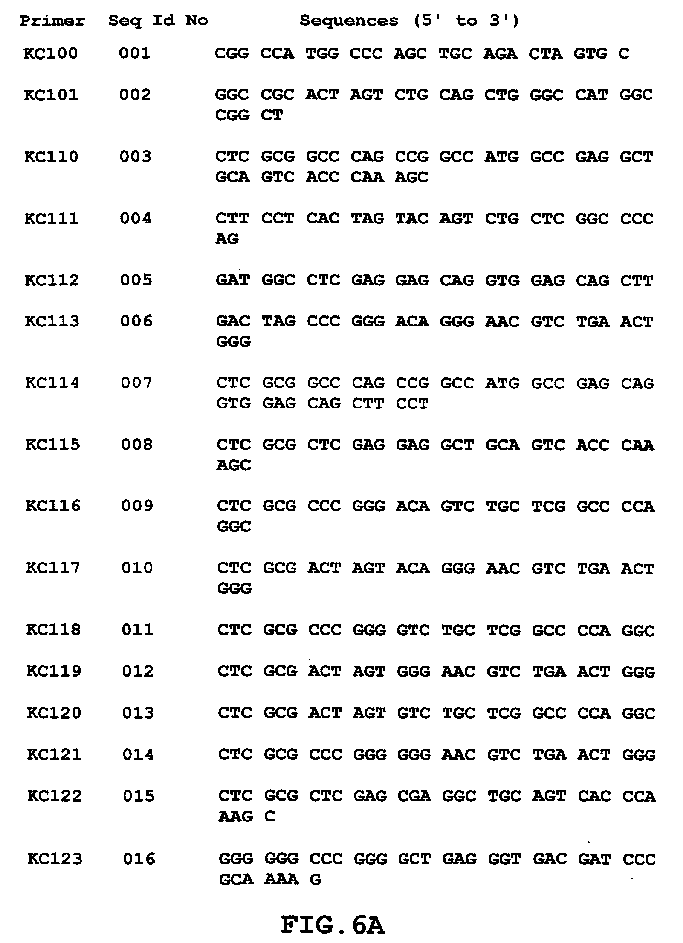

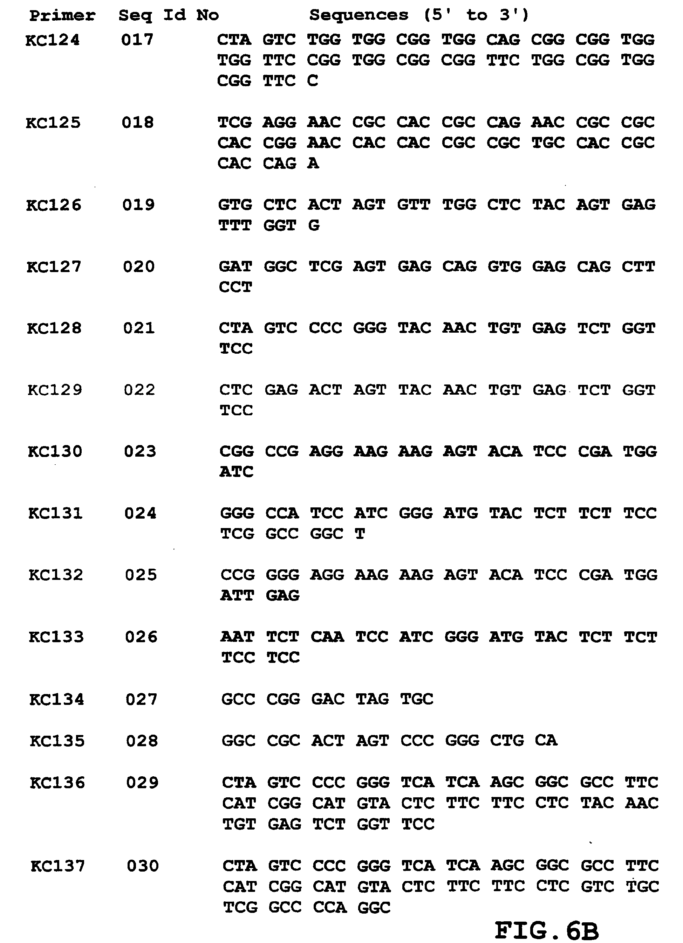

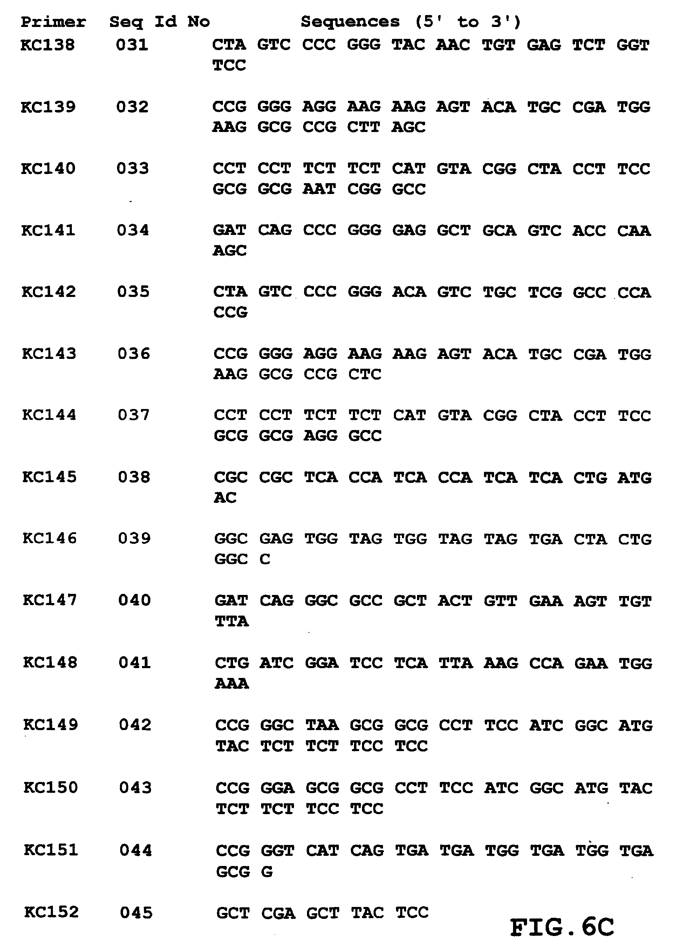

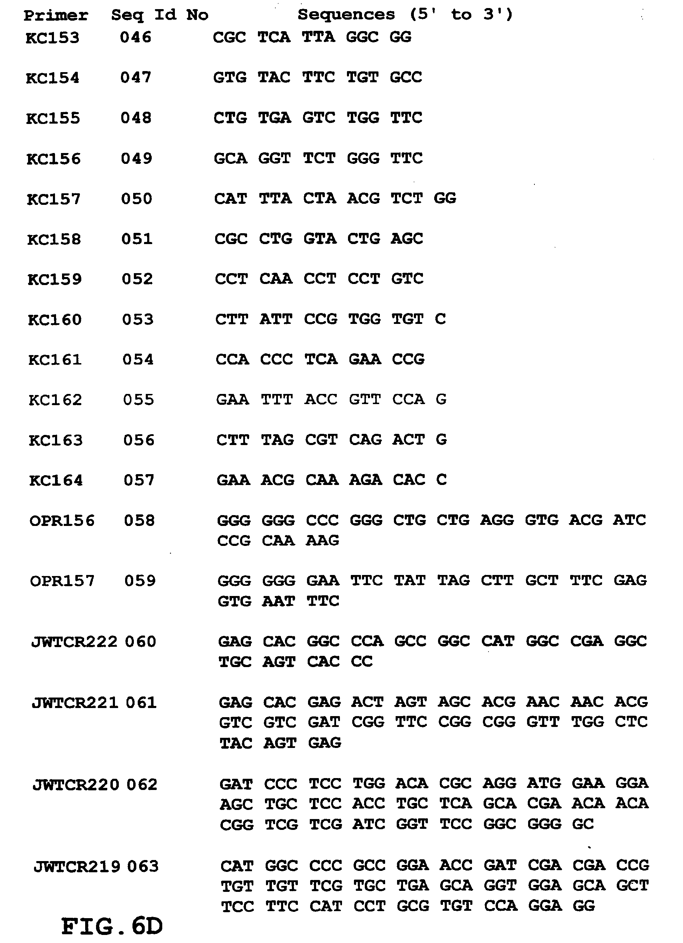

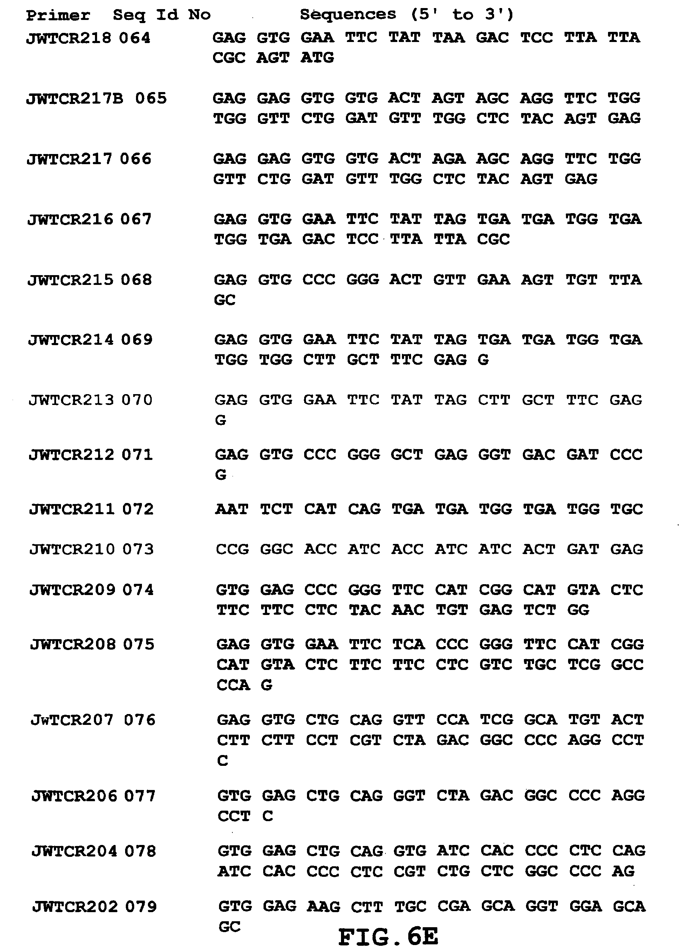

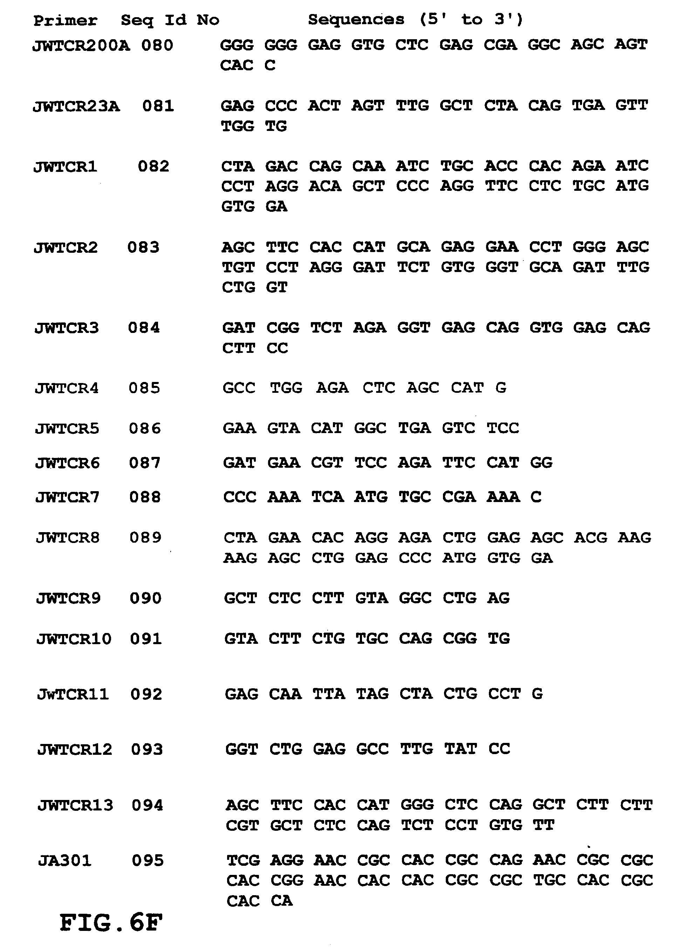

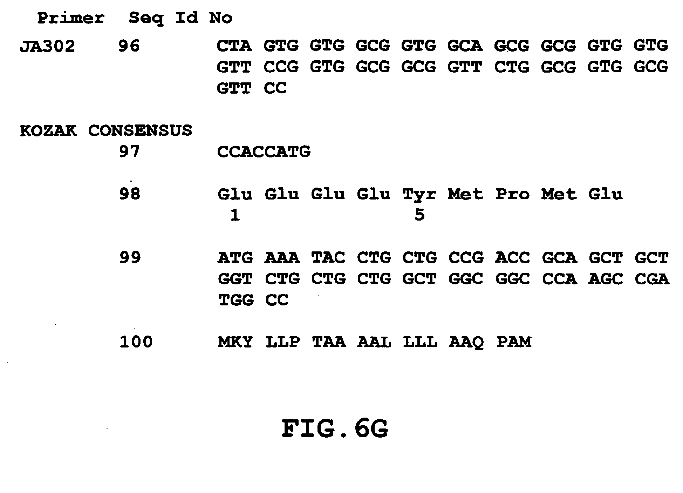

Fig. 6 (6A-6G) are tables showing DNA oligonucleotide primers used to construct DNA vectors described below. Fig. 6A (SEQ ID NOs.: 1-16), Fig. 6B (SEQ ID NOs: 17-30), Fig. 6C (SEQ ID NOs: 31-45), Fig. 6D (SEQ ID NOs: 46-63), Fig. 6E (SEQ ID NOs: 64-79), Fig. 6F (SEQ ID NOs: 80-95), Fig. 6G (SEQ ID NOs: 96-100).
Fig. 7 is a table showing DNA oligonucleotide primers used to construct V-β chains of sc-TCR fusion proteins (SEQ ID NOs: 101-115).
Fig. 8 is a table showing DNA oligonucleotide primers used to construct V-β chains of sc-TCR fusion proteins (SEQ ID NOs: 116-130).
Figs. 9A and 9B are drawings showing inserts of (9A) pKC60-m, pKC67-m, PKC73-M, pKC74-m, pKC75-m, and (9B) pNAG-1-m and pNAG3-m.
Fig. 10 is a drawing showing a mammalian DNA expression vector comprising a sc-TCR fusion protein. A DNA insert encoding an sc-TCR ("sc-TCR variant") is fused to the mouse kappa intron and mouse kappa exon.

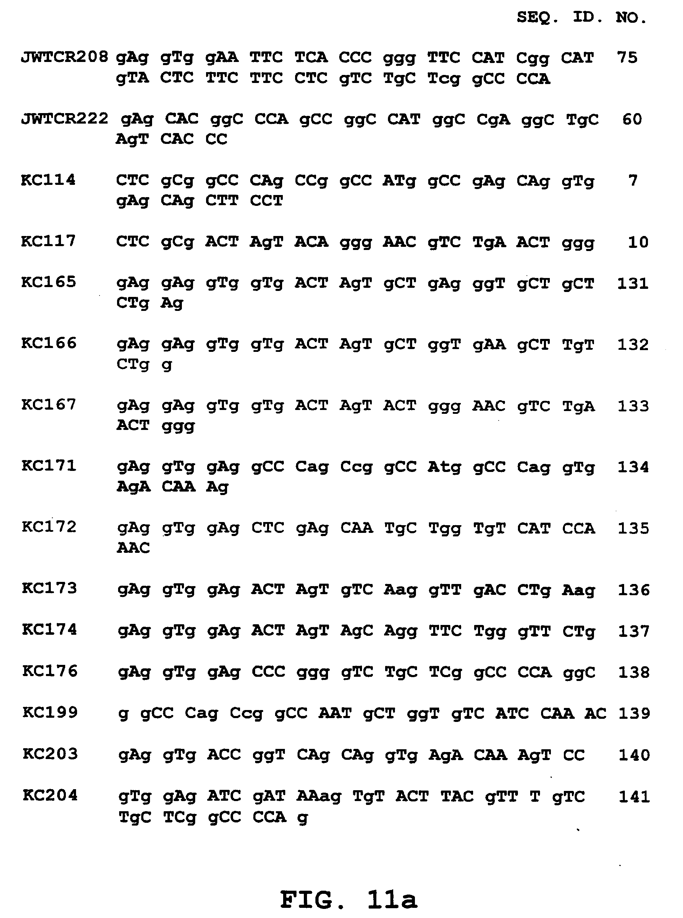

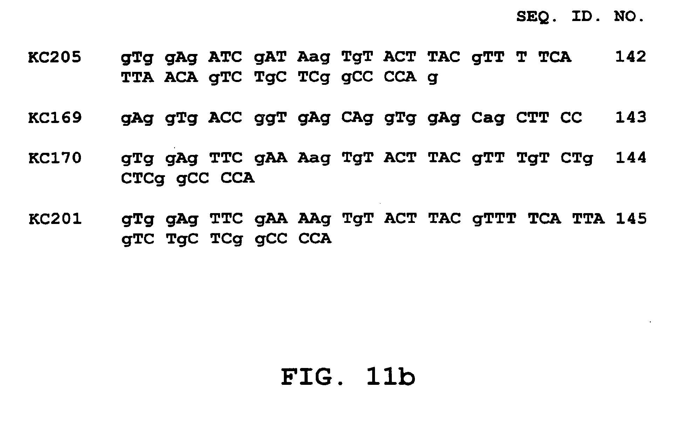

Fig. 11 (includes Figs. 11a and 11b)is a table showing DNA oligonucleotide primers used to construct sc-TCR fusion proteins (SEQ. ID NOs. 7, 10, 60, 75, 131-145).
Fig. 12 is a Western blot showing sc-TCR (D011.10) kappa fusion protein produced in chinese hamster ovary (CHO) cells.
Fig. 13 is a Western blot of purified sc-TCR (D011.10) kappa fusion protein.
Fig. 14 is an SDS-PAGE gel stained with Commassie Brilliant Blue illustrating staining of purified sc-TCR kappa fusion protein.
Fig. 15 is a Western blot showing transient expression of a three-domain scTCR-kappa fusion protein and a four-domain sc-TCR kappa fusion protein.
Fig. 16 is a graph of an ELISA assay showing transient expression of sc-TCR (D011.10) kappa fusion protein varients.
Fig. 17 is a graph of a sandwich ELISA assay depicting mammalian cell expression of scTCR (p-149) kappa fusion protein.
Fig. 18 is a graph of a sandwich ELISA assay depicting transient mammalian cell expression of a p149 sc-TCR and p149 sc-TCR kappa fusion protein.

### DETAILED DESCRIPTION OF THE INVENTION

As summarized above, we have found that it is possible to make and use fully soluble and functional sc-TCR proteins. For example, it is possible to facilitate soluble expression of sc-TCR molecules by covalently linking an Ig-C_{L} chain or suitable Ig-C_{L} fragment thereto. Generally stated, the sc-TCR proteins include a V-α chain covalently linked to a V-β chain by a single-chain peptide linker sequence. The sc-TCR fusion proteins include an Ig-C_{L} chain or suitable chain fragment fused to the V-α or the V-β chain. Fusion of the Ig-C_{L} chain or fragment enhances soluble expression of fully functional sc-TCR in a variety of cells.

In general, preparation of the present sc-TCR proteins can be accomplished by procedures disclosed herein and by recognized recombinant DNA techniques. For example, preparation of plasmid DNA, DNA cleavage with restriction enzymes, ligation of DNA, introduction of DNA into a cell, culturing the cell, and isolation and purification of the expressed protein are known techniques. See generally Sambrook et al. in Molecular Cloning: A Laboratory Manual (2d ed. 1989); and Ausubel et al. (1989), Current Protocols in Molecular Biology, John Wiley & Sons, New York.

The general structure of sc-TCR molecules and methods of making and using same have been disclosed in the pending U.S. application No. 08/813,781.

Briefly stated, the sc-TCR molecule includes V-α and V-β chains covalently linked through a suitable peptide linker sequence. For example, the V-α chain can be covalently linked to the V-β chain through a suitable peptide linker sequence fused to the C-terminus of the V-α chain and the N-terminus of the V-β chain. The V-α and V-β chains of the sc-TCR fusion protein are generally about 200 to 400 amino acids in length, preferably about 300 to 350 amino acids in length, and will be at least 90% identical, and preferably 100% identical to the V-α and V-β chains of a naturally-occurring TCR. By the term "identical" is meant that the amino acids of the V-α or V-β chain are 100% homologous to the corresponding naturally-occurring TCR V-β or V-α chains.

The V-α chain of the sc-TCR molecule further includes a C-β chain or fragment thereof having a length of approximately 50 to 126 amino acids fused to the C-terminus of the V-β chain. Further, the V-α chain can include a C-α chain or fragment thereof fused to the G-terminus of the V-α chain and the N-terminus of the peptide linker sequence. Generally, in those fusion proteins including a C-β chain fragment, the fragment will usually not include the last cysteine residue at position 127. For those fusion proteins comprising a C-α chain, the length can vary between approximately 1 to 90 amino acids (ie. the C-α chain up to but not including the fmal cysteine). For example, in one embodiment, the fusion protein includes a C-α chain fragment between about 1 to 72 amino acids starting from amino acid 1 to 72. In another embodiment, the C-α chain fragment is between about 1 to 22 amino acids starting from the first amino acid to 22 (leucine). The C-α chain fragment typically does not include any cysteine resides except the C_{∝90} variant which includes two cys residues. To facilitate expression of fully soluble and functional protein, the Ig-C_{L} chain or suitable Ig-C_{L} fragment is covalently linked to the sc-TCR molecule, e.g., to the C-terminus of the C-β chain or C-β fragment. Although typically not preferred, it is possible to covalently link the Ig-C_{L} or fragment thereof to the N- terminus of the V-α chain. In most cases, choice of Cα and Cβ chain length will be guided by several parameters including the particular V chains selected and intended use of the soluble fusion molecule.

Additional sc-TCR proteins of the invention include e.g., two peptide linker sequences, where the first peptide linker sequence is fused between the C-terminus of the V-α chain and the N-terminus of the V-β chain. The C-terminus of the V-β chain can be fused to the N-terminus of a C-β chain fragment. The second peptide linker is then fused to the C-terminus of the V-β chain or C-β chain fragment and the N-terminus e.g., of an Ig-C_{L} chain, or Ig-C_{L} chain fragment or an effector or tag molecule.

In other illustrative embodiments, sc-TCR proteins can be made by fusing the V-β chain to the V-α chain through a suitable peptide linker in which the C-terminus of the V-β chain or C-β chain fragment thereof and the N-terminus of the V-α chain are covalently linked. An Ig-C_{L} chain or fragment can be covalently linked to the C- or N-terminus of the molecule as desired although generally linkage at the C-terminus will be preferred.

An scTCR protein according to the invention can include one or more fused protein tags. For example, with respect to a soluble fusion protein, a protein tag can be fused to the C-terminus of the sc-TCR V-β chain (or C-β chain fragment) and the N-terminus of the Ig-C_{L} chain or Ig-C_{L} chain fragment. The sc-TCR fusion protein can thus include, e.g., two protein tags in which the first tag is fused the C-terminus of the V-β chain (or C-β chain fragment) and the N-terminus of the Ig-C_{L} chain or fragment, and the second protein tag (the same or different) is fused to the C-terminus of the Ig-C_{L} chain or fragment. Alternatively, a single protein tag can be fused to the C-terminus of the Ig-C_{L} chain or fragment.

Other sc-TCR fusion proteins are contemplated such as fusions comprising one or more protein tag fused to the N-terminus of the V-α chain.

The peptide linker sequence of the sc-TCR proteins are typically selected so that the sc-TCR molecule forms a binding site which resembles that of a naturally-occurring TCR V-α and V-β chain. The Vα and Vβ chains can be derived from uninduced T-cells although in most cases the V chains are those associated with a pathology, e.g., an immune-related disorder or disease.

More particularly, the peptide linker sequence separating the Vα,β chains flexibly positions the V-chains in a pocket that is capable of specifically binding a ligand such as a desired antigen. For example, ligand binding to the sc-TCR fusion protein can be used to modulate T-cell activity as determined by assays described below. Exemplary of such assays include *in vitro* assays involving sequential steps of culturing T-cells expressing a TCR, contacting the T-cells with the sc-TCR protein (or sc-TCR obtained therefrom) under conditions which allow binding between the TCR and the ligand, and then evaluating whether the soluble fusion protein is capable of modulating activity of the T-cells. Fusion of Ig-C_{L} chain or Ig-C_{L} chain fragment to the sc-TCR molecule can increase assay performance in some settings, e.g., by facilitating soluble expression of the sc-TCRs and maintaining sc-TCR integrity in aqeuous solutions such as cell media.

As discussed above, an sc-TCR fusion protein of the invention includes a covalently linked Ig-C_{L} chain or suitable Ig-C_{L} chain fragment linked, e.g., to the C-terminus of the sc-TCR molecule. In one embodiment, the sc-TCR fusion protein includes a fused mammalian Ig-C_{L} chain, preferably a full-length murine or human Ig-C_{L} chain such as the Cκ chain. The nucleic acid and protein sequences of several Ig-C_{L} chains have been disclosed. See, e.g., Fundamental Immunology, (1993) 3rd Edi. W. Paul. Ed. Rsen Press Ltd. New York; and Kabat, E.A., et al., (1991) Sequences of Proteins of Immunological Interest (5th Ed.) Public Health Services, National Institutes of Health.

The term Ig-C_{L} chain is also meant to include an immunoglobin light chain constant region which varies from a disclosed full-length sequence by one or more amino acid substitutions or additions. For example, an amino acid can be added to a disclosed Ig-C_{L} chain sequence at one or both ends of the chain, e.g., by conventional recombinant methods. In addition, the recombinant methods can be used to substitute a given amino acid in the chain if desired. Generally, the amino acid addition will include between about 1 to 30 neutral or hydrophillic amino acids, preferably between about 1 to 10 of such amino acids. An amino acid substituted for another amino acid in the Ig-C_{L} chain will be a conservative or non-conservative amino acid replacement. Accordingly, a tyrosine amino acid in a Ig-C _{L} chain sequence substituted with a phenylalanine will be an example of a conservative amino acid substitution, whereas an arginine replaced with an alanine would represent a non-conservative amino acid substitution. As will be pointed out below for Ig-C _{L} chain fragments, a sc-TCR fusion protein comprising the fused Ig-C _{L} chain will be fully soluble and functional.

In addition, amino acid substitutions or additions to preferred Ig-C_{L} chain sequence specifically disclosed below are within the scope of the present invention.

In some instances, it will be useful to use an Ig-C_{L} chain fragment such as a murine or human Cκ chain fragment. For example, a suitable Ig-C_{L} chain fragment can be fused to a sc-TCR molecule when it is desirable to minimize moleular weight of the fusion protein. By the phrase "suitable Ig-C_{L} chain fragment" or related term is meant an Ig-C_{L} fragment which when fused to a desired sc-TCR molecule forms a fully soluble and functional sc-TCR fusion protein as defined below. A desired Ig-C_{L} chain fragment (both Cκ and Cλ type) can be made according to standard recombinant methods, e.g., by PCR amplification of a murine or human Cκ or Cλ chain fragment followed by ligation of the PCR product to the DNA segment or vector encoding a desired sc-TCR molecule. As noted below, the PCR product can be manipulated to include restriction enzyme cleavage sites to facilitate cloning. Generally, a suitable murine or human Cκ chain fragment will be between about 70 to 150, preferably between about 90 to 120, and more preferably between about 100 to 110 amino acids in length. Examples of suitable murine or human Cκ chain DNA sequences are disclosed below. See Examples 5, 6 and 7 below.

The sc-TCR proteins of the invention are fully functional and soluble. By the term "fully functional" or similar term is meant that the fusion protein specifically binds ligand. Assays for detecting such specific binding are disclosed herein and include standard immunoblot techniques such as Western blotting.

By the term "fully soluble" or similar term is meant that the fusion protein is not readily sedimented under low G-force centrifugation from an aqeuous buffer e.g., cell media. Further, the sc-TCR fusion protein is soluble if the fusion protein remains in aqueous solution at a temperature greater than about 5-37° C and at or near neutral pH in the presence of low or no concentration of an anionic or non-ionic detergent. Under these conditions, a soluble protein will often have a low sedimentation value e.g., less than about 10 to 50 svedberg units. Aqeuous solutions referenced herein typically have a buffering compound to establish pH, typically within a pH range of about 5-9, and an ionic strength range between about 2 mM and 500 mM. Sometimes a protease inhibitor or mild non-ionic detergent is added and a carrier protein may be added if desired such as bovine serum albumin (BSA) to a few mg/ml. Exemplary aqueous buffers include standard phosphate buffered saline, tris-buffered saline, or other known buffers and cell media formulations.

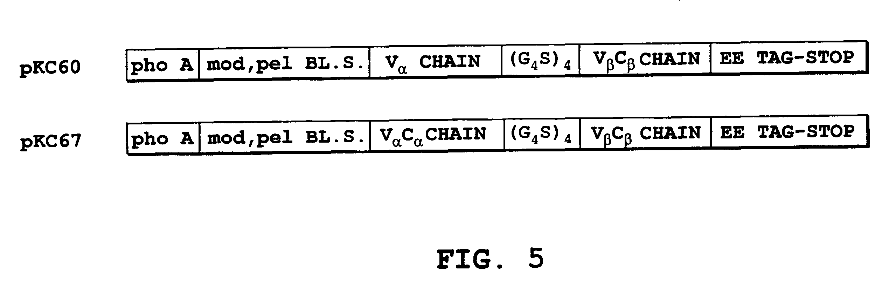

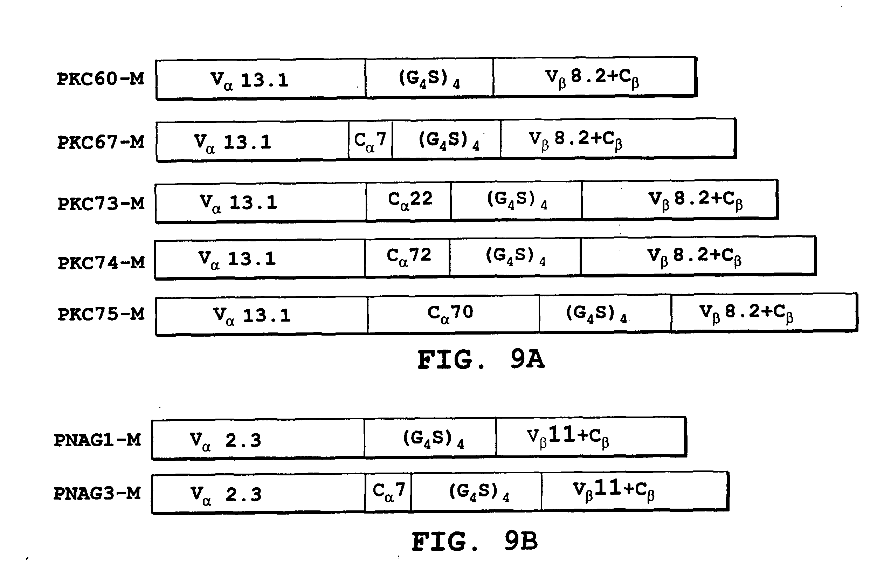

As noted above, it has been found that many sc-TCR molecules are fully functional and soluble when expressed in mammalian cells without a fused Ig-C_{L} chain or fragment thereof. For example, the pNAG3-m and p149-m vectors illustrated in Fig. 9B below encode an sc-TCR molecule that is fully functional and soluble when expressed in mammalian cells specified below. The sc-TCR molecule encoded by the pNAG3 -m vector includes covalently linked in sequence: a Vα chain, Cα chain, peptide linker sequence, Vβ chain and a Cβ chain. Further disclosed are sc-TCR molecules comprising amino acid deletions in the C a chain up to the entire length of the C a chain. See Figs. 9A and 9B below and the pKC60 vector illustrated in Fig. 5. In addition, the pending U.S. Application No. O8/813,781 discloses methods of making sc-TCR molecules including covalently linked in sequence: a Vα chain, Cα chain fragment, peptide linker sequence, and a Vβ chain. Accordingly, it is possible to make a variety of sc-TCR molecules comprising modified (or deleted) Cα and Cβ chains without fusing the Ig-C_{L} chain or suitable fragment to the molecule. The sc-TCR molecules can be tested in accordance with the methods described herein for soluble expression in specified mammalian cells.

A sc-TCR protein is usually encoded by a nucleic acid segment which can be RNA, mRNA, cDNA or genomic DNA. Typically, the nucleic acid segment will be a DNA segment. For example, a DNA segment according to the invention typically includes an operably linked leader sequence to provide appropriate cell processing signals. In general, the leader sequence is fused to the 5' end of the DNA sequence encoding the sc-TCR molecule. For example, the leader can be covalently linked to the 5' end of the DNA sequence encoding the V-α chain, or in some embodiments, the V-β chain. It will be recognized however that although a specific leader sequence is linked to a particular a or β chain, leader sequences can often be exchanged using recombinant techniques without a detrimental effect on the processing of the fusion protein. Thus in one embodiment, the 5' end of the V-α chain is covalently linked to the 3' end of a suitable leader sequence. The leader sequence will often be between approximately 12 to 26 amino acid residues in length. A DNA segment designed for insertion into a bacterial expression vector can include a Pel B sequence whereas those DNA segments for insertion into vectors designed for expression in mammalian cells will include an Ig-C_{L} leader such as a mammalian Cκ leader sequence. An exemplary Cκ leader is provided below.

By the term "operably linked" is meant a genetic sequence operationally (i.e., functionally) linked to a nucleic acid segment, or sequences upstream (5') or downstream (3') from a given segment or sequence. Those nearby sequences often positively impact processing and/ or expression of the nucleic acid segment or sequence in a desired cell type.

A DNA vector according to the invention used for bacterial expression and can include a promoter such as the trp operon promoter, lac promoter, trp-lac promoter, lac^{uvs} or phoA promoter. Exemplary promoters are those such as phoA which provide strong, regulated expression during slow induction conditions lasting about several hours (e.g., 2 to 10 hours). Under suitable culture conditions, most strong promoters are capable of providing soluble fusion protein at levels up to and exceeding approximately 10% of the total host cell protein.

The single-chain V regions of the soluble fusion proteins can be derived from nearly any TCR V region. Generally, suitable Vα, β chains will be those for which there is an increase in gene expression following immunological induction. Methods for assaying an increase in TCR V chain expression are known (see e.g, Hafler, D.A. et al. J. Exp. Med. 167: 1313 (1988); Mantgazza R., et al. Autoimmunity 3, 431 (1990)).

The sc-TCR fusion proteins can comprise Vα,β chains for which a substantially full-length coding sequence is readily available. Methods for obtaining full-length TCR V chain sequences from cell sources are well known. Alternatively, the Vα,β chains regions can be obtained by PCR amplication of publicly available Vα, β chains for which at least a portion of the sequence is known. Exemplary Vβ gene sequences include V β 8.1, V β 6.1, V β 5.1, V β 5.2, V β 5.3, V β 2.1, and V β 2.3 gene sequences. See Abe et al. (1992) PNAS (USA) 89: 4066; Wang, et al., 1993); PNAS (USA) 90: 188; Lahesma et al. (1993) J. Immunol. 150: 4125; Kotzin, et al., (1991) PNAS (USA) 88: 9161; Uematsu, et al. (1991) PNAS (USA) 88: 8534. See also, Kabat, E.A., et al. *supra* and Chotia, C. et al., (1988) EMBO J. 7:3745 for additional TCR V chain sequence.

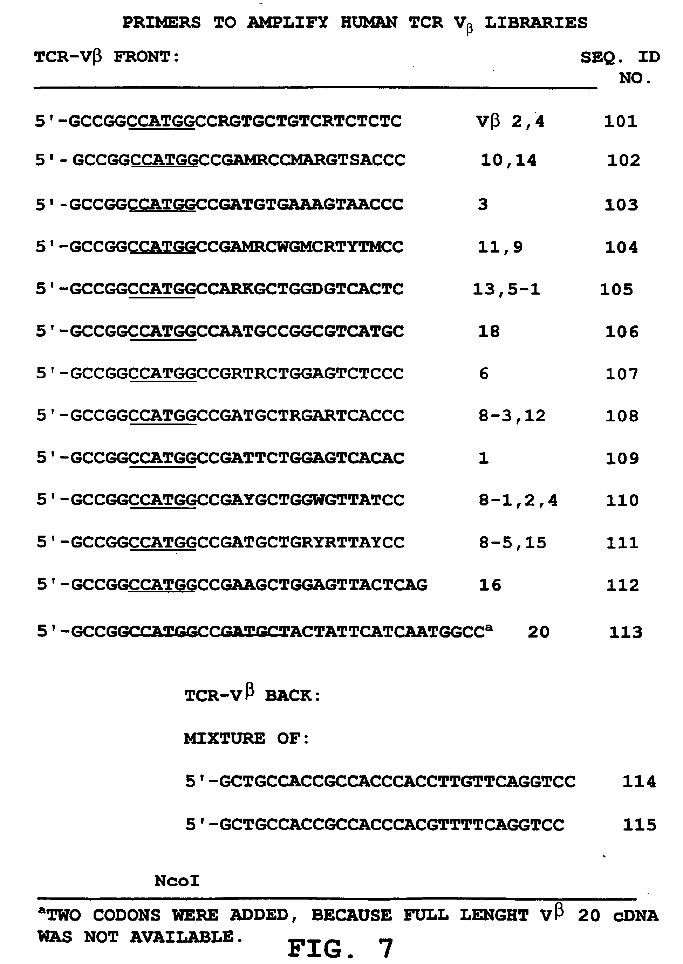

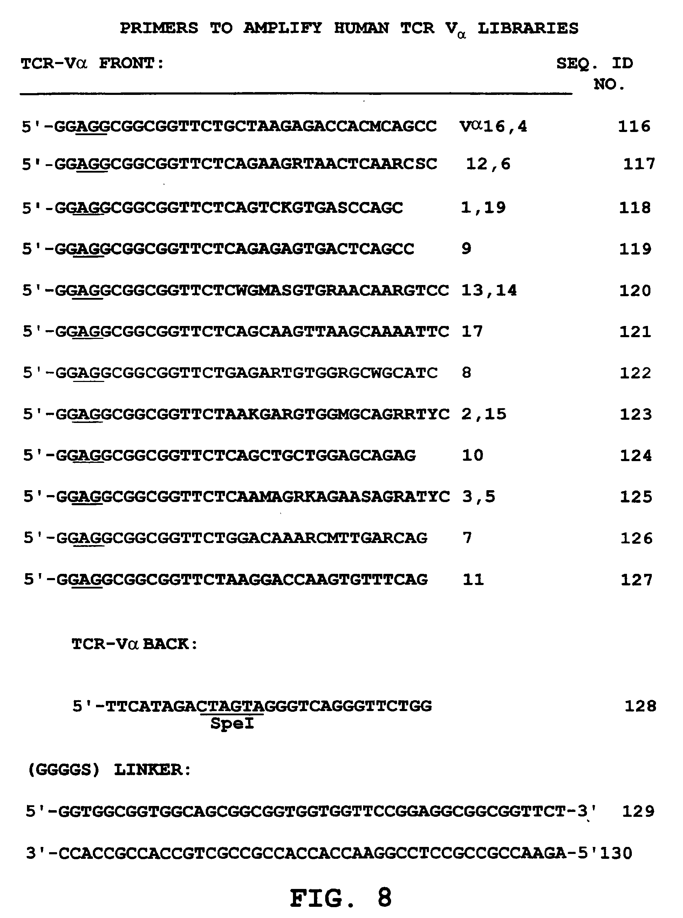

Additionally, Example 3 below provides oligonucleotide primers for PCR amplifying a variety of V-α and V-β chains. See also, Figs. 7 and 8 below for additional examples of suitable oligonucleotide primers.

In cases where it is desired to obtain TCR V chains from a biological source, a desired TCR can be identified by conventional immunological methods including use of TCR-specific antibodies which predominantly bind and preferably are specific for an epitope of the TCR V region. Typically, surface expression can be detected by using known techniques such as fluorescence microscopy, flow cytometry, or immunochemistry. A number of antibodies which specifically bind TCR variable regions are known. See e.g, published PCT application WO 90/06758.

The DNA or RNA of the detected TCR can be probed directly, or preferably after PCR amplification, by specific hybridization with oligonucleotide probes for the various TCR gene families, using hybridization methods well-known in the field. Generally, high stringency nucleic acid hybridization conditions will be preformed. As used herein the term "high stringency hybridization" means nucleic acid incubation conditions approximately 65°C in 0.1 x SSC. See Sambrook, et al., *supra.* The TCR DNA sequence or desired portion thereof can be obtained directly from the amplified DNA or RNA and can be subcloned into a suitable vector as desired.

Other methods are known for obtaining TCR V region DNA. For example, a desired TCR comprising V region genes can be identified by sequencing the TCR or preferably a portion thereof corresponding to the V region. The DNA sequence can be determined, e.g., after cloning DNA into a suitable sequencing vector as are known in the field or by first determining the protein sequence of at least part of the TCR and determining the DNA sequence. It is readily apparent to those skilled in this field that the above-mentioned manipulations as well as others known to the artisan can be employed to successfully identify a desired TCR and to obtain the V region genes from that TCR so that a single-chain Vαβ construct can be made for fusion to a desired Ig-C_{L} chain or suitable Ig-C_{L} fragment.

More specifically, when it is desired to obtain TCR V region DNA from a biological source, a DNA segment encoding the desired V-α and V-β chain can be obtained from cells such as T-cell hybridomas or cytotoxic T-cells (CTLs). The T-cells (e.g., Ts, Tc or T_{H} cells) can be obtained *in vivo,* or the T-cells can be cultured T-cell hybridoma(s) (e.g., D10 or B12 cell lines). See Example 1 which follows. CTLs can be uninduced or can be associated with a pathogenic immune system response in a rodent (e.g., mouse, rat, rabbit) or primate (e.g. human or chimpanzee). For example, CTLs or other T-cells can be derived from patients suffering from or suspected of having Lyme disease, Kawasaki disease, leprosy, cancer (i.e. immune responses against tumor associated antigens such as CEA), or an autoimmune disorder, particularly those associated with transplantation rejection, multiple sclerosis, insulin dependent diabetes, rheumatoid arthritis, and allergies; or an infectious disease, particularly an infectious disease involving an RNA or DNA virus. Particular viruses of interest include the human immunodeficiency viruses (HIV), cytomeglovirus (CMV), influenza, hepatitis, pox virus, Epstein Barr, adenovirus or polyoma viruses. Exemplary sources of CTLs are antigen-specific CTLs and TILs isolated from patients with established carcinomas and melanomas (see e.g., Cox A. et al. Science (1994) 264: 716; Rosenberg, S.A. et al. N. Eng. J. Med. (1988) 319: 1676; Kawakami, Y. et al., J. Exp. Med. (1994) 180: 347); Kawakami, Y. et al. PNAS (1994) 91:6458).

As mentioned previously with respect to obtaining V-α and V-β chains from cell sources, several alternative procedures can be used to prepare nucleic acids isolated therefrom. More particularly, to prepare V-α and V-β chain DNA, mRNA is isolated from those cells demonstrating a desired TCR binding specificity. Such methods generally include use of a suitable PCR protocol using first-strand cDNA template made from the mRNA. Standard recombinant techniques can then be employed to make the desired a and β chains. The DNA segment encoding the desired V-α and V-β chains is then modified to include a suitable peptide linker sequence and protein tag(s), if desired.

Generally, a DNA oligonucleotide primer for use in the PCR methods will be between approximately 12 to 50 nucleotides in length preferably approximately 20-25 nucleotides in length. The PCR oligonucleotide primers may suitably include restriction sites to add specific restriction enzyme cleavage sites to the PCR product as needed, e.g., to introduce a ligation site. Exemplary primers are provided in the Examples and Drawings which follow. The PCR products produced will include amplified V-α and V-β chain sequences and can be modified to include, as desired, ribosome binding, leader and promoter sequences for optimal expression of the fusion protein.

A DNA segment encoding a desired sc-TCR molecule can be made in significant quantities (milligram quantities per gram cells) in accord with methods disclosed below.

Particular sc-TCRs used to make the soluble fusion proteins provided below include those sc-TCRs with V-α and V-β chains derived from a mammal. Examples include primates, particularly human and chimpanzees; rodents, e.g., immunologically naive mice such as nude mice or mice which include a transgene capable of expressing an HLA-A2 antigen complex (Vitiello, A. et al., J. Exp. Med., (1991) 175, 1002). Particular humans of interest include those suffering from any of the previously mentioned pathologies, such as an autoimmune disorder. Chimeric constructs comprising V-α and V-β DNA sequences derived from different mammals can be constructed in accordance with known methods and are also within the scope of the present invention.

As mentioned above, the peptide linker sequences effectively position the V-α and V-β chains of the fusion protein to form a ligand binding pocket. The soluble fusion protein is thus capable of specifically binding a ligand such as a superantigen, or peptide antigen in the context of an MHC/HLA peptide complex, or small molecule. Particularly, it is an object of the present invention to provide soluble and fully functional sc-TCR fusion molecules that can compete with naturally-occurring TCRs on the surface of T-cells. By the word "compete" is meant that the soluble fusion protein is able to bind the ligand at a level which is equal to, or in some instances exceeds the specific binding affinity of the TCR for the same ligand.For example, in accordance with methods described below, the sc-TCR fusion protein (or sc-TCR molecule derived therefrom) can exhibit a binding affinity which is about equal or up to approximately 2 to 10 fold higher than the naturally-occurring TCR.

Typically, fusion of a desired Ig-C_{L} chain or suitalbe Ig-C_{L} chain fragment will not reduce specific binding of the sc-TCR fusion protein by more than 30%, preferably not more than 10% and more preferably not more than 5% or less when compared to a sc-TCR molecule lacking a fused I_{g}-C_{L} chain or fragment thereof. Examplary binding assays are disclosed herein and include standard Western blotting assays and surface plasmon resonance assays disclosed below.

In an illustrative embodiment of the present invention, the polypeptide linker sequence comprises from about 7 to 20 amino acids, more preferably from about 10 to 20 amino acids, still more preferably from about 12 to 20 amino acids. The linker sequence is typically flexibly disposed in the fusion protein so as to position the V-α and V-β chains in a configuration which provides for specific binding of a desired ligand such as a peptide antigen. The linker preferably predominantly comprises amino acids with small side chains, such as glycine, alanine and serine, to provide optimal flexibility. Preferably, about 80 or 90 percent or greater of the linker sequence comprises glycine, alanine or serine residues, particularly glycine and serine residues. Preferably, the linker sequence does not contain any proline residues, which could inhibit flexibility. The linker sequence is suitably attached to the C-terminus of the V-α chain and the N-terminus of the V-β chain of a fusion protein. See, Examples 1 and 2 below.

More specifically, suitable linker sequences include the (GGGGS)4 sequence (i.e., Gly Gly Gly Gly Ser)4 are JA302 (SEQ ID NO: 96) and JA301 (SEQ ID NO: 95). Preferably, a selected linker sequence is covalently linked between the C-terminal residue of the V-α chain, and the first amino acid of the V-β chain of the sc-TCR. However, as mentioned previously, other peptide linker sequence configurations are possible, some of which include fusion to the Ig-C_{L} chain or suitable Ig-C_{L} chain fragment such as a Cκ chain fragment. Several polypeptide linker sequences have been disclosed as being acceptable for use in joining antibody variable regions (see M. Whitlow et al., Methods: A Companion to Methods in Enzymology, 2:97-105 (1991)). Alternatively, other suitable linker sequences can be readily identified empirically. For example, a DNA vector including a DNA segment encoding a fusion protein that includes the linker sequence can be cloned and expressed, and the fusion molecule tested to determine if the molecule is capable of binding antigen. An exemplary assay is a conventional antigen binding assay such as those disclosed in Harlow and Lane, *supra.* Alternatively, the expressed fusion protein comprising the linker sequence can be tested for capacity to modulate the activity of a T-cell as determined by assays referenced herein See Examples 8, 10 and 11 below. Suitable size and sequences of linker sequences also can be determined by conventional computer modeling techniques based on the predicted size and shape of the fusion protein. Exemplary peptide linker sequences are those which include suitable restriction sites (e.g. XhoI and SpeI) at the ends of the polypeptide linker sequence between the V a and V-β chains.

In most cases, a DNA segment encoding a sc-TCR molecule of interest will be recombinantly engineered into an appropriate DNA vector. For example, in embodiments where the desired V chain is PCR-amplified, the oligonucleotide primers are usually configured with desired restriction sites on both ends of the primers so that the DNA segment can be replaced with another desired DNA segment. Thus, a suitable DNA vector of the invention is one in which the desired sc-TCR molecule can be readily inserted in the vector. Sometimes, as when a soluble fusion molecule is to be made, the Ig-C_{L} chain or suitable Ig-C_{L} fragment will be encoded by the vector and will be fused to the DNA segment by ligation. In other cases, the Ig-C_{L} chain or the fragment will be fused to the DNA segment prior to the ligation to the vector.

The term "vector" as used herein means any nucleic acid sequence of interest capable of being incorporated into a host cell resulting in the expression of a nucleic acid segment of interest such as those segments or sequences encoding the sc-TCR fusion proteins described above. The vectors can include e.g., linear nucleic acid segments or sequences, plasmids, cosmids, phagmids and extra chromosomal DNA. Specifically, the vector can be recombinant DNA. Also used herein the term "expression," or "gene expression", is meant to refer to the production of the protein product of the nucleic acid sequence of interest including transcription of the DNA and translation of the RNA transcription. Typically, a DNA segment encoding an sc-TCR fusion protein of the invention is inserted into the vector, preferably a DNA vector, to replicate the DNA segment in a suitable host cell.

A number of strategies can be employed to express the sc-TCR proteins of the invention in desired cells. For example, the DNA sequence encoding the sc-TCR protein can be incorporated into a DNA vector by known means such as by use of enzymes to restrict the vector at pre-determined sites, followed by ligation of the DNA into the vector. The vector containing the DNA sequence is then introduced into a suitable host for soluble expression of the sc-TCR protein. Selection of suitable vectors can be empirically based on factors relating to the cloning protocol. For example, the vector should be compatible with, and have the proper replicon for the host cell that is being employed. Further, the vector must be able to accommodate the DNA sequence coding for the protein that is to be expressed. Preferred vectors are those capable of expressing the soluble proteins in mammalian cells e.g., pCDNA3 available from in Vitrogen. See also Sambrook et al., *supra* and Ausubel et al. *supra* for other suitable vectors for use in, mammalian, cells. Typically, DNA vectors designed for expression in bacteria and encoding soluble fusion proteins will not include a full-length Cλ or Cκ intron although these sequences can be included in vectors designed for expression in mammalian cells capable of RNA splicing.

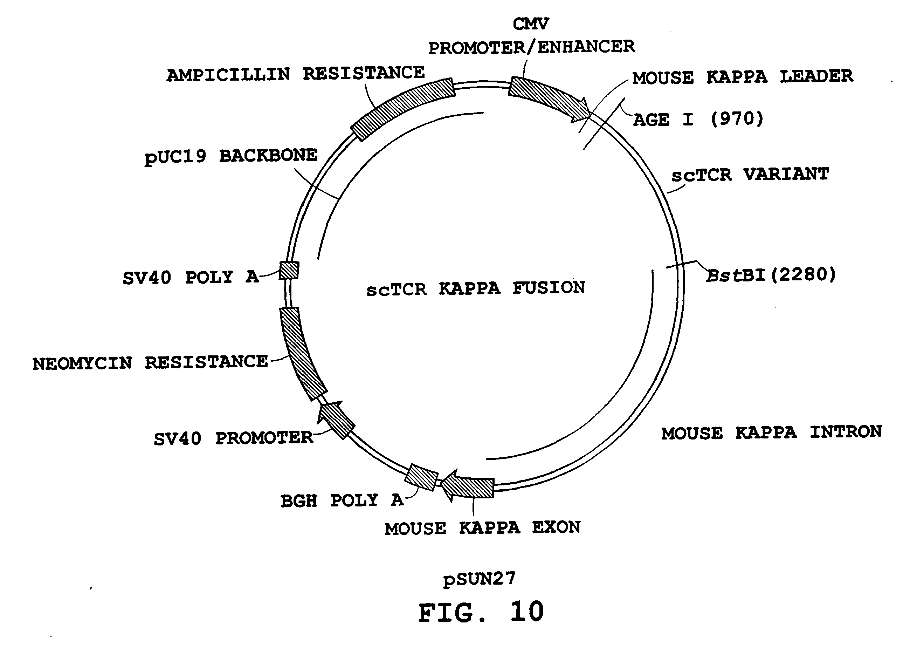

Preferred DNA vectors are designed to express the soluble sc-TCR in eukaryotic cells, particularly mammalial cells. The DNA vectors can be formatted for replication in a bacterial host if desired so that suitable amounts of the DNA vector can be obtained. For example, a DNA vector can generally include (i) the origin of replication functional in *E. coli*; (ii) the selectable antibiotic resistance gene; (iii) a strong viral promoter such as the cytomeglovirus (CMV) promoter and optional CMV enhancer element, (iv) an Ig-C_{L} leader sequence, (v) a sc-TCR molecule of interest, (vi) a full-length Ig-C_{L} intron linked to an Ig-C_{L} exon, (vii) a growth hormone polyadenlyation sequence, e.g., bovine growth hormone (bgh) poly A sequence and (viii) DNA encoding a selectable eukaryotic marker such as a strong viral promoter (e.g., simian virus 40 (SV40) promoter) linked to an antibiotic resistance gene (e.g. neomycin) and fused to a viral polyadenlyation sequence (e.g., the SV40 polyA sequence). Alternatively, the DNA vector can include all of (i)-(v), and (vii)-(viii), above, without the full-length Ig-C_{L} intron linked to the Ig-C_{L} exon of (vi). An exemplary Ig-C_{L} leader sequence is the mouse kappa leader. An example of a full-length Ig-C_{L} intron and exon is the full-length Cκ gene. An example of a suitable DNA vector for mammalian cell expression is illustrated in Fig. 10 (pSUN27 vector). See Example 5 below.

A DNA vector of the invention for use in a desired mammalian cell can be modified according to conventional techniques to optimize soluble expression in other mammalian cells. For example, the eukaryotic marker encoding the neomycin resistance gene described above can be replaced by DNA encoding the thymidine kinase (TK) gene to facilitate expression of the sc-TCR fusion protein in TK- (TK deficient) mammalian cells. The DNA vector can be modified in other ways well-known in the art (e.g., changing promoters, antibiotic resistance genes, replacing the CMV promoter with a promoter obtained from an immunoglobin, SV40, an adenovirus or papilloma virus promoter to optimize sc-TCR fusion protein expression in a desired mammalian cell. Alternatively, the DNA sequence encoding the sc-TCR protein can be inserted into well-known vectors suitable for expression in yeast or insect cells, as desired. See e.g. Ausubel, et al., *supra* and Summer and Smith, *supra..*

Suitable host cells can be transformed by a variety of methods including retroviral transfer, viral or bacteriophage infection, calcium- , liposome-, or polybrene- mediated transfection, biolistic transfer, or other such techniques known in the art.

As noted previously, in some cases it may be desirable to express the soluble fusion protein in non-mammalian cells. For example, suitable host cells for expressing the fusion proteins in bacteria include cells capable of being readily transformed and exhibiting rapid growth in culture medium. Particularly preferred hosts cells include *E. coli, Bacillus subtillus,* etc. Other host cells include, yeasts, e.g., *S*. *cerevisiae* and insect cells. Exemplary cells for insect cell expression are those capable of being infected by a baculovirus such as Sf9 cells. See also, Summer and Smith (1988) A Manual of Methods for Baclovirus Vectors and Insect Cell Culture Procedures, Texas Agricultural Expreimental Station Bulletin No. 1555, College Station, Texas.

In general, cell culturing conditions are employed in which stably transformed or transfected cell lines are selected e.g., by incorporation of a suitable cell selection marker into the vector (e.g., an antibiotic resistance gene or G418). Cells which express the sc-TCR fusion protein can be determined by known procedures e.g., ELISA assay using commercially available monoclonal antibodies which specifically bind the V-α or V-β chain. Alternatively a monoclonal antibody can be chosen which specifically binds the Cκ or Cλ chain (or fragment) of the soluble fusion protein. Examples of monoclonal antibodies and suitable assays are provided in the examples below.

A vector designed for replication and expression of a desired soluble fusion protein in bacteria includes e.g., (i) an origin of replication functional in *E. coli* and derived e.g., from pBR322, preferably from well-known pUC19 vectors; (ii) a selectable antibiotic resistance gene, e.g., ampicillin and/or neomycin resistance gene; (iii) a transcriptional termination region, e.g., the termination region of the *E. coli* trp operon; (iv) a transcriptional promoter, e.g., a phoA, tac, tac-lac, lacZ, lac^{uvs}, T7, or T3 promoter; (v) a leader sequence, e.g., a pelB or ompA leader; (vi) a DNA segment encoding the sc-TCR fused to a desired Ig-C_{L} chain or suitable Ig-C_{L} chain fragment, e.g., the full-length murine or human Cκ exon; and (vii) a transcriptional terminator, e.g., the T1T2 sequence from the ribosomal RNA locus of *E. coli.* Alternatively, the vector can include (i)-(vii), above, except that the sc-TCR is provided without a fused Ig-C_{L} chain or fragment.

Inclusion of particular nucleotide sequences in the DNA vectors designed for bacterial expression can assist soluble expression of the sc-TCR fusion proteins under slow induction conditions. For example, the phoA promoter and pelB leader sequences described below can be used for expressing the sc-TCR fusion proteins under slow induction conditions. See Example 6 which follows. A strong translation initiation sequence also can be included in the construct to enhance translation efficiency. For mammalian cell expression, a preferred initiation sequence is the Kozak consensus sequence (CCACCATG) (SEQ ID NO:97).

The leader sequence of the DNA vector suitably directs expression of the fusion protein to host cell membranes or to the host cell media and often includes a restriction site so that DNA encoding, e.g., V-α chain of interest can be conveniently ligated to the construct. Suitably, the restriction site is incorporated into the 3'-end of the leader sequence, sometimes referred herein as a junction sequence, e.g. of about 2 to 10 codons in length, and linked to the V-α chain so that the coding region for the V-α chain is typically the first amino acid of the V-α coding region. For example, one restriction site is the Sfi I site, although other cleavage sites can be incorporated before the V-α chain coding region to augment convenient insertion of the V-α chain into the vector construct. As discussed above, use of such a restriction site in combination with a second restriction site, typically positioned at the beginning of the V-α chain, enables rapid and straightforward insertion of sequences coding for a wide variety of V-α chains, or V-α,C-α chains. Preferred leader sequences contain a strong translation initiation site and can sometimes include a cap site at the 3'-end of their mRNA. As mentioned above, exemplary leader sequences include pelB, and OmpA for bacterial expression and a Cκ mouse kappa chain leader sequence for mammalian expression.

The sc-TCR proteins can be expressed in bacteria by a variety of means. For example, DNA vectors encoding sc-TCR fusion proteins can be expressed in bacteria by slowly inducing host cells over an extended time period, about two to eight hours. For example, as described below in Example 6, a DNA vector can be made that includes a phoA promoter (strong) operably linked to sequences encoding an sc-TCR protein. Host cells can then be transformed with the DNA vector and phosphate in the host cell media can be allowed to deplete from the media over several hours, generally about 2 to 10 hours, more preferably about 4 to 6 hours.

The sc-TCR proteins encoded by the DNA vectors provided herein can be purified by several conventional techniques. For example, as previously mentioned, the soluble fusion proteins can include one or more protein tags (same or different), including tags which comprise a chemical or protease cleavage site. Particularly, a protein tag can be a polypeptide bearing a charge at physiological pH, such as e.g., 6xHIS. In this embodiment, a suitable synthetic matrix can be used to purify the fusion protein. More particularly, the synthetic matrix can be a commercially available sepharose matrix, such as e.g. Ni-Sepharose or other such suitable matrixes capable of binding the 6XHIS tag at about pH 6-9. Other suitable tags include EE or myc epitopes which are specifically bound by commercially available monoclonal antibodies. In general, a wide variety of epitopes capable of being specifically bound by an antibody, e.g., a monoclonal antibody, are capable of serving as a protein tag. Other suitable synthetic matrices includes those with a bound antibody capable of specifically binding the present sc-TCR proteins. Exemplary protein tags include those with an enterokinase, Factor Xa, snake venom or thrombin cleavage site. See e.g., published PCT application WO 96/13593.

An expressed sc-TCR protein can be isolated and purified by known methods including immunoaffinity chromatography, immunoabsorption, immunoprecipitation and the like. Importantly, the preparative procedures will not usually require prolonged isolation steps to obtain significant yields of the fusion protein. In accordance with the protein purification methods described more fully below, yields for most sc-TCR proteins are in the range of 2 to 20 milligrams per approximately 50 to 100 grams of host cell paste.

In general, to prepare the sc-TCR proteins disclosed herein, a cell extract or host cell culture medium is centrifuged and the resulting supernatant purified by affinity or immunoaffinity chromatography, e.g. Protein-A or Protein-G affinity chromatography or an immunoaffinity protocol comprising use of an antibody that specifically binds the expressed sc-TCR fusion molecule. Examples of such an antibody are commercially available monoclonal antibodies capable of specifically binding the V-α chain or V-β chain of the sc-TCR. Exemplary of such antibodies include H57, MR5-2, and F23.1 obtainable from Pharmagen. Alternatively, a commercially available anti-idiotypic antibody which specifically binds an immunoglobin chain in the sc-TCR protein can be used to purify the sc-TCR fusion protein. Examples of use of such antibodies are provided in Example 5 below. Affinity purification of proteins using a monoclonal antibody is generally known and has been disclosed, e.g., see Harlow and Lane in, Antibodies: A Laboratory Manual (1988).

As described above, the sc-TCR proteins of the present invention are provided in a soluble and fully functional form. Thus, the sc-TCR protein is stably secreted into culture medium and is capable of specifically binding a ligand of interest such as a peptide antigen or synthetic small molecule. More particularly, the sc-TCR proteins are generally stable under physiological conditions in the substantial or complete absence of a chaotropic agent such as a detergent or the like. Thus, the present soluble proteins will not generally include regions rich in hydrophobic amino acids such as those amino acids found in a TCR transmembrane region. However, in some cases, suitable portions thereof may be included provided that the sc-TCR protein remains fully soluble. That is, expression of the fusion protein will not lead to formation of significant quantities of inclusion bodies in suitable host cells. Inclusion bodies are readily detectable by microscopy or conventional biochemical techniques such as centrifugation sedimentation.

The sc-TCR proteins of the present invention can be prepared as discussed above, as well as the examples which follow. Generally, DNA coding for a desired V-α or V-β chain can be obtained from a suitable source such as a T-cell, T-cell hybridoma line, or publicly available V-α and V-β chain sequence as described previously. The DNA can be amplified by PCR, cloning or other suitable means. For example, DNA encoding a desired V-α chain can be cloned into a suitable vector, followed by cloning of DNA encoding a desired V-β chain and a suitable single chain linker sequence to produce a desired sc-TCR. As disclosed previously, in some cases the sc-TCR will include a DNA encoding a C-α and/or C-β chain fragment. For a soluble sc-TCR fusion protein, a sc-TCR molecule construct is further fused to a Ig-C_{L} chain or fragment, e.g., the murine or human Cκ chain or a Cκ chain fragment which is about 50 and 126 amino acids in length. As noted previously, DNA encoding the Cκ chain can be PCR amplified and ligated to DNA encoding the sc-TCR. Alternatively, the Cκ chain can be included in a DNA vector such as those disclosed by Near, et al., *infra.* The DNA segment encoding the fusion protein is then introduced into the DNA vector. The DNA vector is then expressed in a host cell and fusion protein harvested and purified if desired.

Illustrative sc-TCR fusion proteins of the present invention are generally encoded by a DNA segment including covalently linked in sequence: promoter/leader sequence/V-α chain/single-chain linker sequence/V-β chain, C-β chain fragment/Cκ chain; promoter/leader sequence/ V-α sequence/V-β chain/Cκ chain; or promoter/leader sequence/ V-α chain, G-a chain fragment/single-chain linker sequence/V-β chain, C-β chain fragment/Cκ chain. Exemplary sc-TCR molecules are as described above except that an the Cₖ chain is not encoded by the DNA segment. The DNA vectors encoding the sc-TCR proteins are introduced into desired cells, including those specific expression systems disclosed herein, for soluble expression of the fusion protein.

The sc-TCR proteins provided herein can be modified by standard methods to include a variety of covalently linked effectors or tags. Suitable effectors or tags include those which impart a desired biological, chemical or physical property. More specifically, the effector molecule can be a cell toxin of, e.g., plant or bacterial origin such as, e.g., diphtheria toxin (DT), shiga toxin, abrin, cholera toxin, ricin, saporin, pseudomonas exotoxin (PE), pokeweed antiviral protein, or gelonin. Biologically active fragments of such toxins are well known in the art and include, e.g., DT A chain and ricin A chain. Additionally, the toxin can be an agent active at the cell surface such as, e.g., phospholipase enzymes (e.g., phospholipase C). Further, the effector molecule can be a chemotherapeutic drug such as, e.g., vindesine, vincristine, vinblastin, methotrexate, adriamycin, bleomycin, or cisplatin. Additionally, the effector molecule can be a detectably-labelled molecule suitable for diagnostic or imaging studies such as a radionuclide e.g., iodine-131, yttrium-90, rhenium-188 or bismuth-212. See e.g., Moskaug, et al. J. Biol. Chem. 264, 15709 (1989); Pastan, I. et al. Cell 47, 641, 1986; Pastan et al., Recombinant Toxins as Nobel Therapeutic Agents, Ann. Rev. Biochem. 61, 331, (1992); *"*Chimeric Toxins" Olsnes and Phil, Pharmac. Ther., 25, 355 (1982); published PCT application no. WO 94/29350; published PCT application no. WO 94/04689; and U.S. Pat. 5,620,939 for disclosure relating to making and using proteins comprising effectors or tags.

See also A.K. Abbas, *infra,* and the discussion that follows for disclosure relating to additional methods and materials for conducting diagnostic and imaging studies using the sc-TCR fusion molecules and sc-TCR molecules without a fused Ig-C_{L} chain (or fragment)disclosed herein.

A soluble sc-TCR protein that includes a covalently linked effector molecule has several important uses. For example, the sc-TCR protein can be employed to deliver the effector molecule to certain cells capable of specifically binding the sc-TCR. Accordingly, the sc-TCR protein provide means of selectively damaging or killing cells comprising the ligand. Examples of cells or tissue capable of being damaged or killed by the sc-TCR proteins include tumors and virally infected cells expressing one or more ligands capable of being specifically bound by the sc-TCR. Cells or tissue susceptible to being damaged or killed can be readily assayed by the methods disclosed herein.

A specific example of a sc-TCR protein fused to an effector molecule is as follows: an sc-TCR such as the p 149 sc-TCR disclosed below in Examples 6 and 7 below can be produced by transfecting mammalian cells with the pNAG1 or pNAG3 vector illustrated in Fig. 9B. The sc-TCR p149 protein recognizes a processed peptide fragment from human wild-type p53 tumor suppressor protein presented in the context of human HLA antigen; HLA-2.1. The sc-TCR p 149 and its peptide ligand have been described in Theobald, M.J., et al., PNAS (USA) (1995), 92:11993. The peptide sequence is STPPPGTRV (SEQ ID NO. 146). Expression of tumor suppressor protein p53, is upregulated on malignant cells. It has been shown that 50% of all tumors expressed increased levels of p53 on the surface (Holliston, M.D., et al., Science (1991), 253:49). Therefore, scTCR molecules specific for this epitope could be labeled with a toxin that could than be delivered to the malignant cells expressing the p53 peptide fragment HLA-2.1 ligand. This target specific immunotherapy could be effective at killing only malignant cells. Methods for measuring cytotoxicity *in vitro* are well-known and include conventional viability assays as described below. A sc-TCR molecule comprising p 149 sc-TCR linked to an effector has other important uses. For example, the sc-TCR molecule an be used to selectively kill human breast cancer cells expression p149 peptides. *In vitro* studies can be conducted in which the ability of the toxin labeled p149 molecule to kill breast cancer cells is evaluated using a non-radioactive cell cytotoxic assay using a Eu³⁺ release cytotoxicity assay (Bouma, G.J., et al., (1992) Hum. Immunol. 35:85). A sc-TCR molecule comprising a fused effector molecule can be readily tested *in vivo.* For example, *in vitro* stuides can be carried out by grafting p149/HLA.A21 expressing breast cancer cells into HLA/A2 transgenic mouse. (Theobald, et al., (1995) *supra*). Toxinlabeled scTCR p149 molecules can be injected into mice at pre-determined dosages and the effect on tumor size can be measured to indicate efficacy of the sc-TCR molecules. In addition, extension of life can be used as a second criterion to evaluate the efficeincy of the novel anti-tumor therapy.

Other suitable effector or tag molecules are known. For example, one tag is a polypeptide bearing a charge at physiological pH, such as, e.g., 6xHIS. In this instance, the sc-TCR molecule or soluble sc-TCR fusion protein can be purified by a commercially available metallo-sepharose matrix such as Ni-sepharose which is capable of specifically binding the 6xHIS tag at about pH 6-9. The EE epitope and myc epitope are further examples of suitable protein tags, which epitopes can be specifically bound by one or more commercially available monoclonal antibodies.

Molecular weights of sc-TCR fusion proteins of the present invention will vary depending on a number of factors including whether a full-length Cκ or Cλ chain is chosen or a suitable fragment thereof, or whether one or more protein tags are employed. In general, a soluble fusion protein will have a molecular weight of greater than approximately 45 kDA, in which the V-α and V-β chains therein will have a molecular weight of greater than about 20 kDA, more typically between about 21 to about 26 kDa. Typically, a fusion protein of the present invention will have a molecular weight of about 50 to about 75 kDa. All of the above mentioned molecular weights are determined by conventional molecular sizing experiments such as SDS-PAGE gel electrophoresis. See generally Sambrook, et al., *supra* Harlow and Lane, supra; Ausubel et al, *supra.*

In some settings it can be useful to make the sc-TCR proteins of the present invention polyvalent, e.g., to increase the valency of the sc-TCR. Briefly stated, the polyvalent sc-TCR protein is made by covalently linking together between one and four proteins (the same or different) by using e.g., standard biotin-streptavidin labeling techniques, or by conjugation to suitable solid supports such as latex beads. Chemically cross-linked proteins (for example cross-linked to dendrimers) are also suitable polyvalent species. For example, the protein can be modified by including sequences encoding amino acid residues with chemically reactive side chains such as Cys or His. Such amino acids with chemically reactive side chains may be positioned in a variety of positions in the fusion protein, preferably distal to the antigen binding region of the sc-TCR. For example, the C-terminus of a C-β chain fragment of a soluble fusion protein can be covalently linked to a protein purification tag or other fused protein which includes such a reactive amino acid(s). Suitable side chains can be included to chemically link two or more fusion proteins to a suitable dendrimer particle to give a multivalent molecule. Dendrimers are synthetic chemical polymers that can have any one of a number of different functional groups of their surface (D. Tomalia, Aldrichimica Acta, 26:91:101 (1993)). Exemplary dendrimers for use in accordance with the present invention include e.g. E9 starburst polyamine dendrimer and E9 combust polyamine dendrimer, which can link cysteine residues.

It also may be desirable to construct DNA vectors encoding a soluble protein of the invention and other growth agents, particularly a T-cell co-stimulatory factor such as those of the B-7 gene family (e.g., B7-1 or B7-2) to boost activity of cells including the fusion proteins.

The soluble proteins of the present invention can be used to detect and characterize superantigens. For example, the methods of the invention can be used to map an uncharacterized epitope for T-cells as follows: sequences encoding either a library of random peptides or selected peptides can be provided in a peptide library. The library is then screened with a soluble sc-TCR protein of the invention, preferably a detectably-labelled fusion protein (e.g., labeled with a radioactive atom or luminescent molecule). Peptides specifically bound by the fusion protein are released from the fusion molecule and then amplified. Sequence analysis of the peptide will identify sequences bound by the fusion protein. Additionally, the cloned peptide sequence can be tested for binding to the T-cells expressing a TCR corresponding to the V-α and V-β chains of the fusion protein. Any one of several random peptide libraries can be suitably employed. See e.g., J. Scott et al., Science (1990) 249:386; J. Devlin et al., Science, (1990) 249:404; S. Cwirla et al., PNAS (USA), (1990); 87:6378; J. Hammer et al., J. Exp. Med. (1992) 176:1007; Rhode, P.R. et al. J. Immunol. (1996) 157: 4885; and D. O'Sullivan et al., J. Immunolo., (1991) 147:2663.

Highly useful single-chain class I and class II MHC/peptide complexes (often referred to therein as scMHC class I or class II complexes) are disclosed in published PCT Application Nos. PCT/US95/09816 and PCT/US97/01617, as well as pending U.S. Patent Application Serial Nos. 08/382,454, filed February 1, 1995. The published PCT application Nos. PCT/US95/09816, PCT/US97/01617, and pending U.S. Application Serial No. 08/382,454 also disclose highly useful *in vitro* and *in vivo* T-cell binding assays which can be readily adapted to test the function of the sc-TCR proteins disclosed herein.

The ability of a sc-TCR protein of the present invention to modulate activity of a T-cell (i.e. reduce or eliminate T-cell activity such as proliferation) can be readily determined in accordance with the assays and materials for performing the assays disclosed in said published PCT Application Nos. PCT/US95/09816, PCT/US97/01617, as well as said pending U.S. Patent Application Ser. No. 08/382,454.

More specifically, as disclosed in said published PCT Application Nos. US95/09816, PCT/US97/01617, as well as said pending U.S. Patent Application Ser. No. 08/382,454, *in vitro* assays can be performed to determine if a molecule is capable of modulating T-cell activity. Such assays can be modified to determine functionality of the sc-TCR proteins. Generally, a exemplary assay is conducted as follows, by the sequential steps 1-4 below. T-cells suitably express a marker that can be assayed and that indicates T-cell activation, or modulation of T-cell activity after activation. Thus, as disclosed in the prior applications, the murine T-cell hybridoma DO11.10 expressing interleukin-2 (IL-2) upon activation can be employed. IL-2 concentrations can be measured to determine if a particular sc-TCR fusion molecule is capable of modulating activity of the T-cell hybridoma (e.g., decreasing IL-2 production). A general example of such a suitable assay is conducted by the following sequential steps:
1. A T-cell hybridoma or T-cells which include a TCR corresponding to an sc-TCR protein of the invention are obtained.
2. The T-cell hybridoma or T-cells are then cultured under conditions that allow proliferation.
3. The proliferating T-cell hybridoma or T-cells are then contacted with one or more sc-TCR fusion proteins.
4. The T-cell hybridoma or T-cells are contacted with a ligand capable of specifically binding the TCR and activating the T-cell hybridoma or T-cells. Exemplary ligands include antigens such as superantigens, an sc-MHC class I or II complex bearing a presenting peptide as disclosed above, or a suitable APC.
5. The T-cell hybridomas or T-cells are contacted with a suitable co-stimulatory factor to provide signals necessary for activation. The T-cell hybridomas or T-cells are subsequently assayed for a marker, e.g. IL-2 production is measured. A decrease in IL-2 production, e.g., a 40 percent or greater decrease in IL-2 production after a period of 24 hrs., indicates the sc-TCR fusion protein binds the ligand and can thus modulate the activity of the T-cells.

As disclosed previously in said published PCT Application Nos. PCT/US95/09816, PCT/US97/01617, and in said pending U.S. Patent Application Ser. No. 08/382,454, the T-cells employed in the assays are usually incubated under conditions suitable for proliferation. For example, a DO 11.10 T-cell hybridoma is suitably incubated at about 37°C and 5% CO₂ in complete culture medium (RPMI 1640 supplemented with 10% FBS, penicillin/streptomycin, L-glutamine and 5x10-5 M 2-mercaptoethanol). Serial dilutions of a fusion protein can be added to the T-cell culture medium in concentrations typically in the range of from 10⁻⁸ to 10⁻⁵ M. T-cell activation signals are preferably provided by antigen presenting cells that have been loaded with the appropriate antigen. It is believed that use of antigen dose and APC numbers giving slightly submaximal T-cell activation is preferred to detect inhibition of T-cell responses with fusion proteins. A decrease in production of IL-2 following contact with the sc-TCR fusion protein indicates the fusion protein modulates activity of the T-cells.

As disclosed previously in said published PCT Application Nos. PCT/US95/09816, PCT/US97/01617 and in said pending U.S. Patent Application Ser. No. 08/382,454, rather than measurement of an expressed protein such as IL-2, modulation of T-cell activation can be suitably determined by changes in antigen-dependent T-cell proliferation as measured by radiolabelling techniques as are recognized in the art. For example, a detectably-labeled (e.g., tritiated) nucleotide may be introduced into an assay culture medium. Incorporation of such a tagged nucleotide into DNA serves as a measure of T-cell proliferation. This assay is not suitable for T-cells that do not require antigen presentation for growth, e.g., T-cell hybridomas. It is suitable for measurement of modulation of T-cell activation for untransformed T-cells isolated from mammals. A decrease in the level of T-cell proliferation following contact with the fusion protein indicates that the molecule modulates activity of the T-cells and can suppress immune response. The *in vitro* T-cell proliferation assay is preferred for measuring the effects of fusion proteins on antigen-specific changes in T-cell colony expansion *in vivo.* Measurement of IL-2 production or T-cell proliferation can be employed to determine if the sc-TCR fusion protein is capable of modifying T-cell activation. For example, a decrease in IL-2 production of APC-stimulated T-cells after contact by the fusion protein indicates that the fusion molecule modulates activity of the T-cells and can suppress a T-cell mediated immune response.

In general, suitable T-cells for the assays are provided by transformed T-cell lines such as T-cell hybridomas or T-cells isolated from a mammal, e.g., a primate such as from a human or from a rodent such as a mouse, rat or rabbit. Other suitable T-cells include: 1) T-cell hybridomas which are publicly available or can be prepared by known methods, 2) T helper cells, and 3) T cytotoxic cells, preferably cytotoxic CD8+ cells. T-cells can be isolated from a mammal by known methods. See, for example, R. Shimonkevitz et al., J. Exp. Med., (1983) 158:303.

*In vivo* assays also may be suitably employed to determine the ability of the soluble fusion proteins to modulate the activity of T-cells, including the ability to inhibit or inactivate T-cell development. For example, the sc-TCR fusion protein can be assayed for its ability to inhibit immunoglobulin class switching (i.e. IgM to IgG). See e.g., P. Linsley et al., Science, (1992) 257:792-795.

Diagnostic methods using the soluble sc-TCR proteins are also provided including *in vivo* diagnostic imaging and HLA typing (see, e.g., A.K. Abbas, Cellular and Molecular Immunology, page 328 (W.B. Saunders Co. 1991). For *in vivo* imaging applications, the fusion protein includes a radioactive label (e.g., ¹²⁵I, ³²P, ⁹⁹Tc) or other detectable tag which can be administered to a mammal and the subject scanned by known procedures for binding of the sc-TCR. Such an analysis of the mammal could aid in the diagnosis and treatment of a number of disorders, including e.g. undesired expression of APCs accompanying immune system disord.

Assays also may be employed to evaluate use of the sc-TCR protein (or sc-TCR obtained from a soluble fusion protein) for treatment of an autoimmune disorder. For example, as disclosed in said published PCT Application Nos. PCT/US95/09816, PCT/US97/01617 as well as said pending U.S. Patent Application Ser. No. 08/382,454, experimental allergic encephalomyelitis (EAE) is an autoimmune disease in mice and a recognized model for multiple sclerosis. In one exemplary assay, a mouse strain can be treated to develop EAE and then a suitable sc-TCR protein (or sc-TCR obtained from a soluble fusion protein) can be administered. The animal can then be evaluated to determine if EAE development is inhibited or prevented after administration of the fusion protein or sc-TCR.

The ability of a sc-TCR protein of the invention to induce an immune response, including vaccination against a targeted disorder as disclosed previously, may be readily determined by an *in vivo* assay. For example, the sc-TCR protein (or the sc-TCR obtained from a soluble fusion protein), can be administered to a mammal such as a mouse, blood samples obtained from the mammal at the time of initial administration and several times periodically thereafter (e.g. at 2, 5 and 8 weeks after administration of the sc-TCR protein). Serum is collected from the blood samples and assayed for the presence of antibodies raised by the immunization. Antibody concentrations may be determined in accordance with standard immunological techniques.

The DNA segments of the present invention may also be used in a method of administering a DNA sequence encoding an sc-TCR protein to express the protein within cells of the mammal, particularly a aprimate such as a human Preferably, DNA carrying the coding regions of the fusion protein, suitably under the control of an appropriate promoter such as the CMV promoter, is injected directly into skeletal muscle of the subject according to known methods. Methods for administration of plasmid DNA, uptake of that DNA by cells of the administered subject and expression of protein has been reported (see J.. Ulmer et al. Science, (1993) 259:1745-1749).For a soluble fusion protein, it is generally preferred that the encoded Ig-C_{L} chain or fragment thereof is immunoglologically compatible with the host employed.

The sc-TCR proteins of the present invention have a number of therapeutic applications. For example, the sc-TCR protein can be administered to reduce or eliminate an immune response in a mammal, e.g., to treat a mammal such as a human that suffers from or is susceptible to cancer, an infectious disease, allergy or an autoimmune disorder such as e.g. multiple sclerosis, insulin-dependent diabetes mellitus, rheumatoid arthritis and the like. Administration can be via any suitable means such as direct administration of DNA encoding the fusion protein. Also suitable for treatment are those subjects suffering or likely to suffer from an undesired immune response e.g. patients undergoing transplant surgery such as transplant of heart, kidney, skin or other organs. In situations involving transplant rejection, a treatment protocol may suitably be commenced in advance of the surgical procedure.

A number of distinct approaches can be employed to reduce or eliminate an immune response of a mammal in accordance with the invention. For example, one treatment method for reduction of an undesired immune response provides for the administration of an effective amount of a desired sc-TCR fusion to reduce or eliminate interaction between pathogenic T-cells and a superantigen or peptide-MHC complex. Accordingly, T-cell mediated immune responses such as T-cell proliferation, differentiation, activation or B lymphocyte stimulation can be selectively controlled. Preferably, the fusion protein exhibits at least the same or preferably increased specific binding affinity for antigen as the pathogenic T-cells mediating the undesired immune response. Particularly preferred in this respect are the sc-TCR fusion protein muteins described previously which demonstrate increased specific binding affinity for ligand. The fused Ig-C_{L} chain or fragment can be used to identify fusion protein binding to target cells by standard immunological assays.

Administration of the sc-TCR protein, sc-TCR derived from a suitable sc-TCR fusion protein, or antibody prepared in accordance with the methods disclosed herein can be administered to a mammal by injection, e.g., intraperitoneal or intravenous injection. An fusion protein, least those molecules used in therapeutic applications, are preferably produced from mammalian cells or other suitable cells and purified prior to use so it is essentially or completely free of pyrogens. The optimal dose for a given therapeutic applications can be determined by conventional means and will generally vary depending on a number of factors including the route of administration, the patient's weight, general health, sex, and other such factors recognized by the art-skilled.

Administration can be in a single dose, or a series of doses separated by intervals of days or weeks. The term "single dose" as used herein can be a solitary dose, and can also be a sustained release dose. The subject can be a mammal (e.g,. a human or livestock such as cattle and pets such as dogs and cats) and include treatment as a pharmaceutical composition which comprises the sc-TCR, sc-TCR fusion protein, or the antibody. Such pharmaceutical compositions of the invention are prepared and used in accordance with procedures known in the art. For example, formulations containing a therapeutically effective amount of an the fusion protein may be presented in unit-dose or multi-dose containers, e.g., sealed ampules and vials, and may be stored in a freeze dried (lyophilized) condition requiring only the addition of the sterile liquid carrier, e.g. water injections, immediately prior use. Liposome formulations also may be preferred for many applications. Other compositions for parenteral administration also will be suitable and include aqueous and non-aqueous sterile injection solutions which may contain anti-oxidants, buffers, bacteriostat and solutes which render the formulation isotonic with the blood of the intended recipient; and aqueous and non-aqueous sterile suspensions which may include suspending agents and thickening agents.

Methods of the invention which include reducing or eliminating T-cell responses also may be used in combination with known immunosuppressive, anti-viral, anti-cancer or anti-inflammatory agents to provide a more effective treatment of a T-cell mediated disorder. For example, the fusion proteins can be used in combination with conventional immunosuppressive drugs such as cyclosporin or anti-inflammatory agents such as corticosteroids and non-steroidal drugs for the treatment of autoimmune disorders and allergies.

As mentioned previously, the sc-TCR proteins (or sc-TCR molecule derived from a desired sc-TCR fusion protein) can be used to produce antibodies by techniques generally known in the art, and are typically generated to a purified sample of the fusion protein. An sc-TCR fusion protein chosen for raising the antibodies can be cleaved from the Ig-C_{L} chain or fragment as described previously to reduce immunological reaction against the immunoglobin chain. The sc-TCR thus obtain is used as an immunogen. The antibodies also can be generated from an immunogenic peptide that comprises one or more epitopes of an sc-TCR of interest.

More particularly, antibodies can be prepared by immunizing a mammal with a purified sample of the sc-TCR protein, an sc-TCR molecule separated from a desird sc-TCR fusion protein or an immunogenic peptide as discussed above, alone or complexed with a suitable carrier. Preferably, the sc-TCR molecule will be used as immunogen. Suitable mammals include typical laboratory animals such as sheep, goats, rabbits, guinea pigs, rats and mice. Rats and mice, especially mice, are preferred for obtaining monoclonal antibodies. The antigen can be administered to the mammal by any of a number of suitable routes such as subcutaneous, intraperitoneal, intravenous, intramuscular or intracutaneous injection. The optimal immunizing interval, immunizing dose, etc. can vary within relatively wide ranges and can be determined empirically based on this disclosure. Typical procedures involve injection of the antigen several times over a number of weeks. Antibodies are collected from serum of the immunized animal by standard techniques and screened to find antibodies specific for the sc-TCR. Of particular interest are those antibodies which specifically bind the V-α or V-β chain, particularly the hypervariable region therein, including those antibodies which recognize linear or conformational epitopes thereon. Monoclonal antibodies can be produced in cells which produce antibodies and those cells used to generate monoclonal antibodies by using standard fusion techniques for forming hybridoma cells. See G. Kohler, et al. (1975), Nature, 256:456. Typically, this involves fusing an antibody producing cell with an immortal cell line such as a myeloma cell to produce the hybrid cell. Alternatively, monoclonal antibodies can be produced from cells by the method of Huse, et al.(1989), Science, 256:1275.

One suitable protocol provides for intraperitoneal immunization of a mouse with a composition comprising purified sc-TCR complex conducted over a period of about two to seven months. Spleen cells then can be removed from the immunized mouse. Sera from the immunized mouse is assayed for titers of antibodies specific for the sc-TCR prior to excision of spleen cells. The excised mouse spleen cells are then fused to an appropriate homogenic or heterogenic (preferably homogenic) lymphoid cell line having a marker such as hypoxanthine-guanine phosphoribosyltransferase deficiency (HGPRT-) or thymidine kinase deficiency (TK-). Preferably a myeloma cell is employed as the lymphoid cell line. Myeloma cells and spleen cells are mixed together, e.g. at a ratio of about 1 to 4 myeloma cells to spleen cells. The cells can be fused by the polyethylene glycol (PEG) method. See G. Kohler, et al., Nature, *supra.* The thus cloned hybridoma is grown in a culture medium, e.g. RPMI-1640. See G. E. More, et al.(1967), Journal of American Medical Association, 199:549. Hybridomas, grown after the fusion procedure, are screened such as by radioimmunoassay or enzyme immunoassay for secretion of antibodies that bind specifically to the purified sc-TCR. An ELISA can be used to screen antibody containing sera according to conventional methods. Hybridomas that show positive results upon such screening can be expanded and cloned by limiting dilution method. Further screens are preferably performed to select antibodies that can bind to sc-TCR in solution as well as in a biological sample. The isolated antibodies can be further purified by any suitable immunological technique including affinity chromatography.

For some applications, it may be desirable to produce chimeric antibody derivatives which specifically bind sc-TCRs, e.g. antibody molecules that combine a non-human animal variable region and a human constant region, to thereby render the antibodies less immunogenic in a human subject than the corresponding non-chimeric antibody. A variety of types of such chimeric antibodies can be prepared, including e.g. by producing human variable region chimeras, in which parts of the variable regions, especially conserved regions of the antigen-binding region, are of human origin and only the hypervariable regions are of non-human origin. See also discussions of humanized chimeric antibodies and methods of producing same in S.L. Morrison,(1985) Science, 229:1202; Oi et al.(1986), BioTechniques, 4:214; Teng et al.(1983), PNAS. U.S.A., 80:7308; Kozbor et al.(1983), Immunology Today, 4:7279; Olsson et al.(1982), Meth. Enzymol., 9:3.

As mentioned above, the sc-TCR proteins can be readily modified in accordance with conventional mutagenesis techniques to improve specific binding for a desired ligand. For example, a desired sc-TCR can be made by isolating DNA segments encoding suitable V-α and V-β chains linked by a peptide linker sequence as described previously. The DNA can be ligated to the Ig-C_{L} if desired and then subjected to mutagenesis such as site directed mutagenesis within a desired V-α or V-β chain. Exemplary mutagenesis methods include alanine scanning mutagenesis (see e.g., Nisbet, I.T. et al., (1985) Gene Anal. Tech. 2, 23; Hines, J.C., Gene (1980) 11, 207; see also Sambrook et al. *supra*). If it is desired to modulate the specific binding affinity of the sc-TCR fusion protein, then the mutations selected will typically be those amino acid substitutions which affect the binding affinity of the complimentarily determining region (ie. CDR, sometimes referred to as the hypervariable region) of the sc-TCR. More specifically, the amino acid substitutions will be conservative or non-conservative substitutions which, as used herein, means substitution of one amino acid in the sc-TCR for another amino acid. In some cases, the substitution will consist of replacement of an amino acid with another amino acid with substantially similar chemical properties (conservative). In other cases, the substitution will consist of replacement of an amino acid with another amino acid having substantially different chemical properties (non-conservative). Accordingly, a tyrosine residue in the sc-TCR replaced with a phenylalanine residue would represent a conservative amino acid substitution, whereas an alanine residue replaced with a proline residue would represent a non-conservative substitution. It will be understood by those skilled in the art that the mutagenesis may also provide changing the length or amino acid composition of the peptide linker sequence separating the V-α and V-β chains of the sc-TCR. Additionally, mutagenesis may be directed to the Ig-C_{L} chain or fragment to change the length or amino acid composition thereof as desired. In most cases however, mutagenesis will be targeted to the V-α or V-β chains and will provide, on average, one amino acid mutation substitution in the V-α or V-β chain of the fusion protein. The extent of mutagenesis can be conveniently assayed by sequencing the DNA of the mutagenized V-α or V-β chain.

Preferably, mutagenesis of the V-α or V-β chains will increase the specfic binding of the sc-TCR fusion protein for ligand to a level that is at least about 2-fold higher than the naturally-occuring TCR.

Although a less preferred method, the sc-TCR fusion proteins can be modified by well known chemical mutagenesis techniques as desired.

The invention provides soluble fusion proteins which can be used in a method of detecting a molecule capable of inhibiting specific binding between a ligand and a T-cell receptor. The method includes incubating a single-chain T-cell receptor fusion protein in the presence of a ligand capable of specifically binding a T-cell receptor, incubating the single-chain T-cell receptor fusion protein in the presence of the ligand and a molecule of interest; and evaluating the interaction between the ligand and the single-chain T-cell receptor fusion protein in the absence and presence of the molecule, wherein less interaction between the fusion protein and the ligand in the presence of the molecule than in the absence of the molecule is indicative of the molecule being capable of inhibiting specific binding between the ligand and the T-cell receptor. The method can be used with a sc-TCR molecule instead of the sc-TCR fusion protein if desired.

The term antibody as used herein generally refers to whole immunoglobulin as well immunologically active fragments which bind the sc-TCR molecule of the soluble fusion protein. The immunoglobins and immunologically active fragments thereof include an antibody combining site (i.e., peritope capable of specifically binding the fusion protein). Exemplary antibody fragments include, for example, Fab, F(v), Fab', F(ab')2 fragments, "half molecules" derived by reducing the disulfide bonds of immunoglobulins, single chain immunoglobulins, or other suitable antigen binding fragments (see e.g., Bird et al.(1988), Science, 242; Huston et al.(1988), PNAS, (USA), 85:5879; Webber et al.(1995), Mol. Immunol., 32:249.. The antibody or immunologically active fragment thereof may be of animal (e.g., a rodent such as a mouse or a rat), or chimeric form (see Morrison et al.(1984), PNAS, 81:6851; Jones et al.(1986), Nature, 321.

By the term, "specific binding" or a similar term is meant a molecule disclosed herein which binds another molecule, thereby forming a specific binding pair. However, the molecule does not recognize or bind to other molecules as determined by, e.g., Western blotting ELISA, RIA, mobility shift assay, enzyme-immuno assay, competitive assays, saturation assays or other protein binding assays know in the art. See generally, Ausubel, et al *supra*; Sambrook, et al, *supra*; Harlow and Lane, *supra* and references cited therein for examples of methods for detecting specific binding between molecules.

Substantially pure soluble fusion proteins or nucleic acids are at least about 90 to 95% pure and preferably at least 98% to 99% or more pure for pharmaceutical use. Once purified partially or to substantial purity, the soluble fusion proteins can be used therapeutically (including extracorporeally), or in developing or performing *in vitro* or *in vivo* assays as disclosed herein.

The following examples are illustrative of the present invention.

### Example 1- Construction of Soluble sc-TCR Fusion Proteins

The DNA sequence of the murine DO11.10 cell TCR has been reported (Kappler, J. et al. PNAS (1994) 91 8462). The TCR recognizes and binds a chicken ovalbumin peptide spanning amino acids 323-339 (ie. OVA) in the context of an I-A^{d} MHC class II molecule. DNA encoding the TCR was prepared generally along the lines of the method disclosed in Kappler and Marrack, *supra*.

Briefly, mRNA from 1x10⁶ DO11.10 cells was isolated using oligo-dT coated magnetic beads in accordance with the manufacturer's instructions (Dynal). TCR a chain cDNA was made by incubating a mixture containing the C-α specific "back" primer, KC 113 (SEQ ID No. 6) along with the D011.10 mRNA. Subsequently, standard amounts of nucleotides and reverse transcriptase were added to the mixture to form cDNA. The β chain cDNA was made in a similar manner with the exception that the "back" primer KC 111 (SEQ ID NO.4) was used instead of the KC113 primer. Alpha chain cDNA was used as a template with primers KC112 (SEQ ID NO. 5) and KC113 in a PCR reaction to amplify a 650 bp 5'XhoI- 3'XmaI α chain fragment. A 750 bp chain fragment was PCR-amplified using primers KC 111 and KC110 (SEQ ID NO. 3) containing SfiI and SpeI sites at the 5' and 3' ends respectively.

The sc-TCR was constructed to include a covalently linked V-α chain and V-β chain from the D011.10 TCR. In some cases, the V-α chain further included the C-β chain fragment and the C-β chain fragment (See Fig. 5). Generally, the Cα chain fragment was between approximately 9 amino acids in length, although larger C-α chains are disclosed below in Example 4. The C-β chain was typically truncated at amino acid residue 126 just before the cysteine residue at amino acid residue 127 of the full-length C-β chain. It was found that inclusion of this cysteine residue was deleterious to sc-TCR expression.

### Example 2 - Vectors For Expressing sc-TCR Fusion Proteins in E. coli

The constuction of DNA vectors including a DNA segment encoding a sc-TCR molecule fused to a bacteriophage coat protein (gene III or gene VIII) have been described in said pending U.S. application No. 08/813,781. Briefly, to construct the present DNA vectors encoding soluble fusion proteins selected DNA vectors of the said pending U.S. application were modified. See Examples 4-7.

### A. DNA Vector pJRS 149

The pJRS 149 DNA vector is a phagemid with a pBluScript™ (Invitrogen) backbone.

### B. DNA Vector pKC12

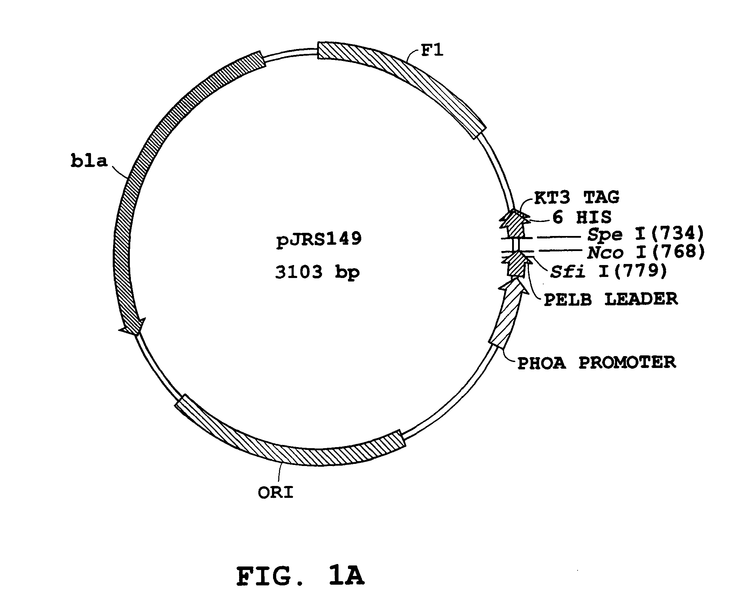

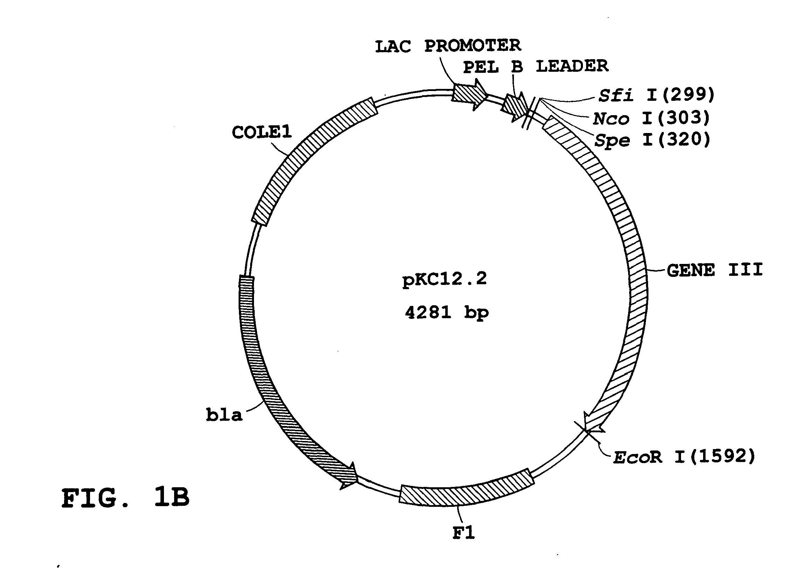

DNA encoding bacteriophage gene III was cloned into the pKC12 vector illustrated in Fig. 1.

### C. DNA Vectors pKC 14 and pKC 15

The bacteriophage gene VIII DNA sequence was PCR amplified from fd tet bacteriophage (ATCC accension No.37000) as template with primers OPR156 ("front" SEQ ID. NO.58) and OPR157 ("back" SEQ ID. NO. 59). The gene VIII PCR product was then cloned as a XmaI-EcoRI fragment into vector pLL001 to generate vector pKC 14. The pLL001 plasmid was derived from PUC-19 DNA and contained an XmaI and EcoRI site in the polylinker region. That site was convenient for subcloning the gene VIII segments. Additionally, the pLL001 vector was used as a shuttle vector to clone gene VIII DNA. Vector pKC 15 was derived from pKC 14 by cloning a 96 bp NcoI-EcoRI fragment into the pJRS149 vector shown in Fig. 1. The NcoI-EcoRI fragment contains a synthetic polylinker with multiple cloning sites (e.g., SfiI, NcoI, SpeI, XhoI and XmaI), a pelB leader, a phoA promoter and the gene VIII gene. Vector pKC 15 has a second pelB leader which originated from the pJRS 149 backbone and allows gene cloning to be under the control of a di-cistronic operon.

### D. DNA Vectors pKC16 and pKC 18

Alpha chain cDNA was used as template to amplify the TCR V-α, C-α gene using primers KC 113 (SEQ ID NO: 6) ("front") and KC112 (SEQ ID NO. 5) ("back") for cloning a XhoI-XmaI fragment from pKC16. The V-β, C-β gene fragment was amplified using PCR and β-chain cDNA as template with primers KC110 (SEQ ID NO. 3) ("front") and KC111 (SEQ ID NO:4) ("back") and cloned into pKC 15 to form vector pKC 18.

### E. DNA Vectors pKC27, pKC42 and pKC44

Vector pKC27 was constructed by annealing primers JA301 (SEQ ID NO. 95) and JA302 (SEQ ID NO. 96) to make a (G4S)4 polypeptide linker. The linker was subsequently cloned as a SpeI-XhoI fragment into the pKC 15 vector. The V-α 13.1 and V-β, C-β regions were then each cloned into pKC27 DNA. Briefly, a V-α 13.1 gene fragment was produced by PCR amplification using primers KC114 (SEQ ID NO: 7) ("front") and KC126 ("back") (SEQ ID NO: 19) with vector pKC16 DNA as template. The V-α13.1 gene was cloned into pKC42 as a SfiI-SpeI fragment. V-β 8.2, and C-β chain DNA was gel purified after digesting pKC 18 with XhoI and XmaI. The fragment was cloned into pKC42 to make the pKC44 vector. In general, the pKC44 vector included the sc-TCR as an sc-TCR/gene VIII fusion protein under the transcriptional control of the phoA promoter.

### F. pKC12, pKC45, pKC46, and pKC51 DNA Vectors

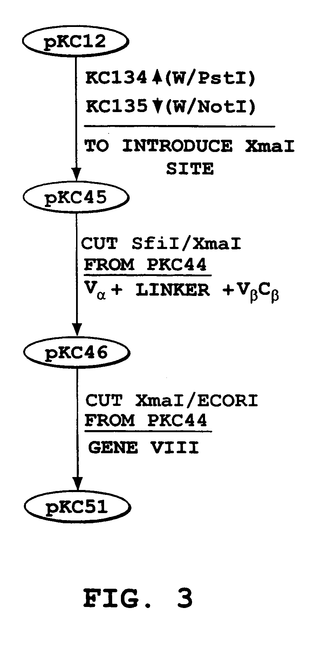

The construction of vectors pKC45, pKC46, and pKC51 from pKC12 is outlined in Fig. 3 and described as follows.

The pKC12 vector was modified so that DNA encoding the sc-TCR from Example 1 was expressed under the transcriptional control of the lacZ promoter. Briefly, the pKC12 vector was modified by cloning annealed primers KC134 (SEQ ID NO: 27) ("front") and KC135 (SEQ ID NO:28) ("back") into pKC12 to produce vector pKC45. The modification added an XmaI site into the polylinker region of pKC 12 which already included SfiI and EcoRI sites. Additionally, the pKC45 included a DNA sequence encoding gene III attached to the 5' end of an EE-tag and an amber stop codon.

The amber stop codon reduced the level of sc-TCR fusion protein expression to approximately 15-20% in a lacI^{q} amber suppressor host such as XL1-blue. To clone DNA encoding the sc-TCR/geneIII fusion protein into pKC45, pKC44 DNA was cleaved with SfiI and XmaI. The sc-TCR fragment was then gel purified and cloned into pKC45 to produce pKC46. The sc-TCR insert of pKC46 is illustrated in Fig. 3 fused to the EE-tag and gene III coat protein.

The sc-TCR/gene VIII fusion protein was placed under the transcriptional control of the lacZ promoter by digesting the pKC44 and pKC46 vectors with XmaI and EcoRI. The gene VIII DNA sequence was isolated from the digested pKC44 DNA and cloned into gel-purifed vector DNA pKC46 as an SfiI-EcoRI fragment to make vector pKC51. The sc-TCR insert in vector pKC51 was then fused to the gene VIII coat protein. Unlike vector pKC46, the pKC51 vector does not contain an EE-tag or an amber stop codon.

### Example 3- Cloning DNA encoding Fusion Proteins Into DNA Vector pEN2

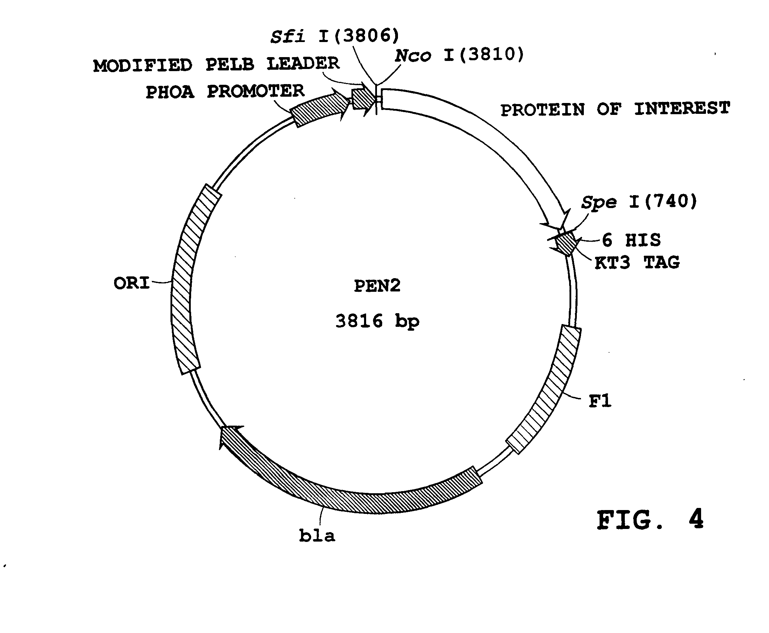

Expression of the soluble sc-TCR produced in Example 1 was increased by subcloning into the pEN2 vector illustrated in Fig. 4. The pEN2 vector includes a phoA promoter, a gene 10 ribosomal binding site, and a modified pel B leader. sc-TCR inserts pKC60 and pKC67 in a pEN2 vector background are schematically illustrated in Fig. 5 and described as follows.

### A. Construction of DNA Vector pKC60

The pKC60 vector was made by introducing a 1204 bp SfiI-EcoRI fragment into pEN2. The SfiI-EcoRI fragment consisted of the V-α and V-β, C-β regions. The fragment was made by amplifying pKC51 DNA as template and using primers KC114 ("front" SEQ ID NO: 7) and JWTCR208 ("back" SEQ ID NO:75). The JWTCR209 primer included a EE-tag and a XmaI site 3' of the C-( region. The addition of an XmaI site facilitated cloning of the gene III and gene VIII genes. The pKC60 vector was made by cloning the sc-TCR XmaI-EcoRI fragment described in above into the pEN2 vector.

### B. Construction of DNA Vector pKC61

To create a truncated version of the sc-TCR, the V-α, V-β sequence of vector pKC46 was PCR-amplified with primers KC115 ("front" SEQ ID NO: 8 ) and JWTCR209 ("back" SEQ ID NO:74). The resulting PCR amplification products were subcloned into vector pKC60 to yield vector pKC61. The pKC61 vector was further modified by adding a 6XHis tag to the 3' end of the EE-tag sequence.

### C. Construction of pKC62 and pKC64 DNA vectors

DNA encoding bacteriophage geneIII was amplified with primers TCR215 (SEQ ID NO 6F) and 218 (SEQ ID NO 64) using pKC46 vector DNA as the template and cloned into pKC60 to make vector pKC64. The gene VIII gene was amplified with primers TCR212 (SEQ ID NO:71) and 213 (SEQ ID NO: 70) using vector pKC51 DNA as template and then cloned into pKC60 to make vector pKC62.

### D. Construction of DNA Vectors pKC63 and pKC65

GeneIII (pKC65) and gene VIII (pKC63) fusion proteins are cloned into vector backbone pKC61 after PCR amplification of the respected gene fragments. Both pKC65 and pKC63 will then contain the 6xHis tail encoded in the primer sequence.

### E. Construction of pKC66 and pKC67 DNA Vectors

The pKC66 and pKC67 vectors were made by amplifying an SfiI-SpeI fragment containing the V-α and the first 8 amino acids of the C-α chain fragment using pKC51 DNA as template and primers KC114 (front SEQ ID NO: 7) and JWTCR 217-( (back SEQ ID NO: 66). To make the pKC66 and pKC67 vectors, the KC114 and JWTCR 217-( primers were used to PCR-amplify TCR DNA, afterwhich the amplified DNA was subcloned into pKC62 or pKC60 which was previously digested with SfiI and SpeI. These vectors each include 8 amino acid residues from the N- terminus of the C-α region. The vectors were used in the construction of the TCR library described below. Vector pKC66 includes the gene VIII gene.

As disclosed in the pending U.S. Application No. 08/813,781, the DNA vectors pKC46 (pSUN18) and pKC62 (pSUN19) have been deposited pursuant to the Budapest Treaty with the American Type Culture Collection (ATCC) at 12301 Parklawn Drive, Rockville, MD. The DNA vectors were deposited with the ATCC on February 26, 1997 and were assigned Accession Nos. 97895 (pSUN18) and 97896 (pSUN19). The DNA vector pKC62 (pSUN19) includes a phoA promoter, modified pelB sequence, gene 10 ribosome binding site and bacteriophage gene VIII promoter. The DNA vector pKC46 (pSUN18) includes the lac Z promoter, an EE tag and bacteriophage gene III protein. The DNA vectors can be propagated in *E. coli* or other suitable host cells in accordance with standard methods.

The DNA vectors pKC46 (pSUN18) and pKC62 (pSUN19) are designed to accommodate a variety of Va, Vβ-Cβ and polypeptide linker sequences. The Va chain of both DNA vectors can be removed by restriction digestion with SFil and SpeI. The Vβ-Cβ chain can be removed by restriction digestion with XhoI-XmaI. Additionally, the DNA vectors allow exchange of the peptide linker sequence by restriction digestion with SpeI and XhoI.

### Example 4- Construction of Vectors pKC24, pKC73, pKC74, pKC75

DNA vectors were constructed to express the D011.11 sc-TCR made in Example 1 as a protein fusion with the murine IgG kappa chain as follows.

To prepare DNA vectors comprising a DNA segment encoding the D011.11 sc-TCR fused to the murine IgG kappa chain, the VαCα chain of pKC16 was reamplified by PCR with primers KC114 (SEQ. ID. NO. 7) and KC117 (SEQ ID. NO 10) to generate a 5'SfiI-3'SpeI fragment. The DNA was cloned into pKC 15 and sequenced. The correct fragment was restriction digested and ligated into SfiI-SpeI digested pKC 15 DNA to yield the pKC24 vector. The derivation of the pKC 15 and pKC16 constructs have been described in Examples 2C and 2D above.

The pKC24 DNA was then used as a template in PCRs with primers KC114 (SEQ ID. NO.7) and KC165 (SEQ ID. NO. 131), KC114 and KC166 (SEQ ID. NO. 132), and KC114 and KC167 (SEQ. ID. NO. 133) to produce the V-α chain with different lengths of the C-α chain added. These PCR products were cloned into the pGEM-T Easy Vector System (Promega) for DNA sequence determination. Correct fragments were restriction digested and cloned into the expression vector pKC60. In all three fragments, the C-α chain fragment ended at a cysteine residue. This 'final" cysteine was changed to a serine residue but no internal cysteines were mutated. Final construct pKC73 has 22 amino acids of the C-α chain, pKC74 has 72 amino acids, and pKC75 has 90 amino acids (i.e. the entire C-α chain). The pKC73, pKC74 and pKC75 DNA vector inserts are schematically represented in Fig. 9A.

The V-β C-β chain was PCR amplified as a 5'SfiI-3'XmaI fragment using primers JWTCR222 (SEQE ID. NO. 60) and JWTCR208 (SEQ. ID NO. 75). It was sequenced and cloned into the expression vector as a (chain-only negative control).

### Example 5 -Construction and Expression of Soluble DO11.10 sc-TCR Fusion Proteins in Mammalian Cells

It is highly desirable to produce significant amounts of soluble sc-TCR. To increase the level of soluble protein expression, the sc-TCR constructs were cloned into a mammalian cell expression system.

The *E. coli* DNA constructs pKC60 and pKC67 prepared as described in Examples 3A,E above were reamplified by PCR with primers KC169 (SEQ ID NO. 143) and KC170 (SEQ ID. NO. 144) to generate a 5'AgeI-3'BstBI sc-TCR-IgG kappa chain (Cκ) fusion gene. The construction of the TCR-IgCκ expression vector was as follows: the backbone of the vector was the plasmid pCDNA3 (Invitrogen). This plasmid was cut HindIII/XhoI and the "light chain polylinker" DNA fragment was inserted to create the starting "light chain vector". This linker contained the restriction sites HindIII, KpnI, ClaI, PmlI, EcoRV, AgeI, XmaI, BamHI and XhoI to facilitate subsequent cloning steps to create the plasmid pCDNA.LCPL. A SmaI/BclI DNA fragment containing a light chain leader, anti-CKMB kappa light chain genomic fragment, and 3' UTR was cloned into the EcoRV/BamHI sites of pCDNA3.LCPL. The mouse kappa intron, exon and the 3' UTR in this fragment was derived from pNeo/26-IOVL received from Dr. Richard Near (Near, R., et al., (1990), Mol. Immunol. 27:901-909). Mutagenesis was then performed to eliminate an NruI (209bp), MluI(229 bp), and BstBI (2962 bp) and to introduce an NheI (1229 bp) and a BamHI (1214 bp) site to create pCDNA3mut. LCPL.LCVK.

The vector was then further modified to remove the variable light chain CKMB Vκ from the vector. This was carried out by digesting the plasmid pCDNA3mut.LCPL.LCVK with EcoRV/XhoI and inserting a linker oligonucleotide fragment containing EcoRV, AgeI, BstBI, and XhoI sites to create pSUN9.

The DO11.10 sc-TCR was cloned into pSUN6 vector as an *AgeI*/*BstBI* fragment after PCR amplification using primer set KC169 Front (5' gAg gTg ACC ggT gAg Cag TAC g TTT gTC TgC Tcg gCC CCA g-3) and KC 170 Back (5' gTg gAg TTC gAA Aag TgT ACT TAC g TTT gTC TgC Tcg gCC CCA g-3) to construct pSUN 27 vector (pKC60-M) and to express the vector as an sc-TCR-κ constant fusion protein.

The pSun27 template DNA can also be amplified by PCR using primers KC169 (SEQ ID. NO. 143) and KC201 (SEQ ID. NO. 145) to generate a 5'AgeI-3'BstBI sc-TCR without an IgG kappa chain fusion. pKC73, pKC74 and pKC75 DNA can also be PCR amplified with either primer pair to generate sc-TCR and sc-TCR-IgG Cκ fusion fragments for ligation into a suitable mammalian expression vector.

For example, the DO11.10 sc-TCR-IgG Cκ fusion fragments were cloned into the pGEM-T Easy Vector System (Promega) for DNA sequence determination. Correct fragments were restriction digested and cloned into a mammalian expression vector. The resulting mammalian expression vector contains the CMV promoter, neomycin resistance gene, mouse IgG kappa leader peptide, the mouse kappa intron and mouse kappa constant region exon sequences. The resulting vectors were named pKC60-M (pSUN27) and pKC67-M. The sc-TCR inserts of these vectors are illustrated in Fig. 9A. The vector is illustrated in Fig. 10.

CHO cells were prepared for transfection by washing with cold PBS. The cells were resuspended in PBS and mixed with 10-40µg of Pvul linearized pKC60KM. After ten minutes on ice, the cells were electroporated using a Gene Pulser (BioRad) set to deliver one pulse of 250 volts, 960 µ Fd. The pulsed cells were placed on ice and 10% IMDM medium, (IMDM, 10% FBS, 2mM glutamine, 5000 units/ml penicillan, 5000 ug/ml strepomycin) was added. The cells were diluted and plated in 96 well plates. After overnight incubation at 37o C with 10% CO₂, the cells were fed with neomycin selective medium (IMDM, 0.77 mg/ml G418) and thereafter every 3-7 days. A "mock" transfection was performed using cells and PBS only as a negative control.

Transfectants were screened for expression of soluble sc-TCR-IgG Cκ molecules using an anti-idiotypic, anti-DO11.10 TCR mAb, KJ-1, in an ELISA assay format. Previously published reports on the sc-TCR have demonstrated a strong correlation between functional TCR and binding to an anti-idiotypic mAb, i.e. sc-TCR recognized by an anti-idiotypic mAb can essentially recognize peptide associated with MHC. See Relter, Y., et al., (1995) Immunity 2, 281-287. To screen the transfectants, 96 well plates were passively coated overnight with KJ-1 in sodium bicarbonate buffer, pH 8.2. On assay day, the plates were blocked with 3-5% nonfat dry milk (NFDM) for a minimum of one hour. The wells were washed and supernatant from the transfectants was added to the plate. After incubation and washing, biotinylated anti-Cβ mAb H57-597 (ATTC Accession NO. HB-218) was added to the plate for 30 minutes, followed by washing and incubation with strepavidin-HRP. Positive wells were identified by the addition of TMB substrate, quenched with 1N sulfuric acid, and read at an absorbance of 450nM. Purified sc-TCR produced in *E. coli* served as the reference standard.

Multiple positive transfectants were identified but only clones from the most dilute plate were selected for expansion. Four clones were retested by ELISA prior to passage out of the original 96 well. Based on this initial quantitative screening , the clones are producing soluble DO11.10 sc-TCR-IgG Cκ in the range of 200-400 ng/ml. The data strongly suggests that the soluble sc-TCR- IgG Cκ fusion protein has a correctly folded Vα and Vβ pair.

The pSUN27 vector has been deposited pursuant to the Budapest Treaty with the ATCC at the address disclosed above. The DNA vector was deposited with the ATCC on September 17, 1997 and was assigned Accession No. 209276. The pSUN27 vector includes a CMV promoter, murine light chain leader sequence, Kozak consensus sequence, and the murine Cκ gene intron and exon sequence. See Fig. 10 and Near, et al., *supra.*

The pSUN27 vector is designed to allow fusion of a variety of sc-TCR molecules to the murine Cκ chain. For example, to insert a desired sc-TCR molecule into the pSUN27 vector, the vector polylinker sequence can be digested with the restriction enzymes Age I and BSTBI and ligated to the sc-TCR molecule digested with Age I and BSTBI or Cla I. In addition, the pSUN9 vector can accommodate several Ig-C_{L} chains or other Ig-C_{L} chain fragments. For example, to construct a soluble fusion protein comprising a human Cκ chain, the pSUN9 vector was digested with BstB1 and Xho1. A human kappa fragment was obtained from a vector pDRHK by restriction digestion with EcoNI and Xho1 and cloned into the BstB1 and Xho1 site of the pSUN9 vector.

The pDRHK vector has been deposited pursuant to the Budapest Treaty with the ATCC at the address disclosed above. The DNA vector was deposted with the ATCC on September 17, 1997 and was assigned Accession No. 209274. The pDRHK vector is a mammalian expression vector which includes a CMV promoter, mouse IgC kappa leader peptide, cloning region, mouse kappa intron and human kappa constant domain exon sequence.

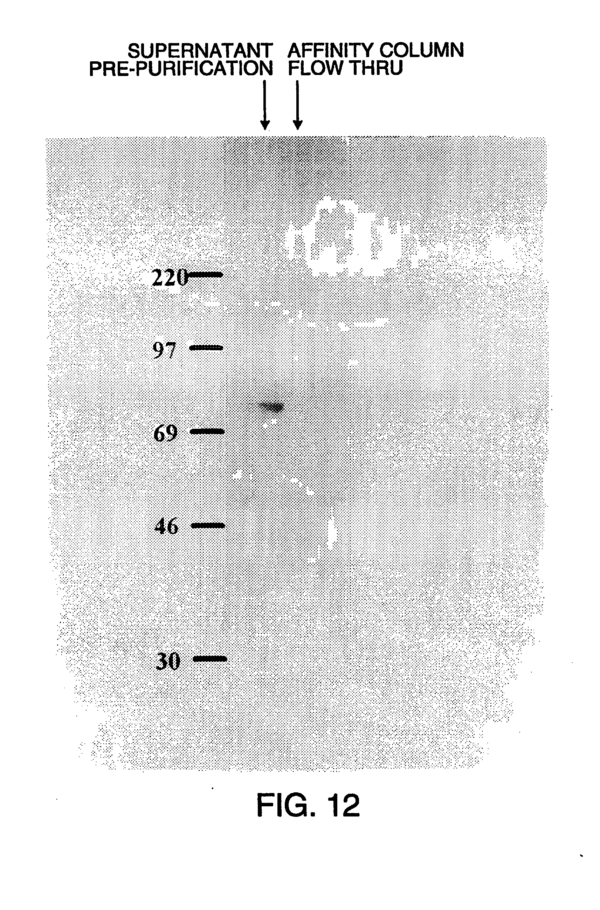

Figure 12 shows a Western blot of single-chain TCR (DO11.10)-kappa fusion produced by CHO cells. Supernatant taken before affinity purification (Lane 2) and from flow-thru fraction (lane 3). 5 ul samples were mixed in a 1:1 ratio with 2x SDS-cracking buffer and run on a 12% SDS-PAGE under denaturing and non-reducing conditions. Protein was detected using a two step detection system. First, the membrane was probed with biotin labeled H57 mAb at a 1:5000 dilution followed by incubation with streptavidin-horseradish peroxidase (1:2500). The data indicates expression of the sc-TCR-kappa fusion by CHO cells and suggests the H57 mAb coupled to CNBr activated sepahrose beads can be used to efficiently remove the sc-TCR-kappa fusion from culture supernatant.

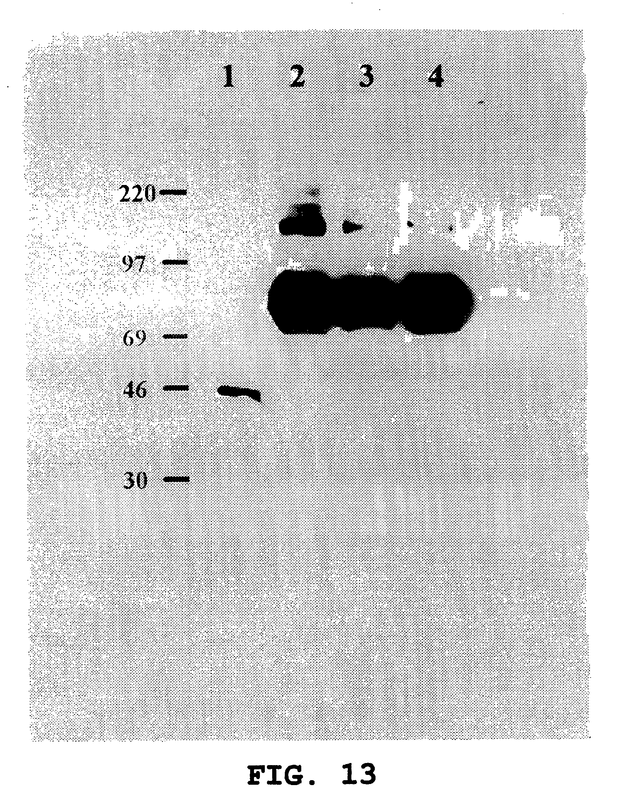

Figure 13 shows a Western blot of purified sc-TCR(DO11.10)-kappa fusion. 300 ml of culture supernatant containing the sc-TCR-kappa fusion (see Figure 12) was passed over an H57 mAb affinity column. Standard affinity chromatography procedures were followed and the sc-TCR-kappa fusion was eluted off the column in 0.1M glycine pH 3.0. Lane 1 represents sc-TCR(DO11.10) produced in E. coli (positive control) and migrates at approximately 50 kilodaltons (kD). Lanes 2-4 represent dilutions of the purified sc-TCR(DO 11.10)-kappa fusion going from most concentrate (lane 2) to least concentrate (Lane 4). Membranes were probed essentially the same way as described in Figure 12.

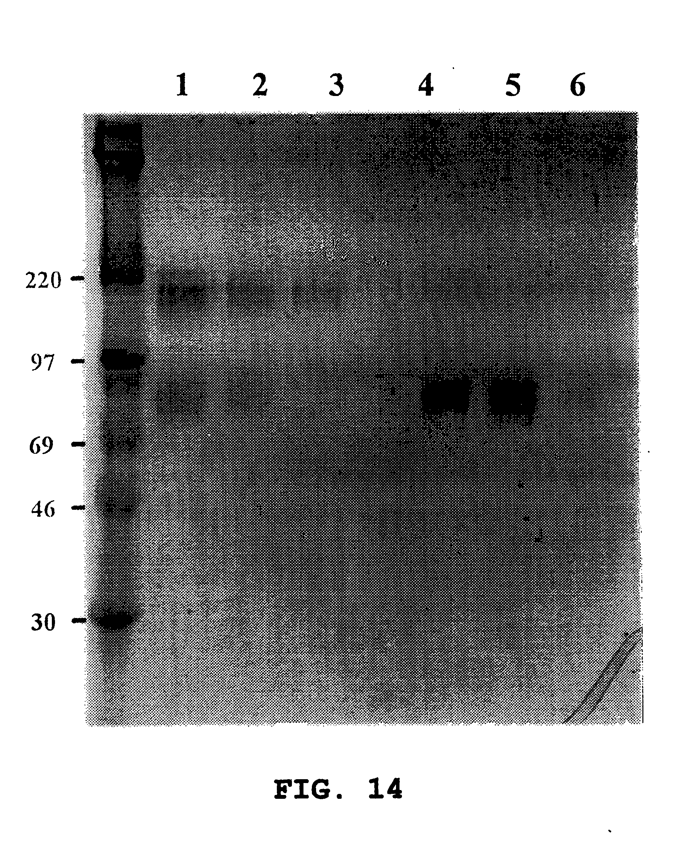

Figure 14 shows a Commassie Brilliant blue stain of the purified sc-TCR-kappa fusion. The purification was carried out using a two antibody affinity columns. For the first step culture supernant containing the sc-TCR-kappa fusion was passed over a KJ-1 antibody column. KJ-1 is an anti-clonotypic mAb that recognizes the correctly paired Va and Vb chains of DO11.10 TCR. Lanes 1(greatest)-3(least) represent aliquots of purified sc-TCR-kappa fusion. After pooling fractions containing purified sc-TCR-kappa fusion from the KJ-1 column the sample was then passed over a H57 mAb column. Lanes 5&6 illustrate enrichment of the correct size protein molecule. Sample were run under denaturing and non-reducing conditions on a 12% SDS-PAGE and after completion the gel was stained with commassie brilliant blue.

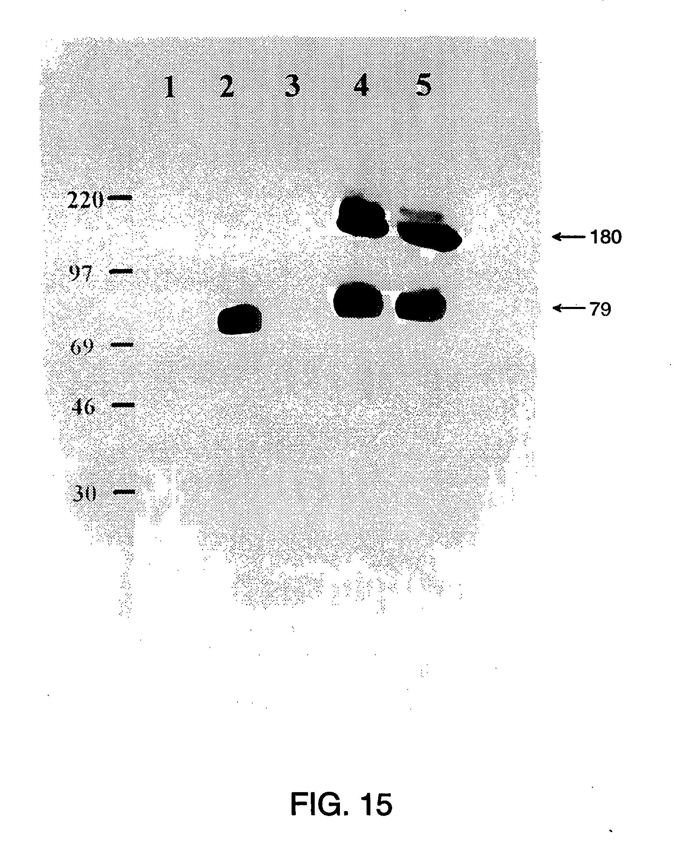

A transient expression study of three-domain sc-TCR-kappa fusion and four-domain sc-TCR-kappa fusions was conducted. COS cells were transiently transfected with either plasmid pKC60-M (pSUN27), three domain sc-TCR or pKC75-M, the four-domain sc-TCR. After 48 hours, 1 ml of culture supernatant was incubated overnight with 0.5 ug of mAb F23.1 (anti-Vb8.2). The following day the immune complexes formed between the sc-TCR-kappa fusion and the F23.1 mAb were precipitated using a Goat anti-mouse coated magnetic bead(Dynal). After extensive washing the beads were suspended in 10 ul of 1x SDS-cracking buffer and boiled. After electrophoresis on a 12% SDS-PAGE gel under denaturing and nonreducing conditions and transfer of proteins, probing of nylon membranes for sc-TCR-kappa fusion molecules was carried out using biotin labeled H57 mAb and streptavidin-HRP conjugate. The results are depticted in Fig. 15. Lane 1 is a negative control F23.1 incubating with supernatant from mock transfected COS cells; lane 2 pSUN27 (three-domain sc-TCR, consists of a Vα-VβCβ-kappa fusion); lane 4 and 5 represent two different pKC75-M isolates (four-domain st-TCR, includes in addition the Cα fragment). Significantly, the four-domain sc-TCR-kappa fusion is expressed as well if not better than the three-domain sc-TCR and unlike the pSUN27 sc-TCR-kappa fusion, the pKC75-M fusion exists as both a homodimer and as a monomer in an apparently equivalent ratio.

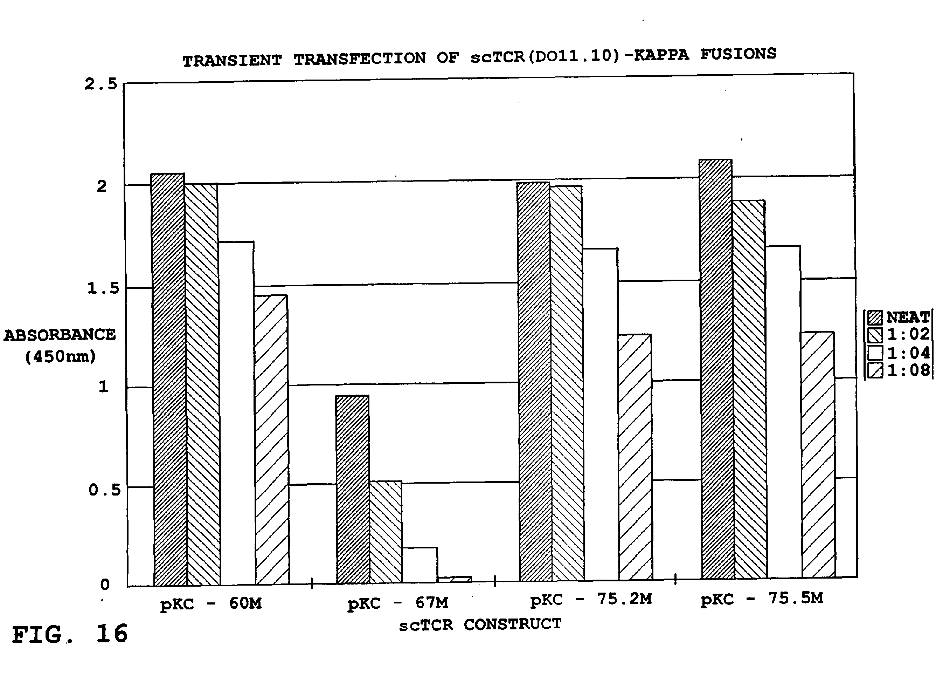

Figure 16 shows an ELISA of transiently expressed sc-TCR(DO 11.10)-kappa fusion variants. Assaying for the sc-TCR fusion was carried out using a sandwich ELISA in which the KJ-1 anti-clonotypic mAb was used for capturing and the biotin-labeled H57 mAb and strepavidin-HRP was used for detection of bound sc-TCR-kappa fusion.

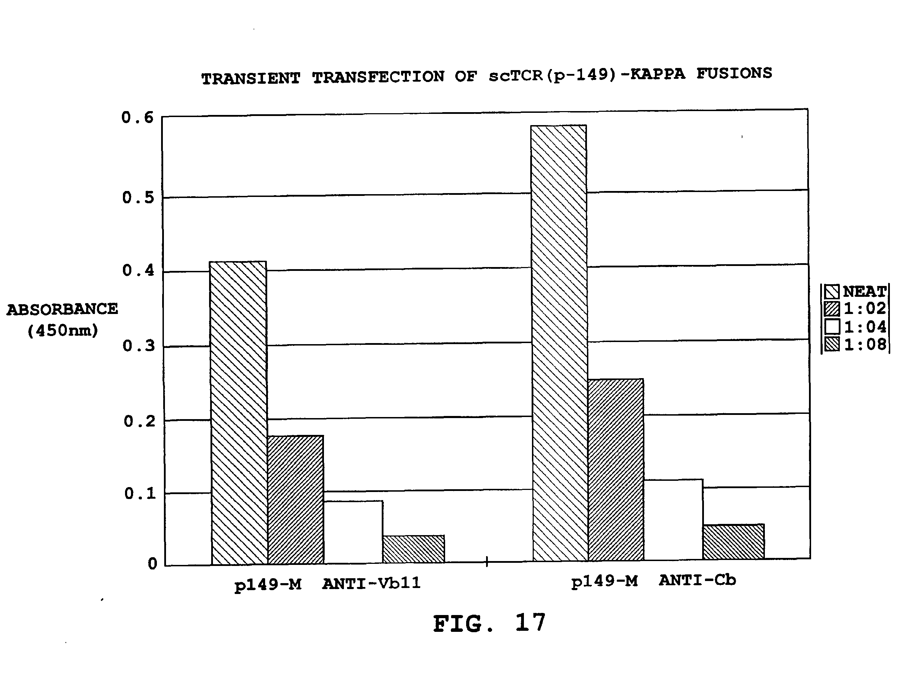

Figure 17 shows a sandwich ELISA to detect mammalian cell expression of sc-TCR(p-149)-kappa fusion protein. Wells were coated with a mAb specific for Va2.3 and bound sc-TCR fusion was detected with either mAb anti-Vβ11 or mAb H57.

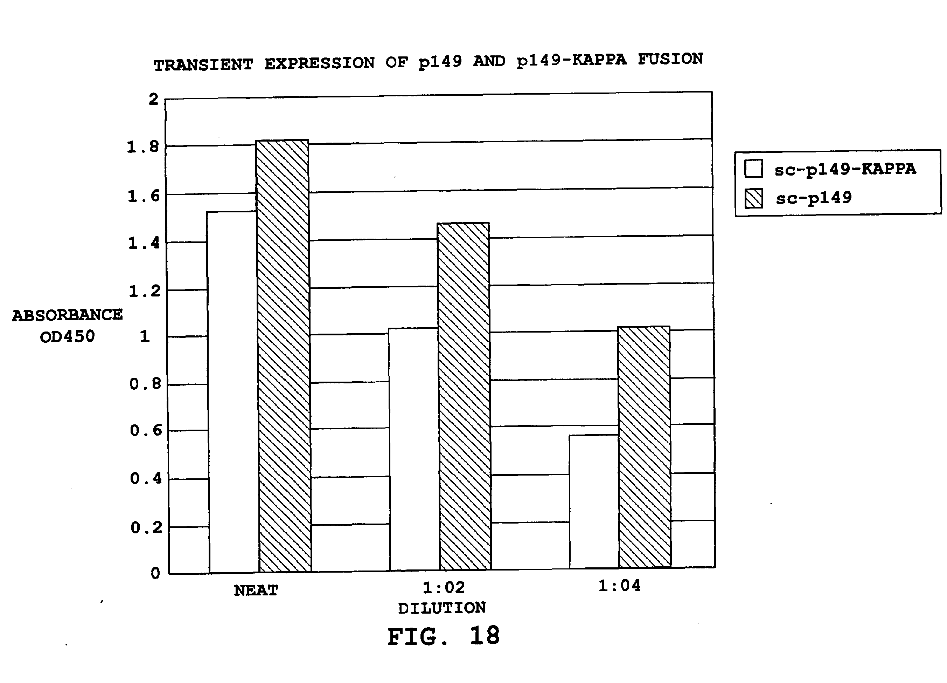

Figure 18 shows transient expression of scTCR p149 and scTCR p149 kappa fusion proteins in COS cells. The design of the scTCR was Vα-Cα_{8.2}-Vβ/Cβ with or without the kappa constant domain. Expressed protein was detected using a sandwich ELISA in which cells were coated with α-V_{α2.1} mAb and the receptor was detected by binding with α-V_{β11.0} biotin-labeled mAb and streotavidin -HRP as conjugate.

### Example 6- Construction and Expression of Soluble p-149 sc-TCR Fusion Proteins in E. coli

The T cell clone, p-149, recognizes a peptide (STPPPGTRV) of Hu wild-type p53 restricted by HLA-A2.1. See Theobald, M., et al., (1995) PNAS (USA) 92, 11993-11997. The T cell receptor gene was cloned into a three domain single-chain format previously shown to produce soluble TCR and functional receptor molecules.

In brief, mRNA was isolated from the T cell clone and cDNA was made using the Marathon cDNA Amplification Kit (Clontech). The cDNA was used as a template in polymerase chain reaction (PCR) with primers KC 171 (SEQ ID. NO. 134) and KC 173 (SEQ ID.NO.136) to generate a 5'SfiI-3'SpeI V( chain fragment and again with primers KC 171 (SEQ ID. NO134) and KC 174 (SEQ ID. NO. 137) to produce a similar V (fragment with seven amino acids of the C( chain included. The same cDNA was then used as a PCR template with primers KC172 (SEQ ID. NO. 135) and KC176 (SEQ ID. NO. 138) to generate a 5'XhoI-3'XmaI VβCβ chain fragment. The Cβ chain was truncated just before the cysteine residue at amino acid 127 of the full-length Cβ chain.

The a and β chain fragments were cloned into the pGEM-T Easy Vector System for DNA sequence determination. Correct fragments were restriction digested and cloned into the expression vector pKC60 or pKC67 to create a Vα-(G₄ S)₄-VβCβ sc-TCR molecule.

The pKC60 and pKC67 vectors were digested with the appropriate restriction enzymes to drop out the previous gene fragment encoding the single-chain molecule and allow for ligation with the p-149 a and β chain fragments. The new vectors were named pNAG1 and pNAG2. The DNA inserts of the pNAG1 and pNAG2 vectors are illustrated in Fig. 9B.

The DNA fragment containing the VβCβ gene was PCR amplified as a 5'SfiI-3XmaI fragment using primers KC 199 (SEQ ID. NO. 139) and KC 176 (SEQ ID. NO.138). It was sequenced and cloned into the expression vector as a β chain-only negative control.

K91 *E.coli* cells were transformed with the sc-TCR construct pNAG1. The transformed cells were grown overnight in high phosphate medium to prevent protein induction, then washed and resuspended in phosphate deficient mediums to allow for sc-TCR protein expression. Briefly, after overnight growth in shaker flasks the cells were washed several times in phosphate deficient media. Expression of the sc-TCR fusion protein was initiated by resuspending washed cells in phosphate deficient media. After about a four hour induction, the cells were harvested. Typically, a four hour induction time gave suitable levels of soluble sc-TCR fusion protein as determined, e.g., by Western blot analysis. Greater yields of the sc-TCR fusion protein (e.g., between approximately 10 to 400 mg) under phoA promoter control can be obtained by propagating cells in 3 and 10 liter fermentors or other large-scale preparative container such as a bioreactor.

It was found that expression of the sc-TCR fusion proteins under slow induction conditions facilitated soluble expression of the fusion protein.

### Example 7- Construction and Expression of Soluble p-149 sc-TCR Fusion Proteins in Mammalian Cells

The *E. coli* DNA constructs pNAG1 and pNAG2 were used as the template DNA in PCR with primers KC203 (SEQ ID. NO. 140) and KC204 (SEQ ID. NO. 141) to generate a 5'AgeI-3'CIaI sc-TCR-kappa chain fusion fragment. The same template DNA can be amplified by PCR with primers KC203 (SEQ ID. NO. 140) and KC205 (SEQ ID. NO. 142) to generate a 5'AgeI-3'CIaI sc-TCR for expression without a kappa chain. Mammalian cells were transfected with the DNA and screened for sc-TCR protein expression by an ELISA assay.

### Example 8 - Production of sc-TCR Fusion Protein Strains

It will be desirable to select a specific *E. coli* strain to maximize soluble expression of a sc-TCR fusion protein. For example, several well-known *E. c*oli strains can be analyzed for capacity to express the sc-TCR fusion proteins (e.g., XL1-B, MM294, TB1, UT5600 and K91Kan). Briefly, the host cell strains can be transformed with, e.g, pKC60, pKC73, pKC74, or pKC75 and grown under conditions which favor expression of the encoded sc-TCR fusion protein. Transformed strains are then adapted to grow in phosphate media at 30°C. Induction of the sc-TCR fusion proteins is preformed by growth in media deficient in phosphate. The level of sc-TCR expression can be determined by several conventional methods including a Western blot probed with an antibody that specifically recognizes the sc-TCR fusion protein.

Briefly, the Western blot is conducted by adding 5 ul of a 5 OD/ml cell suspension of transformed cells on a 12% SDS-PAGE gel and transferred to a blot according to standard Western blot techniques. The sc-TCR is detected by probing the blot with, e.g., an anti-EE tag antibody licensed from Gemot Walter's laboratory at the University of San Diego, San Diego California. Alternatively, other anti-EE tag antibody and EE-tags can be used (e.g., those obtained from Pharmacia). Antibody binding was followed by the addition of a goat anti-mouse-HRP labeled conjugate (Jackson Laboratories).

In the pending U.S. Appl. No. 08/813,781 it was found that the *E. coli* strain K91KAN was well-suited for the expression of fusion proteins comprising sc-TCR molecules, particularly the pKC60 vector. The K91KAN strain may produce significant amounts of the present sc-TCR fusion proteins.

### Example 9 - Purification of sc-TCR Fusion Proteins

The sc-TCR fusion proteins disclosed herein can be purified if desired from transformed cells by immunoaffinity chromatography in accordance with conventional methods. Purification schemes for sc-TCR fusion proteins comprising bacteriophage coat protein have been disclosed in the pending U.S. Appl. No. 08/813,781.

Briefly, one suitable purificaton scheme is as follows. A chromatographic column can be coupled to about 5 mg of hamster anti-mouse αβ TCR antibody H57-597 (ATTC Accession No. HB-218) per ml of protein-A coated sepharose beads. *E. coli* lysates are prepared by solubilizing 50 g of fermentor-derived cell paste in 100 ml of solubilization buffer (0.05 M Tris, pH 8.0, 150 mM NaCl and 5 mM EDTA. Resuspended cells were lysed by two passages through a French Press. Insoluble material is removed by centrifugation at 10,000 g for 20 minutes. The supernatant is retained and applied to the antibody column at a flow rate of 0.2 ml/min. Subsequently, the column is washed with 20 column volumes of PBS and bound sc-TCR fusion proteins are eluted in 0.1 M glycine pH 3.0 and one ml fractions are collected in tubes containing 0.05 ml of 2 M Tris, pH8.0. Fractions containing sc-TCR are pooled and dialyzed overnight against 4 liters of PBS. The next day purified protein is concentrated 5 to 10 fold using a centricon filtration device (mw 100 cutoff).

The sc-TCR fusion protein preparations can be evaluated for purity by several known methods such as electrophoresis on an SDS-PAGE gel followed by Commassie Brilliant Blue staining. Protein integrity can be determined by the method disclosed in Example 5 above or by using the antibody H57-597. Alternatively, for DNA vectors comprising an operably linked EE tag, an EE tag antibody can be employed as a probe. The EE antibody recognizes a nine amino acid linear epitope. Anti-Glu-Glu (EE) EEEEYMPME (SEQ ID NO 98).

### Example 10 - Characterization of sc-TCR Fusion Proteins

The following assays disclosed in the pending U.S. Application No. 08/813,781 can be used to characterize the sc-TCR fusion proteins of the invention.

### A. Molecular weight

As noted in Example 5, above, for the DO11.10 sc-TCR fusion protein, the molecular weights of other sc-TCR fusion proteins can be determined by conventional SDS-PAGE gel electrophoresis followed by staining such as with silver stain or Coomassie Brilliant Blue. The U.S. Application No. 08/813,781 gives examples of molecular weight determinations of sc-TCR molecules using SDS-PAGE electrophoresis. It is expected that the molecular weight of the most sc-TCR proteins will be generally in the range of 40 to 60 Kd, preferably about 45 to 58 Kd.

### B. Antibody Competition

A panel of anti-αβ TCR mAbs can be used to characterize the sc-TCR fusion proteins disclosed herein particularly those comprising the mouse kappa chain. For example, the hamster mAb, H57-597, recognizes an epitope on the C-β region. It is possible to conduct competition tests to analyze competing effects between selected antibodies that bind closely spaced epitopes on the single-chain variable chain. For example, the MR5-2 and H57-597 antibodies can be used to map epitopes on sc-TCR fusion proteins comprisin suitable Vα,Vβ regions. As disclosed in the pending U.S. Application No. 08/813,781 it was found that the MR5-2 and H57-597 antibodies recognized closely spaced epitopes. Other antibodies can be used to further characterize the sc-TCR portion of the soluble fusion proteins, including those that specifically recognize tags such as the nine amino acid EE sequence.

### C. Conformational Folding

A variety of anti-idiotypic mAbs can be used to test for proper folding of Vα,β regions in the sc-TCR fusion proteins disclosed herein. For example, as disclosed in the U.S.Appl. No. 08/813,781, the fusion protein encoded by the pKC60 DNA vector was properly folded as evidenced by binding of the anti-V-β 8.2 antibodies (MR5-2 and F23.1) and with anti-idotype mab KJ1 (a kind gift from Drs. Kappler and Marrack). Briefly, the sc-TCR fusion protein was incubated with the anti-V-β 8.2 antibody overnight at 4°C, followed the next day by binding with goat anti-mouse coated magnetic beads (Dynal). The material was allowed to incubate for an additional hour at room temperature (RT). Immune complexes were precipitated according to standard procedures. Beads were then extensively washed with 0.5 ml of PBS + 0.5 M NaCl to remove non-specifically bound proteins. After 4 washes, the magnetic beads were resuspended in 50 ul of cracking buffer containing SDS with and without β-2 mercaptoethanol and boiled for 3 minutes. The magnetic beads were removed and the solution was loaded onto a 12% SDS-PAGE and run for 1 hour at 120 volts. The samples were electrophoresd and a Western blot was performed on the samples by probing the blot with anti-TCR antibody-HRP labeled (H57 obtained from Pharmingen) or anti-EE tag antibody.

Example 5 above discloses particular anti-iodiotypic antibodies that were used to test for proper folding of the DO11.10 sc-TCR protein fusion.

### D. Surface Plasmon Resonance (BioCore)

Surface Plasmon Resonance can be used to characterize the sc-TCR fusion proteins of the present invention. For example, as disclosed in the pending U.S. Application No. 08/813,781, the sc-TCR fusion protein encoded by pKC51 was characterized using surface plasmon resonance. Fusion proteins were detected in a conventional sandwich assay by first capturing the sc-TCR molecule on a biosensor chip coated with either MR5-2 or H57 antibody in general accordance with the manufacturer's instructions (Pharmacia). In general, if one of the antibodies was used to capture a sc-TCR fusion protein, the other antibody was used to form the sandwich complex. The data indicated that the two antibodies recognized different regions of variable chain and competed for binding to the sc-TCR.

Superantigens (ie. SAg) are capable of binding some V-β chains independent of presentation in an MHC format. The interaction between TCR and SAg occurs within the hypervariable 4 (HV4) region of the V-β chain. Since SAg interaction with the HV4 region is conformationally dependent, surface plasmon resonance can be used to determine if the sc-TCR fusion protein can bind to SAg coated on chips. SAgs are known to bind TCR V-β8.2. The highest affinity reported using surface plasmon resonance is believed to be 5x10⁻⁵ moles, thereby making the interaction comparable to TCR-MHC/peptide interactions.

It is within the scope of the present invention to construct sc-TCR fusion proteins comprising single-chain variable regions that bind superantigens. For example, as disclosed in the pending U.S Application No. O8/813,781, the *Streptococcus* SAg known as SEC3 (Toxin Technology, Tampa FL.) binds sc-TCR molecules comprising V-β8.2 regions. As disclosed, the superantigen was coupled to a chip using standard amine coupling chemistry. The purified fusion protein was passed over the chip at a flow rate of 2 ul/min at concentrations ranging from approximately 2 uM to 16 uM. As a control, non-specific binding of the fusion protein to a blank chip was done in parallel with experiments measuring specific binding to the SEC3 coated chip. Binding data was compiled by comparing the difference in sc-TCR binding between the two chips. The data showed that there was a specific interaction between the sc-TCR molecule and the SEC3 SAg on the chip. These data indicated that the sc-TCR molecule included a conformationally correct V-β8.2 region.

The pending U.S. application No. 08/813,781 disclosed that fusion protein produced by the PKC60 vector bound the SEC3 superantigen as determined by Biocore. Related Biocore experiments can be performed on sc-TCR fusion proteins of the present invention capable of binding supernatigens to determine correct folding.

### Examples 11 - Activity of Fusion Proteins in an OVA Specific T-cell Hybridoma Binding Assay

Assays for testing functionality of MHC complexes comprising sc-MHC class I or II molecules have been disclosed in said published PCT Application Nos. PCT/US95/09816, PCT/US97/01617 as well as said pending U.S. Patent Application Ser. No. 08/382,454. More particularly, the published PCT application PCT/US97/01617 discloses T-cell hybridoma cell assays, cells for use in such assays (e.g., DO11.10), sc-MHC class I and II molecules carrying a covalently linked or non-covalently bound presenting peptide (e.g., OVA or HSV gD12 peptide).

The previously disclosed assays are particularly suited to determine the functionality of the sc-TCR fusion proteins disclosed herein. Generally stated, the assays are cell-based competition inhibition assays that can be used to determine the function and biological activity of sc-TCR fusion proteins. For example, soluble fusion proteins produced in Examples 1 and 2 above can be used to block DO11.10 cells from engaging superantigen or an antigen in the context of an MHC molecule. In accordance with the previously disclosed methods, the interaction is detected by measuring IL-2 production.

### A) T-cell Hybridoma Cell Assay

A T-cell hybridoma assay has been disclosed in the published PCT Application Nos. PCT/US95/09816, PCT/US97/01617, and pending U.S. Patent Application Ser. Nos. 08/382,454. The assays are readily adapted to test the functionality of the sc-TCR fusion proteins of the present invention.

Briefly, the DO11.10 T-cell hybridoma line expresses a cell surface T-cell receptor specific for the 17 amino acid OVA peptide fragment (amino acids 323-339) derived from chicken ovalbumin. The OVA peptide is presented to DO11.10 cells by APCs expressing the murine class II MHC molecule I-Ad. When the peptide is presented by the appropriate APC, DO11.10 cells respond by producing IL-2, which can then be used as a measure of T-cell activation. An exemplary T-cell hybridoma cell assay is described briefly as follows:

The sc-IA^{d}/OVA complex is diluted in DPBS (without Mg²⁺ and Ca²⁺ ions) with the sc-TCR fusion protein and passively coated to wells of a 96 well plate. Preferably, the sc-IAd/OVA peptide includes a covalently linked presenting peptide. After an overnight incubation at room temperature, the wells can be washed two times in DPBS without Mg²⁺ and Ca²⁺ ions. About 1x10⁵ DO11.10 cells are incubated in the presence or absence of wells coated with 0.5 µg MHC/peptide molecules for 4 or 7 hours at 37°C. In related experiments, the sc-TCR fusion protein can be added along with the DO11.10 cells.

To demonstrate specificity of sc-TCR fusion proteins derived from the DO11.10 line (See examples 1 and 2 above), a second T-cell hybridoma can be used (gD) which also is 1Ad restricted but recognizes an HSV peptide. The gD T-cell hybridoma has been disclosed in the published PCT application PCT/US97/01617. This assay is used to demonstrate that sc-TCR fusion proteins are capable of inhibiting IL-2 production by DO11.10 hybridoma cells and have little if any inhibitory effect on IL-2 production by gD12 T-cell hybridomas. Cultures are carried out in complete culture medium (RPMI 1640 supplemented with 10% FBS, penicillin/streptomycin and L-glutamine) in 96 well flat bottom microtiter plates. After either a 4 or 7 hour incubation, culture supernatant is assayed for the presence of IL-2 using an IL-2 sandwich ELISA.

A suitable IL-2 detection protocol has been disclosed in said published PCT Application Nos. PCT/US95/09816, PCT/US97/01617 as well as said pending U.S. Patent Application Ser. Nos. 08/382,454 and 08/596,387. A suitable IL-2 sandwhich ELISA protocol is one conducted essentially as described by PharMingen. Briefly, wells are coated with 50 µl of a 2 µg/ml rat anti-mouse IL-2 antibody. The antibody binds to the plastic by passive diffusion after an overnight incubation at 4°C. The plate is washed two times in PBS/0.5% Tween-20 and then 100 µl of cell culture supernatant is added to each well and allowed to incubate for four hours at room temperature. The plate is then washed in PBS/Tween six times prior to adding the second antibody which is a biotinylated rat anti-mouse IL-2 antibody. This antibody incubates at RT for one hour and the plate is then washed six times in PBS/Tween. Finally, 100 µl of 25 ug/ml of strepavidn-peroxidase is added to each well and incubated for 30 minutes at RT. After 8 washes in PBS/Tween, 100 µl of ABTS substrate is added and the signal is read at OD 405nm. The concentration of IL-2 produced is quantitated by plotting against a IL-2 standard curve.

Optimal doses of I-A^{d}/peptide molecules for activation of DO11.10 and gD12 cells are those which provide slightly submaximal responses. Accordingly, the experiments can be conducted with wells coated with 0.5 µg of I-A^{d}/peptide and a 4 or 7 hour incubation.

In general, soluble sc-TCR fusion proteins of the present invention can be tested for ability to block IL-2 production in DO11.10 cells over a concentration ranging from about 10⁻⁹ to 10⁻⁴ M. Tests can be performed with approximately a 10:1 to 1:1 molar ratio of the soluble sc-TCR and the soluble I-Ad/peptide molecules coated on the plate. Concentrations can be adjusted as needed. Optimally, a decrease in DO11.10 IL-2 production compared to the gD12 IL-2 production following pre-incubation with the soluble sc-TCR fusion proteins will indicate that the soluble sc-TCR fusion proteins can suppress immune responses in a TCR specific manner.

Because the TCR has a low binding affinity for MHC/peptide, it may not always be possible to observe a decrease in IL-2 production using the sc-TCR fusion protein in inhibition assays. However, avidity of the interaction can be increased by making polyvalent sc-TCR fusion proteins as described below. By increasing avidity of the sc-TCR fusion proteins (and hindering dissociation thereof), it is believed fewer TCRs will have the opportunity to bind the MHC/peptide complex.

### Example 12 - Preparation of Polyvalent Fusion Proteins

Several well-known methods are available for making polyvalent molecules. Polyvalent sc-TCR fusion proteins can be produced by biotinylating the sc-TCR fusion proteins of the invention in accordance with standard methods, followed by cross-linking the molecule after strepavidin addition. Biotin-streptavidin conjugation produces the polyvalent sc-TCR fusion protein, typically in a tetrameric format.

The present polyvalent sc-TCR fusion proteins can also be made by covalently linking the fusion protein to latex beads (Polysciences, Inc. Warrington, PA) in accordance with known methods (see e.g., Newman, S.L. et al. J. Immunol. (1995) 154, 753). For example, the sc-TCR fusion proteins can be directly coupled to the beads through either amine groups or disulfide groups. Denaturation of the sc-TCR can be minimized by coating the latex bead with either strepavidin or an antibody which specifically binds the sc-TCR. For example, the EE-tag antibody can be used to coat the latex bead in cases where the sc-TCR includes an EE-tag as described above.

### Example 13 - Effects of sc-TCR Fusion Protein on Antigen Stimulated T-cell Proliferation in vitro

The sc-TCR fusion proteins of the present invention can be tested for capacity to suppress antigen stimulated T-cell proliferation.

For example, in an exemplary method disclosed in said published PCT Application Nos. US95/09816, PCT/US97/01617 and pending U.S. Patent Application Ser. No: 08/382,454, T-cells are first isolated from a mammal such as mice. For example, OVA-primed T-cells can be obtained from BALB/c mice (MHC Class II: I-Ad) by immunizing with 50 µg of OVA 323-339-KLH in complete Freund's adjuvant, subcutaneously at the base of the tail (see Harlow and Lane, *supra*). For example, two immunizations can be performed at 7 day intervals and, one week after the second injection, mice can be sacrificed and the inguinal and parasitic lymph nodes removed and dispersed into a single cell suspension. Subsequently, the single cell suspension can be depleted of antigen presenting cells by incubation on nylon wool and Sephadex G-10 columns. The purified T-cell populations can be further incubated either with Click's medium alone, or with sc-TCR fusion proteins dissolved in Click's medium.

Activated B cells from BALB/c mice can be used as APC in a conventional B cell proliferation assay. For example, B cells can be prepared by culturing spleen cells with 50 µg/ml of LPS (ie. liposaccharides) for 48 to 72 hours at which time activated cells can be isolated by density gradient centrifugation on Ficoll/Hypaque (Pharmacia). Activated B cells can then be pulsed with the OVA 323-339 peptide for 3 hours, washed extensively, fixed with paraformaldehyde to inhibit proliferation of B cells, and added to purified T-cells alone or T-cells plus soluble sc-TCR fusion proteins. See generally Selected Methods in Cellular Immunol. (1980) B.B. Mishell and S.M. Hiigi W.H. Freeman and Co. San Francisco.

The sc-TCR fusion proteins prepared above in Example 1-2 can be added to inhibit T-cell binding to the APCs.

A standard B cell proliferation assay can be carried out in 96 well round bottom microtiter plates at 37°C, 5% CO2 for 3-5 days. Wells can be pulsed with WST-1 (Boehringer Mannheim) reagent for 4 hours prior to termination of cultures. The optical density of the cultures can then be recorded. The degree of peptide-reactive T-cell proliferation in the presence of the sc-TCR fusion protein will be indicative of the TH cell responses (ie. clonal expansion) that took place in the mice following immunization.

### Example 14 - Effects of sc-TCR Fusion Proteins on Antigen Stimulated T-cell Proliferation in vivo.

The sc-TCR fusion proteins of the present invention can be further tested for inhibition of T-cell clonal expansion *in vivo* in accordance with methods disclosed previously in said published PCT Application No. US95/09816, as well as said pending U.S. Patent Application Ser. Nos. 08/382,454 and 08/596,387.

For example, three test groups can be set up as follows: 15 BALB/c mice can be injected intraperitoneally (IP) with approximately 10 to 100 ug of OVA 323-339-KLH conjugate in Complete Freund's adjuvant to induce an immune response to the OVA 323-339 peptide. On the day before immunization and 2 days after immunization with OVA-KLH, 5 of the mice can be injected IP with approximately 10 to 100 µg of the sc-TCR fusion protein derived from the DO11.10 cells (Examples 1 and 2) in PBS. The sc-TCR fusion protein will bind to the I-Ad/OVA MHC Class II molecules and reduce or eliminate engagement by TCR molecules on antigen-specific T-cells. The remaining 10 mice can be used as controls. For example, 5 of the mice can receive PBS and the other 5 can receive sc-TCR fusion protein. The mice can be sacrificed 10 days after immunization. The lymph nodes can be removed and dispersed into a single cell suspension. The suspension can be depleted of antigen presenting cells by incubation on nylon wool and Sephadex G-10 columns.

The resulting purified T-cell populations can be incubated with APCs pulsed with the OVA 323-339 peptide. Activated B cells from BALB/c mice can be used as APCs in the proliferation assay. The B cells can be prepared by culturing mouse spleen cells with 50 µg/ml of LPS for 48 to 72 hours at which time activated cells can be isolated by density gradient centrifugation on Lymphoprep. Activated B cells can then be pulsed with the OVA 323-339 peptide for 3 hours, washed extensively, fixed with paraformaldehyde to inhibit proliferation of B cells, and added to purified T-cells.

The T-cell proliferation assay can be carried out in 96 well round bottom microtiter plates at 37°C, 5% CO₂ for 3-5 days. Wells can be pulsed with WST-1 reagent for 4 hours prior to termination of cultures and then read at different absorbencies. The degree of peptide-reactive T-cell proliferation in this assay will be indicative of the Th cell response (ie. clonal expansion) that took place in the mice following immunization. It is expected that co-injection of the sc-TCR fusion proteins with either the OVA-KLH or the HSV-KLH immunization will limit the amount of clonal expansion and subsequent *in vitro* proliferation of the OVA-reactive T-cell lines without affecting expansion of the HSV-reactive T-cell lines.

### Example 15 - Suppression of a Murine Autoimmune Disease

Experimental allergic encephalomyelitis (EAE) is a murine autoimmune disease that is generally recognized to be an animal model for multiple sclerosis. Encephalitogenic regions of two proteins, myelin basic protein (MBP amino acids 91-103) and proteolipoprotein (PLP amino acids 139-151) have been defined. See generally Martin, R. et al. (1992) Ann. Rev. Immunol. 10: 153.

In the SJL mouse strain, EAE can be induced to develop following immunization with the encephalitogenic peptide or adoptive transfer of MBP-reactive T-cells. To determine whether treatment with soluble anti-MBP 91-103 or anti-PLP 139-151 T-cell receptors will prevent EAE development after T-cell activation, SJL mice can be injected with MBP 91-103 and PLP 139-151 reactive T-cell blasts *in vivo.* Suitable assays to detect T-cell expansion in such mice have been disclosed in said published PCT Application Nos. US95/09816, PCT/US97/01617 as well as said pending U.S. Patent Application Ser. No. 08/382,454.

As further disclosed in said published PCT Application Nos. US95/09816, PCT.US97/01617 and pending U.S. Patent Application Ser. No. 08/382,454, EAE can be induced in SJL mice by immunization with approximately 400 µg of MBP 91-103 in complete Freund's adjuvant on the dorsum. Ten to 14 days later, regional draining lymph node cells can be harvested as described above and cultured in 24-well plates at a concentration of 6x10⁶ cells per well in 1.5 ml of RPMI 1640 medium/10% fetal bovine serum/1% penicillin/ strepomycin/ MBP at 50 µg/ml. After a 4 day *in vitro* stimulation, MBP 91-103 reactive T-cell blasts can be harvested via a Ficoll/Hypaque density gradient (Pharmacia), washed twice in PBS, and 1.3x10⁷ cells can be injected into each mouse.

Mice which receive encephalitogenic MBP 91-103 reactive T-cells can be further injected with approximately 100 µg of an sc-TCR fusion protein comprising Vαβ chains specific for MBP 91-103, 100 µg of an sc-TCR fusion protein Vαβ chains specific for the PLP 139-151 (negative control), or saline (sham control) on day 0, 3, and 7 i.v. (total dose 300 µg). Clinical and histological evaluation can be performed to confirm that the sc-TCR molecule reactive to MBP 91-103 + IAs inhibited the development of EAE in the mice.

As further disclosed in said published PCT Application Nos. PCT/US95/09816, PCT/US97/01617 as well as said pending U.S. Patent Application Ser. No. 08/382,454, to induce EAE in SJL mice with PLP peptide 139-151, the mice can be immunized with PLP peptide 139-151 dissolved in PBS and mixed with complete Freund's adjuvant containing *Mycobacterium tuberculosis* H37Ra at 4 mg/ml in a 1:1 ratio. Mice can then be injected with 152 ug of peptide adjuvant mixture. On the same day and 48 hrs later, all animals can be given 400 ng of pertussis toxin. Adoptive transfer of EAE is then performed as described above.

In accordance with methods described above (see Example 1), TCR DNA can be obtained from normal and EAE diseased SJC mice. The TCR DNA can then be used to construct sc-TCRs which can be ligated into suitable DNA vectors to construct the sc-TCR fusion proteins of the present invention comprising the mouse kappa chain or suitable fragment thereof. The PLP or MBP reactive sc-TCR fusions can then be expressed and purified if desired in accordance with the above examples. The sc-TCR fusion proteins can then be tested for the capacity to prevent development of EAE.

The invention has been described with reference to preferred embodiments thereof. However, it will be appreciated that those skilled in the art, upon consideration of this disclosure, may make modifications and improvements within the spirit and scope of the invention.

## Claims

1. A soluble fusion protein comprising an immunoglobulin light chain constant region or fragment thereof covalently linked to a single-chain T-cell receptor, wherein the single-chain T-cell receptor comprises a V-α chain covalently linked to a V-β chain by a peptide linker sequence and wherein the C-terminus of the V-α chain is covalently linked by the peptide linker sequence to the N-terminus of the V-β chain, and further comprising a C-β chain or fragment thereof covalently linked between the C-terminus of the V-β chain and the N-terminus of the immunoglobulin light chain constant region or fragment thereof, wherein the C-β chain fragment is between about 50 and 126 amino acids in length, and wherein the immunoglobulin light chain constant region or fragment thereof is a Cλ chain, Cκ chain or Cκ chain fragment, wherein the Cκ chain fragment is between about 70 and 150 amino acids in length, and wherein the soluble fusion protein forms a fully soluble and functional molecule.

2. The soluble fusion protein of claim 1 further comprising a C-α chain or fragment thereof, wherein the C-α chain fragment is between about 5 and 90 amino acids in length covalently linked between the C-terminus of the V-α chain and the N-terminus of the peptide linker sequence.

3. The soluble fusion protein of any of claims 1-2, wherein the V-α and the V-β chains are at least 90% identical to T-cell receptor V chains present on a cytotoxic T cell.

4. The soluble fusion protein of any of claims 1-3, wherein the peptide linker sequence is between about 7 to 20 amino acids in length.

5. The soluble fusion protein of any of claims 1-4, wherein the immunoglobulin light chain constant region is a Cκ chain or Cκ chain fragment and the Cκ chain fragment is between about 90 and 120 amino acids in length.

6. The soluble fusion protein of any of claims 1-5, wherein the immunoglobulin light chain constant region is a Cκ chain or Cκ chain fragment and the Cκ chain fragment is between about 100 and 110 amino acids in length.

7. The soluble fusion protein of any of claims 1-6 further comprising at least one protein tag covalently linked to the soluble fusion protein; and/or a protein tag covalently linked between the C-β chain fragment and the Cκ chain, Cλ chain or said fragment thereof.

8. A single-chain T cell receptor produced by contacting the soluble fusion protein of claim 7 with an agent capable of cleaving the protein tag.

9. The single-chain T cell receptor of claim 8, wherein the single-chain T cell receptor V regions have been humanized and the single chain T cell receptor C regions have been humanized.

10. A DNA segment encoding a soluble fusion protein according to any of claims 1-7.

11. The DNA segment of claim 10, wherein the soluble fusion protein further comprises a C-α chain fragment covalently linked between the C-terminus of the V-α chain and the N-terminus of the peptide linker sequence.

12. The DNA segment of claim 11 further comprising a leader sequence, translation initiation signal and a promoter; and/or one or more protein tags.

13. The DNA segment of claim 12, wherein the promoter is phoA and the leader is pelB from *E. coli,* wherein the DNA segment further comprises a ribosome binding site from a gene 10 sequence or the promoter is a cytomeglovirus promoter operably linked to a cytomegalovirus enhancer element and the leader is a mouse kappa chain leader.

14. A DNA vector comprising the DNA segment of any of claims 10-13.

15. A method of isolating a soluble fusion protein of any of claims 1-7, the method comprising:
introducing into host cells a DNA vector comprising a sequence encoding the fusion protein,
culturing the host cells in cultured medium under conditions which permit expression of the fusion protein; and
purifying the fusion protein from the host cell or the medium to isolate the soluble fusion protein.

16. The method of claim 15, wherein the method further comprises contacting an extract of the host cell or the cultured medium with an antibody capable of specifically binding the fusion protein.

17. The method of claim 15, the method comprising:
separating the fused immunoglobulin light chain constant region or fragment thereof from the single-chain T-cell receptor to isolate the soluble single-chain T-cell receptor.

18. The method of claim 15, wherein the host cell is a mammalian cell.

19. Use of a soluble fusion protein of any of claims 1-7, for the preparation of a medicament for immunizing a mammal against T-cell receptor epitopes on the surfaces of pathogenic T-cells.

20. Use of a soluble fusion protein of any of claims 1-7, for the preparation of a medicament for inhibiting T-cell activation in a mammal.

21. Use of a soluble fusion protein of any of claims 1-7 further comprising an effector molecule, for the preparation of a medicament for killing a cell comprising a ligand capable of specifically binding the soluble fusion protein.

22. The use of claim 21, wherein the cell is a tumor cell or a virally-infected cell.

## Patentansprüche

1. Lösliches Fusionsprotein, umfassend eine konstante Region der leichten Kette eines Immunoglobulins oder ein Fragment davon, die bzw. das kovalent an einen einkettigen T-Zellrezeptor gebunden ist, wobei der einkettige T-Zellrezeptor eine V-α-Kette umfasst, die mittels einer Peptid-Linker-Sequenz kovalent an eine V-β-Kette gebunden ist, und wobei das endständige C der V-α-Kette mittels einer Peptid-Linker-Sequenz kovalent an das endständige N der V-β-Kette gebunden ist, und weiterhin umfassend eine C-β-Kette oder ein Fragment davon, die bzw. das kovalent zwischen dem endständigen C der V-β-Kette und dem endständigen N der konstanten Region der leichten Kette des Immunoglobulins oder eines Fragments davon gebunden ist, wobei das C-β-Kettenfragment eine Länge von etwa 50 bis 126 Aminosäuren aufweist und wobei die konstante Region der leichten Kette des Immunoglobulins oder ein Fragment davon eine Cλ-Kette, eine Cκ-Kette oder ein Cκ-Kettenfragment ist, wobei das Cκ-Kettenfragment eine Länge von etwa 70 bis 150 Aminosäuren aufweist, und wobei das lösliche Fusionsprotein ein vollständig lösliches und funktionsfähiges Molekül bildet.

2. Lösliches Fusionsprotein nach Anspruch 1, weiterhin umfassend eine C-α-Kette oder ein Fragment davon, wobei das C-α-Kettenfragment eine Länge von etwa 5 bis 90 Aminosäuren aufweist und kovalent zwischen dem endständigen C der V-α-Kette und dem endständigen N der Peptid-Linker-Sequenz gebunden ist.

3. Lösliches Fusionsprotein nach einem der Ansprüche 1-2, wobei die V-α- und die V-β-Kette eine Identität von mindestens 90 % mit den T-Zell-Rezeptorketten einer zytotoxischen T-Zelle aufweist.

4. Lösliches Fusionsprotein nach einem der Ansprüche 1-3, wobei die Peptid-Linker-Sequenz eine Länge von etwa 7 bis 20 Aminosäuren aufweist.

5. Lösliches Fusionsprotein nach einem der Ansprüche 1-4, wobei die konstante Region der leichten Kette des Immunoglobulins eine Cκ-Kette oder ein Cκ-Kettenfragment ist und das Cκ-Kettenfragment eine Länge von etwa 90 bis 120 Aminosäuren aufweist.

6. Lösliches Fusionsprotein nach einem der Ansprüche 1-5, wobei die konstante Region der leichten Kette des Immunoglobulins eine Cκ-Kette oder ein Cκ-Kettenfragment ist und das Cκ-Kettenfragment eine Länge von etwa 100 bis 110 Aminosäuren aufweist.

7. Lösliches Fusionsprotein nach einem der Ansprüche 1-6, weiterhin umfassend mindestens ein Protein-Tag, das kovalent an das lösliche Fusionsprotein gebunden ist, und/oder ein Protein-Tag, das kovalent zwischen dem C-β-Kettenfragment und der Cκ-Kette, der Cλ-Kette oder dem Fragment davon gebunden ist.

8. Einkettiger T-Zell-Rezeptor, erzeugt durch Kontaktieren des löslichen Fusionsproteins nach Anspruch 7 mit einem Mittel, das zum Spalten des Protein-Tags imstande ist.

9. Einkettiger T-Zell-Rezeptor nach Anspruch 8, wobei die V-Regionen des einkettigen T-Zell-Rezeptors humanisiert wurden und die C-Regionen des einkettigen T-Zell-Rezeptors humanisiert wurden.

10. DNA-Segment, das für ein lösliches Fusionsprotein nach einem der Ansprüche 1-7 kodiert.

11. DNA-Segment nach Anspruch 10, wobei das lösliche Fusionsprotein weiterhin ein C-α-Kettenfragment umfasst, das kovalent zwischen dem endständigen C der V-α-Kette und dem endständigen N der Peptid-Linker-Sequenz gebunden ist.

12. DNA-Segment nach Anspruch 11, weiterhin umfassend eine Leader-Sequenz, ein Translationsinitiationssignal und einen Promotor und/oder ein oder mehrere Protein-Tags.

13. DNA-Segment nach Anspruch 12, wobei der Promotor phoA ist und der Leader pelB von *E. coli* ist, wobei das DNA-Segment weiterhin eine Ribosomenbindungsstelle von einer Gen-10-Sequenz umfasst oder der Promotor ein Cytomeglovirus-Promotor ist, der operabel mit einem Cytomegalovirus-Verstärkerelement verbunden ist, und der Leader ein Maus-kappa-Ketten-Leader ist.

14. DNA-Vektor, umfassend das DNA-Segment nach einem der Ansprüche 10-13.

15. Verfahren zur Isolierung eines löslichen Fusionsproteins nach einem der Ansprüche 1-7, wobei das Verfahren umfasst:
Einführen eines DNA-Vektors, umfassend eine für das Fusionsprotein kodierende Sequenz, in Wirtszellen,
Kultivieren der Wirtszellen in einem Nährmedium unter Bedingungen, die die Expression des Fusionsproteins zu lassen, und
Aufreinigen des Fusionsproteins aus den Wirtszellen oder dem Medium zum Isolieren des löslichen Fusionsproteins.

16. Verfahren nach Anspruch 15, wobei das Verfahren weiterhin das Kontaktieren eines Extrakts der Wirtszelle oder des Nährmediums mit einem Antikörper umfasst, der imstande ist, das Fusionsprotein spezifisch zu binden.

17. Verfahren nach Anspruch 15, wobei das Verfahren umfasst:
Trennen der fusionierten konstanten Region der leichten Kette des Immunoglobulins oder des Fragments davon von dem einkettigen T-Zell-Rezeptor zum Isolieren des löslichen einkettigen T-Zell-Rezeptors.

18. Verfahren nach Anspruch 15, wobei die Wirtszelle eine Säugetierzelle ist.

19. Verwendung eines löslichen Fusionsproteins nach einem der Ansprüche 1-7 zur Herstellung eines Arzneimittels zum Immunisieren eines Säugetiers gegen T-Zell-Rezeptor-Epitope auf den Oberflächen pathogener T-Zellen.

20. Verwendung eines löslichen Fusionsproteins nach einem der Ansprüche 1-7 zur Herstellung eines Arzneimittels zum Hemmen der T-Zell-Aktivierung in einem Säugetier.

21. Verwendung eines löslichen Fusionsproteins nach einem der Ansprüche 1-7 weiterhin umfassend ein Effektormolekül zur Herstellung eines Arzneimittels zum Töten einer Zelle, umfassend einen Liganden, der imstande ist, das Fusionsprotein spezifisch zu binden.

22. Verwendung nach Anspruch 21, wobei die Zelle eine Tumorzelle oder eine virusinfizierte Zelle ist.

## Revendications

1. Une protéine de fusion soluble comprenant une région constante de chaîne légère d'immunoglobuline, ou un fragment de celle-ci, liée de façon covalente à un récepteur de cellules T de la chaîne simple comprend une chaîne V-α liée de façon covalent à une chaîne V-β par une séquence de peptides de liaison et où le terminal C de la chaîne V-α est liée de façon covalente par la séquence de peptides de liaison au terminal N de la chaîne V-β, et comprenant de plus une chaîne C-β ou un fragment de celle-ci liée de façon covalente entre le terminal C de la chaîne V-β et le terminal N de la région constante de chaîne légère d'immunoglobuline ou un fragment de celle-ci, où le fragment de la chaîne C-β see trouve entre 50 et 126 aminoacides environ en longueur, et où la région constante de chaîne légère d'immunoglobuline ou le fragment de celle-ci est une chaîne Cλ, une chaîne Cκ ou un fragment de chaîne Cκ, où le fragment de chaîne Cκ est entre 70 et 150 aminoacides environ en longueur, et où la protéine de fusion soluble forme une molécule totalement soluble et fonctionnelle.

2. La protéine de fusion soluble de la revendication 1 comprenant de plus une chaîne C-α ou un fragment de celle-ci, où le fragment de chaîne C-α est entre 5 et 90 aminoacides environ en longueur, lié de façon covalente entre le terminal C de la chaîne V-α et le terminal N de la séquence de peptides de liaison.

3. La protéine de fusion soluble de n'importe laquelle des revendications 1-2, où les chaînes V-α et V-β sont au moins à 90% identique aux chaînes V du récepteur des cellules T présentes sur une cellule T cytotoxique.

4. La protéine de fusion soluble de n'importe laquelle des revendications 1-3, où la séquence de peptides de liaison est entre 7 à 20 aminoacides environ en longueur.

5. La protéine de fusion soluble de n'importe laquelle des revendications 1-4, où la région constante de chaîne légère d'immunoglobuline est une chaîne Cκ ou un fragment de chaîne Cκ et le fragment de chaîne Cκ est entre 90 et 120 aminoacides environ en longueur.

6. La protéine de fusion soluble de n'importe laquelle des revendications 1-5, où la région constante de chaîne légère d'immunoglobuline est une chaîne Cκ ou un fragment de chaîne Cκ et le fragment de chaîne Cκ est entre 100 et 110 aminoacides environ en longueur.

7. La protéine de fusion soluble de n'importe laquelle des revendications 1-6 comprenant de plus au moins une étiquette de protéine liée de façon covalente à la protéine de fusion soluble ; et/ou une étiquette de protéine liée de façon covalente entre le fragment de chaîne C-β et la chaîne Cκ, la chaîne Cλ, ou ledit fragment de celle-ci.

8. Un récepteur de cellules T de la chaîne simple produit en mettant en contact la protéine de fusion soluble de la revendication et un agent capable de faire adhérer l'étiquette de protéine.

9. Le récepteur de cellules T de la chaîne simple de la revendication 8, où les régions V du récepteur de cellules T de la chaîne simple ont été humanisées et les régions C du récepteur de cellules T de la chaîne simple ont été humanisées.

10. Un segment d'ADN codant une protéine de fusion soluble selon n'importe laquelle des revendications 1-7.

11. Le segment d'ADN de la revendication 10, où la protéine de fusion soluble comprend de plus, un fragment de chaîne C-α lié de façon covalente entre le terminal C de la chaîne V-α et le terminal N de la séquence de peptides de liaison.

12. Le segment d'ADN de la revendication 11 comprenant de plus une séquence directrice, un signal d'initiation de la translation et un promoteur ; et/ou une ou plusieurs étiquettes de protéine.

13. Le segment d'ADN de la revendication 12, où le promoteur est phoA et le leader est pelB à partir du *E. coli,* où le segment d'ADN comprend également un site de liaison ribosomique à partir d'une séquence 10 de gène ou le promoteur est un promoteur du cytomégalovirus lié de façon opérable à un élément amplificateur du cytomégalovirus et le leader est un leader de chaîne kappa de souris.

14. Un vecteur d'ADN comprenant le segment d'ADN de l'une des revendications 10-13.

15. Une méthode d'isolation d'une protéine de fusion soluble de n'importe quelle revendication 1-7, la méthode comprenant :
l'introduction dans des cellules hôtes d'un vecteur d'ADN comprenant une séquence codant la protéine de fusion,
la culture des cellules hôtes dans un milieu de culture dans des conditions qui permettent l'expression de la protéine de fusion ; et
la purification de la protéine de fusion à partir de la cellule hôte ou le milieu pour isoler la protéine de fusion soluble.

16. La méthode de la revendication 15, où la méthode comprend de plus le contact entre un extrait de la cellule hôte ou du milieu de culture et un anticorps capable de lier de façon spécifique la protéine de fusion.

17. La méthode de la revendication 15, la méthode comprenant :
la séparation de la région constante de la chaîne légère d'immunoglobuline fusionnée ou du fragment de celle-ci, du récepteur de cellules T de la chaîne simple pour isoler le récepteur de cellules T de la chaîne simple soluble.

18. La méthode de la revendication 15, où la cellule hôte est une cellule mammalienne.

19. Utilisation d'une protéine de fusion soluble de n'importe laquelle des revendications 1-7, pour la préparation d'un médicament pour immuniser un mammifère contre les épitopes du récepteur de cellules T sur les surfaces de cellules T pathogènes.

20. Utilisation d'une protéine de fusion soluble de n'importe laquelle des revendications 1-7, pour la préparation d'un médicament pour inhiber l'activation des cellules T chez un mammifère.

21. Utilisation d'une protéine de fusion soluble de n'importe laquelle des revendications 1-7 comprenant de plus une molécule effectrice, pour la préparation d'un médicament pour tuer une cellule comprenant un ligand capable de lier de façon spécifique la protéine de fusion soluble.

22. L'utilisation de la revendication 21, où la cellule est une cellule tumorale ou une cellule infectée viralement.


### SEQUENCE LISTING

(1) GENERAL INFORMATION
   (i) APPLICANT: Sunol Molecular Corporation
   (ii) TITLE OF THE INVENTION: SOLUBLE SINGLE-CHAIN T-CELL RECEPTOR PROTEINS
   (iii) NUMBER OF SEQUENCES: 146
   (iv) CORRESPONDENCE ADDRESS:
      (A) ADDRESSEE: Dike, Bronstein, Roberts & Cushman, LLP
      (B) STREET: 130 Water Street
      (C) CITY: Boston
      (D) STATE: MA
      (E) COUNTRY: USA
      (F) ZIP: 02109
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ for Windows Version 2.0
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/943,086
      (B) FILING DATE: 02-OCT-1997
   (viii) ATTORNEY/AGENT INFORMATION:
      (A) NAME: Corless, Peter F
      (B) REGISTRATION NUMBER: 33,860
      (C) REFERENCE/DOCKET NUMBER: 47594-PCT
   (ix) TELECOMMUNICATION INFORMATION:
      (A) TELEPHONE: 617-523-3400
      (B) TELEFAX: 617-523-6440
      (C) TELEX:
INFORMATION FOR SEQ ID NO:1:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:1:
      CGGCCATGGC CCAGCTGCAG ACTAGTGC 28
(2) INFORMATION FOR SEQ ID NO:2:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:2:
      GGCCGCACTA GTCTGCAGCT GGGCCATGGC CGGCT 35
(2) INFORMATION FOR SEQ ID NO:3:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:3:
      CTCGCGGCCC AGCCGGCCAT GGCCGAGGCT GCAGTCACCC AAAGC 45
(2) INFORMATION FOR SEQ ID NO:4:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:4:
      CTTCCTCACT AGTACAGTCT GCTCGGCCCC AG 32
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:5:
      GATGGCCTCG AGGAGCAGGT GGAGCAGCTT 30
(2) INFORMATION FOR SEQ ID NO:6:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:6:
      GACTAGCCCG GGACAGGGAA CGTCTGAACT GGG 33
(2) INFORMATION FOR SEQ ID NO:7:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:7:
      CTCGCGGCCC AGCCGGCCAT GGCCGAGCAG GTGGAGCAGC TTCCT 45
(2) INFORMATION FOR SEQ ID NO:8:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:8:
      CTCGCGCTCG AGGAGGCTGC AGTCACCCAA AGC 33
(2) INFORMATION FOR SEQ ID NO:9:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:9:
      CTCGCGCCCG GGACAGTCTG CTCGGCCCCA GGC 33
(2) INFORMATION FOR SEQ ID NO:10:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:10:
      CTCGCGACTA GTACAGGGAA CGTCTGAACT GGG 33
(2) INFORMATION FOR SEQ ID NO:11:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:11:
      CTCGCGCCCG GGGTCTGCTC GGCCCCAGGC 30
(2) INFORMATION FOR SEQ ID NO:12:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:12:
      CTCGCGACTA GTGGGAACGT CTGAACTGGG 30
(2) INFORMATION FOR SEQ ID NO:13:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:13:
      CTCGCGACTA GTGTCTGCTC GGCCCCAGGC 30
(2) INFORMATION FOR SEQ ID NO:14:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:14:
      CTCGCGCCCG GGGGGAACGT CTGAACTGGG 30
(2) INFORMATION FOR SEQ ID NO:15:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:15:
      CTCGCGCTCG AGCGAGGCTG CAGTCACCCA AAGC 34
(2) INFORMATION FOR SEQ ID NO:16:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:16:
      GGGGGGCCCG GGGCTGAGGG TGACGATCCC GCAAAAG 37
(2) INFORMATION FOR SEQ ID NO:17:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (xi) SEQUENCE DESCRIPTION: SEQ ID NO:17:
   CTAGTCTGGT GGCGGTGGCA GCGGCGGTGG TGGTTCCGGT GGCGGCGGTT CTGGCGGTGG 60
   CGGTTCC 67
(2) INFORMATION FOR SEQ ID NO:18:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (xi) SEQUENCE DESCRIPTION: SEQ ID NO:18:
   TCGAGGAACC GCCACCGCCA GAACCGCCGC CACCGGAACC ACCACCGCCG CTGCCACCGC 60
   CACCAGA 67
(2) INFORMATION FOR SEQ ID NO:19:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:19:
      GTGCTCACTA GTGTTTGGCT CTACAGTGAG TTTGGTG 37
(2) INFORMATION FOR SEQ ID NO:20:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:20:
      GATGGCTCGA GTGAGCAGGT GGAGCAGCTT CCT 33
(2) INFORMATION FOR SEQ ID NO:21:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:21:
      CTAGTCCCCG GGTACAACTG TGAGTCTGGT TCC 33
(2) INFORMATION FOR SEQ ID NO:22:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:22:
      CTCGAGACTA GTTACAACTG TGAGTCTGGT TCC 33
(2) INFORMATION FOR SEQ ID NO:23:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:23:
      CGGCCGAGGA AGAAGAGTAC ATCCCGATGG ATC 33
(2) INFORMATION FOR SEQ ID NO:24:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:24:
      GGGCCATCCA TCGGGATGTA CTCTTCTTCC TCGGCCGGCT 40
(2) INFORMATION FOR SEQ ID NO:25:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:25:
      CCGGGGAGGA AGAAGAGTAC ATCCCGATGG ATTGAG 36
(2) INFORMATION FOR SEQ ID NO:26:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:26:
      AATTCTCAAT CCATCGGGAT GTACTCTTCT TCCTCC 36
(2) INFORMATION FOR SEQ ID NO:27:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:27:
      GCCCGGGACT AGTGC 15
(2) INFORMATION FOR SEQ ID NO:28:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:28:
      GGCCGCACTA GTCCCGGGCT GCA 23
(2) INFORMATION FOR SEQ ID NO:29:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (xi) SEQUENCE DESCRIPTION: SEQ ID NO:29:
   CTAGTCCCCG GGTCATCAAG CGGCGCCTTC CATCGGCATG TACTCTTCTT CCTCTACAAC 60
   TGTGAGTCTG GTTCC 75
(2) INFORMATION FOR SEQ ID NO:30:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:30:
   CTAGTCCCCG GGTCATCAAG CGGCGCCTTC CATCGGCATG TACTCTTCTT CCTCGTCTGC 60
   TCGGCCCCAG GC 72
(2) INFORMATION FOR SEQ ID NO:31:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:31:
      CTAGTCCCCG GGTACAACTG TGAGTCTGGT TCC 33
(2) INFORMATION FOR SEQ ID NO:32:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:32:
      CCGGGGAGGA AGAAGAGTAC ATGCCGATGG AAGGCGCCGC TTAGC 45
(2) INFORMATION FOR SEQ ID NO:33:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:33:
      CCTCCTTCTT CTCATGTACG GCTACCTTCC GCGGCGAATC GGGCC 45
(2) INFORMATION FOR SEQ ID NO:34:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:34:
      GATCAGCCCG GGGAGGCTGC AGTCACCCAA AGC 33
(2) INFORMATION FOR SEQ ID NO:35:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:35:
      CTAGTCCCCG GGACAGTCTG CTCGGCCCCA CCG 33
(2) INFORMATION FOR SEQ ID NO:36:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:36:
      CCGGGGAGGA AGAAGAGTAC ATGCCGATGG AAGGCGCCGC TC 42
(2) INFORMATION FOR SEQ ID NO:37:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:37:
      CCTCCTTCTT CTCATGTACG GCTACCTTCC GCGGCGAGGG CC 42
   (2) INFORMATION FOR SEQ ID NO:38:
      (i) SEQUENCE CHARACTERISTICS:
         (A) LENGTH: 32 base pairs
         (B) TYPE: nucleic acid
         (C) STRANDEDNESS: single
         (D) TOPOLOGY: linear
   (xi) SEQUENCE DESCRIPTION: SEQ ID NO:38:
      CGCCGCTCAC CATCACCATC ATCACTGATG AC 32
(2) INFORMATION FOR SEQ ID NO:39:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:39:
      GGCGAGTGGT AGTGGTAGTA GTGACTACTG GGCC 34
(2) INFORMATION FOR SEQ ID NO:40:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:40:
      GATCAGGGCG CCGCTACTGT TGAAAGTTGT TTA 33
(2) INFORMATION FOR SEQ ID NO:41:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:41:
      CTGATCGGAT CCTCATTAAA GCCAGAATGG AAA 33
(2) INFORMATION FOR SEQ ID NO:42:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:42:
      CCGGGCTAAG CGGCGCCTTC CATCGGCATG TACTCTTCTT CCTCC 45
(2) INFORMATION FOR SEQ ID NO:43:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:43:
      CCGGGAGCGG CGCCTTCCAT CGGCATGTAC TCTTCTTCCT CC 42
(2) INFORMATION FOR SEQ ID NO:44:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:44:
      CCGGGTCATC AGTGATGATG GTGAGCG G 35
(2) INFORMATION FOR SEQ ID NO:45:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:45:
      GCTCGAGCTT ACTCC 15
(2) INFORMATION FOR SEQ ID NO:46:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (xi) SEQUENCE DESCRIPTION: SEQ ID NO:46:
      CGCTCATTAG GCGG 14
(2) INFORMATION FOR SEQ ID NO:47:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:47:
      GTGTACTTCT GTGCC 15
(2) INFORMATION FOR SEQ ID NO:48:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:48:
      CTGTGAGTCT GGTTC 15
(2) INFORMATION FOR SEQ ID NO:49:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:49:
      GCAGGTTCTG GGTTC 15
(2) INFORMATION FOR SEQ ID NO:50:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:50:
      CATTTACTAA CGTCTGG 17
(2) INFORMATION FOR SEQ ID NO:51:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:51:
      CGCCTGGTAC TGAGC 15
(2) INFORMATION FOR SEQ ID NO:52:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:52:
      CCTCAACCTC CTGTC 15
(2) INFORMATION FOR SEQ ID NO:53:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:53:
      CTTATTCCGT GGTGTC 16
(2) INFORMATION FOR SEQ ID NO:54:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:54:
      CCACCCTCAG AACCG 15
(2) INFORMATION FOR SEQ ID NO:55:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:55:
      GAATTTACCG TTCCAG 16
(2) INFORMATION FOR SEQ ID NO:56:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:56:
      CTTTAGCGTC AGACTG 16
(2) INFORMATION FOR SEQ ID NO:57:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:57:
      GAAACGCAAA GACACC 16
(2) INFORMATION FOR SEQ ID NO:58:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:58:
      GGGGGGCCCG GGCTGCTGAG GGTGACGATC CCGCAAAAG 39
(2) INFORMATION FOR SEQ ID NO:59:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:59:
      GGGGGGGAAT TCTATTAGCT TGCTTTCGAG GTGAATTTC 39
(2) INFORMATION FOR SEQ ID NO:60:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO:60:
      GAGCACGGCC CAGCCGGCCA TGGCCGAGGC TGCAGTCACC C 41
(2) INFORMATION FOR SEQ ID NO:61:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (xi) SEQUENCE DESCRIPTION: SEQ ID NO:61:
   GAGCACGAGA CTAGTAGCAC GAACAACACG GTCGTCGATC GGTTCCGGCG GGTTTGGCTC 60
   TACAGTGAG 69
(2) INFORMATION FOR SEQ ID NO:62:
   (i) SEQUENCE CHARACTERISTICS:
      (A) LENGTH: 86 base pairs
      (B) TYPE: nucleic acid
      (C) STRANDEDNESS: single
      (D) TOPOLOGY: linear
   (xi) SEQUENCE DESCRIPTION: SEQ ID NO:62:
   GATCCCTCCT GGACACGCAG GATGGAAGGA AGCTGCTCCA CCTGCTCAGC ACGAACAACA 60
   CGGTCGTCGA TCGGTTCCGG CGGGGC 86
(2) INFORMATION FOR SEQ ID NO:63:
   (i) SEQUENCE CHARACTERISTICS:
      (A) LENGTH: 86 base pairs
      (B) TYPE: nucleic acid
      (C) STRANDEDNESS: single
      (D) TOPOLOGY: linear
   (xi) SEQUENCE DESCRIPTION: SEQ ID NO:63:
   CATGGCCCCG CCGGAACCGA TCGACGACCG TGTTGTTCGT GCTGAGCAGG TGGAGCAGCT 60
   TCCTCCCATC CTGCGTGTCC AGGAGG 86
(2) INFORMATION FOR SEQ ID NO:64:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:64:
      GAGGTGGAAT TCTATTAAGA CTCCTTATTA CGCAGTATG 39
(2) INFORMATION FOR SEQ ID NO:65:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (xi) SEQUENCE DESCRIPTION: SEQ ID NO:65:
      GAGGAGGTGG TGACTAGTAG CAGGTTCTGG TGGGTTCTGG ATGTTTGGCT CTACAGTGAG 60
(2) INFORMATION FOR SEQ ID NO:66:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (xi) SEQUENCE DESCRIPTION: SEQ ID NO:66:
      GAGGAGGTGG TGACTAGAAG CAGGTTCTGG GTTCTGGATG TTTGGCTCTA CAGTGAG 57
(2) INFORMATION FOR SEQ ID NO:67:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (xi) SEQUENCE DESCRIPTION: SEQ ID NO:67:
      GAGGTGGAAT TCTATTAGTG ATGATGGTGA TGGTGAGACT CCTTATTACG C 51
(2) INFORMATION FOR SEQ ID NO:68:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:68:
      GAGGTGCCCG GGACTGTTGA AAGTTGTTTA GC 32
(2) INFORMATION FOR SEQ ID NO:69:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (xi) SEQUENCE DESCRIPTION: SEQ ID NO:69:
      GAGGTGGAAT TCTATTAGTG ATGATGGTGA TGGTGGCTTG CTTTCGAGG 49
(2) INFORMATION FOR SEQ ID NO:70:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:70:
      GAGGTGGAAT TCTATTAGCT TGCTTTCGAG G 31
(2) INFORMATION FOR SEQ ID NO:71:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:71:
      GAGGTGCCCG GGGCTGAGGG TGACGATCCC G 31
(2) INFORMATION FOR SEQ ID NO:72:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:72:
      AATTCTCATC AGTGATGATG GTGATGGTGC 30
(2) INFORMATION FOR SEQ ID NO:73:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:73:
      CCGGGCACCA TCACCATCAT CACTGATGAG 30
(2) INFORMATION FOR SEQ ID NO:74:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (xi) SEQUENCE DESCRIPTION: SEQ ID NO:74:
      GTGGAGCCCG GGTTCCATCG GCATGTACTC TTCTTCCTCT ACAACTGTGA GTCTGG 56
(2) INFORMATION FOR SEQ ID NO:75:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (xi) SEQUENCE DESCRIPTION: SEQ ID NO:75:
   GAGGTGGAAT TCTCACCCGG GTTCCATCGG CATGTACTCT TCTTCCTCGT CTGCTCGGCC 60
   CCAG 64
(2) INFORMATION FOR SEQ ID NO:76:
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (xi) SEQUENCE DESCRIPTION: SEQ ID NO:76:
   GAGGTGCTGC AGGTTCCATC GGCATGTACT CTTCTTCCTC GTCTAGACGG CCCCAGGCCT 60
   C 61
(2) INFORMATION FOR SEQ ID NO:77:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:77:
      GTGGAGCTGC AGGGTCTAGA CGGCCCCAGG CCTC 34
(2) INFORMATION FOR SEQ ID NO:78:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (xi) SEQUENCE DESCRIPTION: SEQ ID NO:78:
      GTGGAGCTGC AGGTGATCCA CCCCCTCCAG ATCCACCCCC TCCGTCTGCT CGGCCCCAG 59
(2) INFORMATION FOR SEQ ID NO:79:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:79:
      GTGGAGAAGC TTTGCCGAGC AGGTGGAGCA GC 32
(2) INFORMATION FOR SEQ ID NO:80:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:80:
      GGGGGGGAGG TGCTGGAGCG AGGCAGCAGT CACC 34
(2) INFORMATION FOR SEQ ID NO:81:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:81:
      GAGCCCACTA GTTTGGCTCT ACAGTGAGTT TGGTG 35
(2) INFORMATION FOR SEQ ID NO:82:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (xi) SEQUENCE DESCRIPTION: SEQ ID NO:82:
   CTAGACCAGC AAATCTGCAC CCACAGAATC CCTAGGACAG CTCCCAGGTT CCTCTGCATG 60
   GTGGA 65
(2) INFORMATION FOR SEQ ID NO:83:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (xi) SEQUENCE DESCRIPTION: SEQ ID NO:83:
   AGCTTCCACC ATGCAGAGGA ACCTGGGAGC TGTCCTAGGG ATTCTGTGGG TGCAGATTTG 60
   CTGGT 65
(2) INFORMATION FOR SEQ ID NO:84:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:84:
      GATCGGTCTA GAGGTGAGCA GGTGGAGCAG CTTCC 35
(2) INFORMATION FOR SEQ ID NO:85:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:85:
      GCCTGGAGAC TCAGCCATG 19
(2) INFORMATION FOR SEQ ID NO:86:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:86:
      GAAGTACATG GCTGAGTCTC C 21
(2) INFORMATION FOR SEQ ID NO:87:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:87:
      GATGAACGTT CCAGATTCCA TGG 23
(2) INFORMATION FOR SEQ ID NO:88:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:88:
      CCCAAATCAA TGTGCCGAAA AC 22
(2) INFORMATION FOR SEQ ID NO:89:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (xi) SEQUENCE DESCRIPTION: SEQ ID NO:89:
      CTAGAACACA GGAGACTGGA GAGCACGAAG AAGAGCCTGG AGCCCATGGT GGA 53
(2) INFORMATION FOR SEQ ID NO:90:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:90:
      GCTCTCCTTG TAGGCCTGAG 20
(2) INFORMATION FOR SEQ ID NO:91:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:91:
      GTACTTCTGT GCCAGCGGTG 20
(2) INFORMATION FOR SEQ ID NO:92:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:92:
      GAGCAATTAT AGCTACTGCC TG 22
(2) INFORMATION FOR SEQ ID NO:93:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:93:
      GGTCTGGAGG CCTTGTATCC 20
(2) INFORMATION FOR SEQ ID NO:94:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (xi) SEQUENCE DESCRIPTION: SEQ ID NO:94:
      AGCTTCCACC ATGGGCTCCA GGCTCTTCTT CGTGCTCTCC AGTCTCCTGT GTT 53
(2) INFORMATION FOR SEQ ID NO:95:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (xi) SEQUENCE DESCRIPTION: SEQ ID NO:95:
   TCGAGGAACC GCCACCGCCA GAACCGCCGC CACCGGAACC ACCACCGCCG CTGCCACCGC 60
   CACCA 65
(2) INFORMATION FOR SEQ ID NO:96:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (xi) SEQUENCE DESCRIPTION: SEQ ID NO:96:
   CTAGTGGTGG CGGTGGCAGC GGCGGTGGTG GTTCCGGTGG CGGCGGTTCT GGCGGTGGCG    60
   GTTCC                                                                65
(2) INFORMATION FOR SEQ ID NO:97:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (xi) SEQUENCE DESCRIPTION: SEQ ID NO:97:
      CCACCATG 8
(2) INFORMATION FOR SEQ ID NO:98:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (xi) SEQUENCE DESCRIPTION: SEQ ID NO:98:
   Glu Glu Glu Glu Tyr Met Pro Met Glu
    1               5
(2) INFORMATION FOR SEQ ID NO:99:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (xi) SEQUENCE DESCRIPTION: SEQ ID NO:99:
   ATGAAATACC TGCTGCCGAC CGCAGCTGCT GGTCTGCTGC TGCTGGCGGC CCAGCCGATG    60
   GCC                                                                  63
(2) INFORMATION FOR SEQ ID NO:100:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (xi) SEQUENCE DESCRIPTION: SEQ ID NO:100:
   Met Lys Tyr Leu Leu Pro Thr Ala Ala Ala Leu Leu Leu Leu Ala Ala
    1               5                   10                  15
   Gln Pro Ala Met
               20
(2) INFORMATION FOR SEQ ID NO:101:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:101:
      GCCGGCCATG GCCRGTGCTG TCRTCTCTC 30
(2) INFORMATION FOR SEQ ID NO:102:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:102:
      GCCGGCCATG GCCGAMRCCM ARGTSACCC 29
(2) INFORMATION FOR SEQ ID NO:103:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:103:
      GCCGGCCATG GCCGATGTGA AAGTAACCC 29
(2) INFORMATION FOR SEQ ID NO:104:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:104:
      GCCGGCCATG GCCGAMRCWG MCRTYTMCC 29
(2) INFORMATION FOR SEQ ID NO:105:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:105:
      GCCGGCCATG GCCARKGCTG GDGTCACTC 29
(2) INFORMATION FOR SEQ ID NO:106:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:106:
      GCCGGCCATG GCCAATGCCG GCGTCATGC 29
(2) INFORMATION FOR SEQ ID NO:107:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:107:
      GCCGGCCATG GCCGRTRCTG GAGTCTCCC 29
(2) INFORMATION FOR SEQ ID NO:108:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:108:
      GCCGGCCATG GCCGATGCTR GARTCACCC 29
(2) INFORMATION FOR SEQ ID NO:109:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:109:
      GCCGGCCATG GCCGATTCTG GAGTCACAC 29
(2) INFORMATION FOR SEQ ID NO:110:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:110:
      GCCGGCCATG GCCGAYGCTG GWGTTATCC 29
(2) INFORMATION FOR SEQ ID NO:111:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:111:
      GCCGGCCATG GCCGATGCTG RYRTTAYCC 29
(2) INFORMATION FOR SEQ ID NO:112:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:112:
      GCCGGCCATG GCCGAAGCTG GAGTTACTCA G 31
(2) INFORMATION FOR SEQ ID NO:113:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:113:
      GCCGGCCATG GCCGATGCTA CTATTCATCA ATGGCC 36
(2) INFORMATION FOR SEQ ID NO:114:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:114:
      GCTGCCACCG CCACCCACCT TGTTCAGGTC C 31
(2) INFORMATION FOR SEQ ID NO:115:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:115:
      GCTGCCACCG CCACCCACGT TTTCAGGTCC 30
(2) INFORMATION FOR SEQ ID NO:116:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:116:
      GGAGGCGGCG GTTCTGCTAA GAGACCACMC AGCC 34
(2) INFORMATION,FOR SEQ ID NO:117:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:117:
      GGAGGCGGCG GTTCTCAGAA GRTAACTCAA RCSC 34
(2) INFORMATION FOR SEQ ID NO:118:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:118:
      GGAGGCGGCG GTTCTCAGTC KGTGASCCAG C 31
(2) INFORMATION FOR SEQ ID NO:119:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:119:
      GGAGGCGGCG GTTCTCAGAG AGTGACTCA GCC 33
(2) INFORMATION FOR SEQ ID NO:120:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:120:
      GGAGGCGGCG GTTCTCWGMA SGTGRAACAA RGTCC 35
(2) INFORMATION FOR SEQ ID NO:121:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:121:
      GGAGGCGGCG GTTCTCAGCA AGTTAAGCAA AATTC 35
(2) INFORMATION FOR SEQ ID NO:122:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:122:
      GGAGGCGGCG GTTCTGAGAR TGTGGRGCWG CATC 34
(2) INFORMATION FOR SEQ ID NO:123:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:123:
      GGAGGCGGCG GTTCTAAKGA RGTGGMGCAG RRTYC 35
(2) INFORMATION FOR SEQ ID NO:124:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:124:
      GGAGGCGGCG GTTCTCAGCT GCTGGAGCAG AG 32
(2) INFORMATION FOR SEQ ID NO:125:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:125:
      GGAGGCGGCG GTTCTCAAMA GRKAGAASAG RATYC 35
(2) INFORMATION FOR SEQ ID NO:126:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:126:
      GGAGGCGGCG GTTCTGGACA AARCMTTGAR CAG 33
(2) INFORMATION FOR SEQ ID NO:127:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:127:
      GGAGGCGGCG GTTCTAAGGA CCAAGTGTTT CAG 33
(2) INFORMATION FOR SEQ ID NO:128:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:128:
      TTCATAGACT AGTAGGGTCA GGGTTCTGG 29
(2) INFORMATION FOR SEQ ID NO:129:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:129:
      GGTGGCGGTG GCAGCGGCGG TGGTGGTTCC GGAGGCGGCG GTTCT 45
(2) INFORMATION FOR SEQ ID NO:130:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (xi) SEQUENCE DESCRIPTION: SEQ ID NO:130:
      CCACCGCCAC CGTCGCCCGC CACCACCAAG GCCATCCGCC GCCAAGA 47
(2) INFORMATION FOR SEQ ID NO:131:
   (i) SEQUENCE CHARACTERISTICS:
      (A) LENGTH: 0 amino acids
      (B) TYPE: amino acid
      (C) STRANDEDNESS: single
      (D) TOPOLOGY: linear
   (xi) SEQUENCE DESCRIPTION: SEQ ID NO:131:
      gAggAggTggTgACTAgTgCTgAgggTgCTgTCCTgAg
(2) INFORMATION FOR SEQ ID NO:132:
   (i) SEQUENCE CHARACTERISTICS:
      (A) LENGTH: 34 base pairs
      (B) TYPE: nucleic acid
      (C) STRANDEDNESS: single
      (D) TOPOLOGY: unknown
   (xi) SEQUENCE DESCRIPTION: SEQ ID NO:132:
      GAGGAGGTGA CTAGTGCTGG TGAAGCTTGT CTGG 34
(2) INFORMATION FOR SEQ ID NO:133:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:133:
      GAGGAGGTGG TGACTAGTAC TGGGAACGTC TGAACTGGG 39
(2) INFORMATION FOR SEQ ID NO:134:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO:134:
      GAGGTGGAGG CCCAGCCGGC CATGGCCCAG GTGAGACAAA G 41
(2) INFORMATION FOR SEQ ID NO:135:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:135:
      GAGGTGGAGC TCGAGCAATG CTGCTGGTGT CATCCAAAC 39
(2) INFORMATION FOR SEQ ID NO:136:
   (i) SEQUENCE CHARACTERISTICS:
      (A) LENGTH': 33 base pairs
      (B) TYPE: nucleic acid
      (C) STRANDEDNESS: single
      (D) TOPOLOGY: linear
   (xi) SEQUENCE DESCRIPTION: SEQ ID NO:136:
      GAGGTGGAGA CTAGTGTCAA GGTTGACCTG AAG 33
(2) INFORMATION FOR SEQ ID NO:137:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:137:
      GAGGTGGAGA CTAGTAGCAG GTTCTGGGTT CTG 33
(2) INFORMATION FOR SEQ ID NO:138:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:138:
      GAGGTGGAGC CCGGGGTCTG CTCGGCCCCA GGC 33
(2) INFORMATION FOR SEQ ID NO:139:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:139:
      GGCCCAGCCG CCAATGCTGG TGTCATCCAA AC 32
(2) INFORMATION FOR SEQ ID NO:140:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:140:
      GAGGTGACCG GTCAGCAGGT GAGACAAAGT CC 32
(2) INFORMATION FOR SEQ ID NO:141:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:141:
      GTGGAGATCG ATAAAGTGTA CTTACGTTTG TCTGCTCGGC CCCAG 45
(2) INFORMATION FOR SEQ ID NO:142:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (xi) SEQUENCE DESCRIPTION: SEQ ID NO:142:
      GTGGAGATCG ATAAGTGTAC TTACGTTTTC ATTAACAGTC TGCTCGGCCC CAG 53
(2) INFORMATION FOR SEQ ID NO:143:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:143:
      GAGGTGACCG GTGAGCAGGT GGAGCAGCTT CC 32
(2) INFORMATION FOR SEQ ID NO:144:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (xi) SEQUENCE DESCRIPTION: SEQ ID NO:144:
      GTGGAGTTCG AAAAGTGTAC TTACGTTTGT CTGCTCGGCC CCA 43
(2) INFORMATION FOR SEQ ID NO:145:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (xi) SEQUENCE DESCRIPTION: SEQ ID NO:145:
      GTGGAGTTCG AAAAGTGTAC TTACGTTTTC ATTAGTCTGC TCGGCCCCA 49
(2) INFORMATION FOR SEQ ID NO:146:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (xi) SEQUENCE DESCRIPTION: SEQ ID NO:146:
   Ser Thr Pro Pro Pro Gly Thr Arg Val
    1               5